# EUROPEAN PATENT SPECIFICATION

(11) **EP 2 609 107 B1**
(45) Date of publication and mention of the grant of the patent: **11.12.2024**
(21) Application number: 11819230.1
(22) Date of filing: 23.08.2011
(51) Int. Cl.: C07H 21/02, C07H 21/00, C07H 23/00

(54) **BLOCK SYNTHESIS OF OLIGORIBONUCLEOTIDES**
BLOCKSYNTHESE VON OLIGORIBONUKLEOTIDEN
PROCÉDÉ DE SYNTHÈSE D'OLIGORIBONUCLÉOTIDES

(30) Priority: 23.08.2010 US 376030 P
(43) Date of publication of application: 03.07.2013
(73) Proprietor: The Royal Institution for the Advancement of Learning / McGill University, Montreal, Québec H3A 2T5 (CA)
(72) Inventor: DAMHA, Masad, J., St. Hubert Québec J3Y 8N9 (CA); HASSLER, Matthew, Saskatoon Saskatchewan S7J 1H2 (CA); CHAN, Tak-Hang, Toronto, Ontario M5J 2G2 (CA); NANDYALA, Mallikarjuna Reddy, Edmonton, Alberta, T6H 4W9 (CA); DONGA, Robert Alexander, Saint-Bruno Québec J3V 5E7 (CA)
(74) Representative: Weickmann & Weickmann PartmbB
(86) International application number: PCT/CA2011/000950
(87) International publication number: WO 2012/024776

(56) References cited:
- WO-A1-2006/096963
- WO-A1-2009/064115
- KELVIN K. OGILVIE ET AL: "The synthesis of oligoribonucleotides VI. The synthesis of a hexadecamer by a block condensation approach", CANADIAN JOURNAL OF CHEMISTRY, vol. 58, no. 14, 15 July 1980 (1980-07-15), pages 1389 - 1397, XP055100597, ISSN: 0008-4042, DOI: 10.1139/v80-219
- KUMAR G ET AL: "IMPROVEMENTS IN OLIGODEOXYRIBONUCLEOTIDE SYNTHESIS: METHYL N,N-DIALKYLPHOSPHORAMIDITE DIMER UNITS OF SOLID SUPPORT PHOSPHITE METHODOLOGY", THE JOURNAL OF ORGANIC CHEMISTRY, AMERICAN CHEMICAL SOCIETY [NOT]ETC. , US, vol. 49, 1 January 1984 (1984-01-01), pages 4905 - 4912, XP002058338, ISSN: 0022-3263, DOI: 10.1021/JO00199A032
- S. W. KIM ET AL: "Synthesis of RNA Dimer and Trimer Blocks and Their Uses", NUCLEIC ACIDS SYMPOSIUM SERIES, vol. 52, no. 1, 1 September 2008 (2008-09-01), pages 403 - 403, XP055028326, ISSN: 0261-3166, DOI: 10.1093/nass/nrn205
- HASSLER M. ET AL.: "RNA synthesis via dimer and trimer phosphoramidite block coupling", TETRAHEDRON LETTERS, vol. 52, no. 20, 15 March 2011 (2011-03-15), pages 2575 - 2578, XP028194553
- DAMHA, M. J. ET AL.: "Oligoribonucleotide synthesis: The silyl-phosphoramidite method", METHODS IN MOLECULAR BIOLOGY, vol. 20, 1993, NEW JERSEY, pages 81 - 114
- OGILVIE K.K. ET AL.: "The synthesis of oligoribonucleotides. II. The use of silyl protecting groups in nucleoside and nucleotide chemistry. VII", CANADIAN JOURNAL OF CHEMISTRY, vol. 56, 1978, pages 2768 - 2780, XP008135930
- BEAUCAGE, S. L.: "Solid-phase synthesis of siRNA oligonucleotides", CURRENT OPINION IN DRUG DISCOVERY AND DEVELOPMENT, vol. 11, no. 2, 2008, pages 203 - 216

## Description

### Field of the Invention

The invention relates to the chemical synthesis of oligonucleotides, in particular oligoribonucleotides. In another aspect, the invention relates to the chemical synthesis of oligoribonucleotides using di- and tri-ribonucleotide building blocks.

### Background of the Invention

The demand for synthetic oligonucleotides has grown exponentially as genome sequencing, functional genomics, and PCR-based detection methods consume enormous quantities of DNA oligonucleotides.

RNA interference (RNAi) as potential therapeutics represents a fundamentally new way to treat human diseases [Manoharan, Curr. Opin. Chem. Biol. 8, 570-579 (2004)]. However, achieving targeted tissue and cellular delivery, stabilization *in vivo,* and cost effective large scale synthesis of RNA are significant bottlenecks in the development of RNAi technology. The reality of mainstream RNAi based therapeutics is rapidly approaching and the demand for these compounds on large scale may soon exceed the capability of manufacturers. Therefore, there is a need to develop synthetic strategies enabling RNA oligomers to be synthesized rapidly and in high purity.

Current methods for DNA and RNA synthesis rely on stepwise addition of monomeric phosphoramidite units on solid supports [Caruthers, M. H. et al. Methods in Enzymology 154, 287-313 (1987); Alvarado-Urbina, G. et al. Science 214, 270-274 (1981)]. Chain elongation from 3'- to 5'-end is preferred, which is achieved by coupling of a nucleoside unit having a 3'-phosphorus (III) group (in its activated form) to a free 5'-hydroxyl group of another nucleoside unit. As solid support, 500 to 1000 Å controlled pore glass (CPG) support or an organic polymer support, such as primer polystyrene support PS200, can be used. Chain elongation begins by cleavage of the 5'-O-dimethoxytrityl group with an organic acid, thus liberating a nucleophilic 5'-hydroxyl group. This terminal nucleophile is then allowed to couple to a protected nucleoside 3'-O-phosphoramidite monomer in the presence of an activator. In the case of RNA synthesis suitable protection of the 2'-hydroxyl group is required (see below). Any unreacted 5'-hydroxyl groups are acetylated in a process referred to as 'capping' . The most commonly used group used for this purpose is an acetyl ester. Thus, 'capping' with acetic anhydride esterifies any unreacted 5'-hydroxyl groups and halts the accumulation of by-products. The newly created phosphite triester 3',5'-linkage is then oxidized to provide the desired and more stable phosphate triester. This process is repeated until an oligomer of the desired length and sequence is obtained. Cleavage of the oligomer from the solid support and removal of the protecting groups from the sugars, phosphates and nucleobases provides the desired target oligomer, which is then separated from shorter failure sequences by ion exchange high pressure liquid chromatography (HPLC), ion-pair reverse phase HPLC, or polyacrylamide gel electrophoresis (PAGE). The full length oligomer is then characterized by mass spectrometry. Meanwhile a large number of DNA oligomers can be synthesized in parallel on DNA microarrays or "gene chips" [Ramsay G., Nature Biotechnology 16, 40-44 (1998)].

The same iterative method may be applied toward the synthesis of DNA and RNA oligonucleotides in solution, for example as described recently by Donga et al. using ionic soluble supports [e.g. Donga, R.A. et al., J. Org. Chem. 71, 7907-7910 (2006); Donga, R.A. et al., Can. J. Chem. 85, 274-282 (2007)]. The use of ionic soluble supports allows for selective precipitations of the growing oligonucleotide over all other reagents used in the oligonucleotide synthesis cycle.

Different chemistries for assembly of the oligonucleotide chain have been described, e.g. the phosphoramidite method (described above), the phosphotriester method, the H-phosphonate method, and the phosphodiester method [Nucleic acids in chemistry and biology (Edited by C. Michael Blackburn and Michael J. Gait); Oxford and New York: Oxford University Press, 1996]. While in the phosphotriester and phosphodiester method the reactive phosphorus group is in the oxidation state +V, more reactive phosphorus +III derivatives are used in the coupling reactions according to the phosphoramidite and H-phosphonate approaches. Both the phosphoramidite and the H-phosphonate methods require oxidation of P(III) to P(V) to yield the stable P(V) derivatives; however, in the H-phosphonate approach the oxidation step is performed only after the entire oligonucleotide chain has been assembled.

Irrespective of the chemistry of coupling used, a problem associated with current DNA synthesis is the high frequency of sequence errors because of the usage of chemically synthesized oligonucleotides. The longer the target oligonucleotide sequence, the more defects there are as a result repeated exposure of the immobilized DNA sequence to deblocking reagents. For example, repeated acidic treatments required to remove the 5'-O-dimethoxytrityl protecting group during assembly of the DNA chain leads to loss of the nucleobase (depurination); as a result, this process is only practical for making relatively short sequences of nucleotides. The current practical limit of the solid-phase phosphoramidite method is about 200 nt for a DNA oligonucleotide of sufficient quality for a biological application. For RNA synthesis on a solid support, the limit appears to be about 100 nt, but for a different reason, i.e., stepwise coupling efficiencies of RNA 3'-phosphoramidite monomers are less (96-99%) than DNA phosphoramidite couplings (98-1000). This is primarily due to steric effects created by the protecting group that masks the 2'-hydroxyl group of the RNA building blocks.

An alternative approach to assemble oligonucleotide chains more rapidly is through "block" condensation reactions, as exemplified by the early work of Khorana *et al.* in the synthesis of a gene fragment through phosphodiester intermediates [Kossel, H. et al., J. Am. Chem. Soc. 89, 2185 (1967); Ohtsuka E. and Khorana, H.G. J. Am. Chem. Soc. 89, 2195 (1967)]. This work culminated in the total synthesis of the structural gene for an alanine transfer ribonucleic acid from yeast [Khorana, G.B. et al. J. Mol. Biol. 72, 209-217 (1972)]. Short oligonucleotide fragments were made in solution, purified and then connected by specific annealing and ligated enzymatically to generate longer duplexes and the alanine tRNA gene sequence. Their initial approach involved successive condensations in solution between "blocks" of protected di-, tri-, and tetra-nucleotides (bearing a 5'-phosphate monoester) and the 3'-hydroxyl end of the growing fully-protected oligonucleotide chain. At each step, the products were separated by anion exchange chromatography and then verified for purity by paper chromatography after removal of the protecting groups. The yields decreased as the chain lengths increased even when large excess of "blocks" was used. Nevertheless, this approach demonstrated two advantages of block coupling over monomer coupling: block couplings reduce the number of synthetic steps and yield more appreciable differences between the starting and the product oligonucleotide chain. For example, the length and overall net charge of the sugar-phosphate backbone are the most important characteristics in the separation of the desired full-length DNA sequence.

Once the coupling approach of choice became the phosphoramidite method, researchers sought methods to synthesize block DNA phosphoramidites for various applications, including performing random mutagenesis in proteins through the replacement of entire codons in a synthetic gene. The synthons became condensable DNA dimer and trimer units, which were compatible with automated synthesizers [Kumar, G. and Poonian, M.S. J. Org. Chem., 49, 4905-4912 (1984); Virnekas, B. et al. Nucleic Acids Research 22, 5600-5607 (1994); Ono, A. et al. Nucleic Acids Research 23, 4677-4682 (1995); Kayushin, A.L. et al. Nucleic Acids Research 24, 3748-3755 (1996); Zehl, A. et al. Chemical Communications (Cambridge) 2677-2678 (1996); Neuner, P. et al. Nucleic Acids Research, 26, 1223-1227 (1998); Eleuteri, A. et al. Nucleosides & Nucleotides 18, 475-483 (1999); Kayushin, A. et al. Nucleosides, Nucleotides & Nucleic Acids 19, 1967-1976 (2000); Yagodkin, A. et al. Nucleosides, Nucleotides & Nucleic Acids 26, 473-497 (2007)].

The principal driver of research into the synthesis of DNA blocks in recent years has been the prospect of oligonucleotides as therapeutic agents. Reese and Yan demonstrated, in their synthesis of Vitravene, that block condensation of trimer units in solution using the less common H-phosphonate method was an achievable goal [Reese, C. B. and Yan, H. Journal of the Chemical Society, Perkin Transactions 1: Organic and Bio-Organic Chemistry pp. 2619-33 (2002)].

To date, there have been many attempts to design protecting groups and methods that embody the conditions required for the construction of high quality oligoribonucleotides [for reviews, see Beaucage, S. L. Curr. Opin. Drug Discov. Devel. 11, 203-16 (2008); Reese, C. B. Organic & Biomolecular Chemistry 3, 3851-3868 (2005)] . In fact, for many years, RNA synthesis has been regarded as far more complicated than DNA synthesis because of the difficulty in finding a compatible 2'-protecting group that (a) affords high step-wise coupling yields, (b) is stable throughout chain assembly, and (c) can be removed selectively at the end of synthesis without phosphodiester bond isomerization or degradation.

The most widely used 2'-protecting group is the 2'*-O-t-*butyldimethylsilyl (TBDMS) group [Ogilvie, K.K. et al. Tetrahedron Letters 15, 2861-2867 (1974)]. This protecting group is removed at the end of RNA chain assembly in the presence of fluoride ions. Other silyl ether based protecting groups described for the protection of nucleosides are triisopropylsilyl (TIPS), methyldiisopropylsilyl (MDIPS), cyclic alkylsilyl and other silyl groups [Ogilvie, K.K. et al. J. of Carbohydrates, Nucleosides, Nucleotides 3, 197-227 (1976); Damha M.J, Ogilvie K.K. (1993), Oligoribonucleotide synthesis: the silyl-phosphoramidite method. In: Protocols for Oligonucleotides and Analogs: Synthesis and Properties, Methods in Molecular Biology (Agrawal S, ed.) Vol. 20. Totowa, NJ: The Humana Press Inc. pp. 81-114]. Among these, TIPS protection has been described primarily for 5'-*O*-monomethoxytrityl N2-isobutyrylguanosine derivatives as the 2' and 3'-*O*-TIPS isomers are more readily separated from each other by silica gel chromatography [Damha M.J, Ogilvie K.K. (1993), Oligoribonucleotide synthesis: the silyl-phosphoramidite method. In: Protocols for Oligonucleotides and Analogs: Synthesis and Properties, Methods in Molecular Biology (Agrawal S, ed.) Vol. 20. Totowa, NJ: The Humana Press Inc. pp. 81-114; Damha, M.J. et al. Tetrahedron Letters 45, 6739-6742 (1992)]. The smaller steric bulk of the TBDMS group relative to TIPS, TBDPS and other bulkier silyl ethers would favor the TBDMS protecting group, which has been used the most compared to other protecting group for RNA synthesis. Coupled with the phosphoramidite condensation-procedure, 2'-*O*-TBDMS monomers have allowed a highly efficient synthesis of oligoribonucleotides [Ogilvie, K.K. et al. Proc. Natl. Acad. Science (USA), 85, 5764-5768 (1988); Usman, N. et al. J. Am. Chem. Soc. 109, 7845-7854 (1987)].

A potential drawback of silyl ethers for the protection of the 2'-hydroxyl group lies in their widely recognized ability to undergo 2'-to-3' isomerization under the influence of protic solvents, nucleophilic catalysts, or basic conditions. For example, the TBDMS group migrates from the O2' to O3' position (and vice versa) in the presence of either methanol, imidazole, pyridine/water, or aqueous ammonia, thereby generating a mixture of nucleoside O2' and O3' silyl regioisomers [Ogilvie, K.K. and Entwistle, D. W. Carbohydrate Res. 89, 203-210 (1981); Ogilvie, K. K. (1983) Proceedings of the 5th International Round Table on Nucleosides, Nucleotides and Their Biological Applications, (Rideout, J.L. et al. eds.), Academic, London, pp. 209-256); Damha M.J, Ogilvie K.K. (1993), Oligoribonucleotide synthesis: the silyl-phosphoramidite method. In: Protocols for Oligonucleotides and Analogs: Synthesis and Properties, Methods in Molecular Biology (Agrawal S, ed.) Vol. 20. Totowa, NJ: The Humana Press Inc. pp. 81-114].

Silyl isomerization is characteristic of other *O*-silyl ether protecting groups. *O*-TIPS derivatives of uridine and 7-deazaguanosine also undergo isomerization in methanol, albeit more slowly than their O-TBDMS counterparts [Ogilvie, K.K. et al. J. or Carbohydrates, Nucleosides, Nucleotides 3, 197-227 (1976); Seela, F. and Mersmann, K., Helvetica Chimica Acta, 76, 1435-1449(1993)]. 5'-*O*-Monomethoxytrityl-N2-isobutyryl-2'-O-TIPS guanosine undergoes isomerization under ethanolic aq. ammonia conditions to give a mixture of 2'/3'-TIPS regioisomers which can be separated by chromatography. This provides a method to convert (recycle) more of the unwanted 3'-isomer into the more useful 2'-isomer [Damha M.J, Ogilvie K.K. (1993), Oligoribonucleotide synthesis: the silyl-phosphoramidite method. In: Protocols for Oligonucleotides and Analogs: Synthesis and Properties, Methods in Molecular Biology (Agrawal S, ed.) Vol. 20. Totowa, NJ: The Humana Press Inc. pp. 81-114].

2'-*O*-Silyl groups do not normally migrate to O3' in dry aprotic solvent. When these conditions are strictly adhered to it is possible to prepare 2'-*O*-silylated ribonucleoside-3'-*O-*phosphoramidite derivatives in regiosomerically pure form [Milecki, J. et al. Nucleosides & Nucleotides, 8, 463-474 (1989); Scaringe, S.A. et al. Nucleic Acids Res., 18, 5433-5341 (1990)]. This is clearly an important requirement as the presence of even traces of the 3'-*O*-silyl regioisomer will impact on the quality and biological activity of the desired RNA sequence.

Many other protecting groups for the 2'-hydroxyl position have been used in the synthesis of RNA [reviewed in Beaucage, S. L. Curr. Opin. Drug Discov. Devel. 11, 203-16 (2008); Reese, C. B. Organic & Biomolecular Chemistry 3, 3851-3868 (2005)]. RNA synthesis using monomers containing the 2'-triisopropylsilyloxymethyl (TOM) group, the 2'-acetal-levulinyl group, and the 2'-*O*-bis(2-acetoxyethoxy)methyl (ACE) group, have been reported to yield higher coupling efficiency, because these protecting groups exhibit lower steric hindrance than the 2'-TBDMS group [for a comparative study, see Lackey, J.G. et al. J. Am. Chem. Soc. 131, 8496-8502 (2009)]. Like the TBDMS group, the TOM protecting group is removed using fluoride.

In all cases the synthesis of oligoribonucleotides is an elaborate multistep process, which entails assembly of the oligonucleotide chain typically from monomeric phosphoramidite building blocks (e.g., 5'-*O*-dimethoxytrityl-N-protected-2'-*O-tert*-butyldimethylsilyl-nucleoside-3'-*O*-phosphoramidites), deprotection of the base labile nucleobase protecting groups (e.g., benzoyl, isobutyryl, acetyl, phenoxyacetyl, levulinyl, etc), cleavage from the support (e.g., glass beads or polystyrene), followed by removal of the 2'-hydroxyl protecting group.

The generation of oligoribonucleotide blocks is more difficult due to the presence of the 2'-hydroxyl group and the protection it requires, thus this line of research has also lagged far behind that of DNA blocks. Nevertheless, there have been several reports describing the synthesis of RNA through block coupling condensation reactions. Ikehara and co-workers coupled RNA trimer and tetramers using the phosphotriester method to give 30% yield after several days [Ohtsuka, E. et al. J. Am. Chem. Soc. 100, 8210 (1978)]. Werstiuk and Nielson reported the coupling of an RNA tetramer and an RNA pentamer affording the desired nonanucleotide RNA sequence in 50% yield after 16 days [Werstiuk, E.S., Neilson, T. Can. J. Chem. 54, 2689 (1976)]. Van Boom and co-workers condensed an RNA tetramer and an RNA decamer in 58% yield in a 3.5 days reaction [van Boom, J.H. et al. Trav. Chim. Pays-Bas, 97, 73 (1978)]. Ogilvie and co-workers described the synthesis of 5'-*O*-monomethoxytrityl-2'-O-tertbutyldimethylsilyl-3'-*O*-levulinyl ribonucleoside monomers and their use in the assembly of a hexadecauridylic acid via the phosphodichloridite procedure [Nemer, M.J, and Ogilvie, K.K., Can. J. Chem. 58, 1389-1397 (1980)].

Solid-phase RNA synthesis is carried out almost exclusively using monomeric phosphoramidite synthons. Given the efficiency of the phosphoramidite chemistry, it is highly desirable to have access to block (dimer and trimer) phosphoramidites for RNA synthesis, as these would permit longer chain extensions at each step during chain assembly, significantly shortening the time required for synthesis.

### Summary of the Invention

The invention is defined in the appended claims. Any disclosure that goes beyond the scope of the said claims is only intended for illustrative purposes and is not part of the invention.

Methods for the synthesis of blockmer (dimer, trimer, tetramer, etc.) ribonucleotides that have applications in the synthesis of RNA through block coupling reactions are described herein. These building blocks allow longer chain extensions at each coupling stage of RNA synthesis, significantly reducing the total number of steps required in the synthesis of a target RNA oligomer. Additionally, the block coupling strategy disclosed herein produces crude RNA oligomers that are more readily separated from shorter failure sequences. The procedure is illustrated by the synthesis of UpU, ApA, and UpUpU blocks and their use in the assembly of oligoribonucleotide chains via the phosphoramidite coupling method. The disclosed compounds and processes benefit two critical aspects of siRNA manufacturing: speed, and purity of the target oligomer.

More particularly, in one aspect there is provided a compound of formula (II): 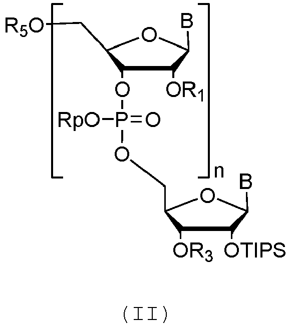 wherein
n is an integer from 1 to 19;
R₁ is a protecting group selected from the group consisting of t-butyldimethylsilyl (TBDMS), triisopropylsilyl (TIPS); triisopropyloxymethyl (TOM); cyanoethylmethyl (CEM); 2-(4-tolylsulfonyl)ethoxymethyl (TEM)2'-O-bis(2-acetoxyethoxy)methyl (ACE) orthoester; 1-(2-fluorophenyl)-4-methoxypiperidin-4-yl (Fpmp); 1-(4-chlorophenyl)-4-ethoxypiperidin-4-yl (Cpep); 4-(N-dichloroacetyl-N-methylamino) benzyloxymethyl (4-MABOM); dimethoxytrityl (DMTr), monomethoxytrityl (MMTr) 2-tert-butyldithiomethyl (DTM); allyl levulinyl (Lev) and acetal levulinyl (ALE); 2-(2-nitrophenyl)propoxycarbonyl (NPPOC), α-methylnitropiperonyloxycarbonyl (MeNPOC), thioxanthone-nitrobenzyl group conjugates, and a 5'-O-dimethoxybenzoincarbonate group (DMBOC);
R3 is a protecting group, which is a levulinyl group (Lev);
R₅ is H, or a protecting group selected from the group consisting of 9-phenylxanthyl (pixyl or Px), MMTr, and DMTr;
Rₚ is a protecting group selected from the group consisting of methyl (Me), 2-cyanoethyl (CNEt), p-nitro-phenylethyl (NPE), and para- and ortho-chloro-phenyl (p- or o-ClPh);
R is lower alkyl, or the N(R)₂ moiety is a cyclic alkylamine, or a substituted cyclic alkylamine, preferably morpholine;
B is a nitrogen-containing base;
wherein each B, R₁ and Rₚ may be the same or different from any other B, R₁ and Rₚ, respectively;
and wherein
the term "lower alkyl" as used herein refers to acyclic, straight or branched chain alkyl groups containing from one to six carbons.

In another aspect, there is provided a process for preparing a compound of formula (II): 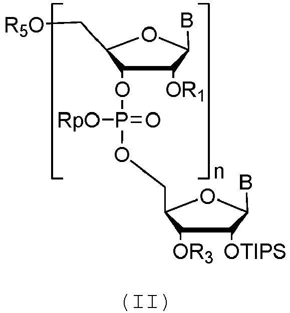 wherein
n is 1, 2, or 3;
R₁ is a protecting group as defined above;
R₃ is a levulinyl group (Lev);
R₅ is a protecting group as defined above;
Rₚ and B are as defined above;
wherein each B, R₁ and Rₚ may be the same or different from any other B, R₁ and Rₚ, respectively;
the process comprising the steps of:
   a) condensing a phosphoramidite of formula (III): 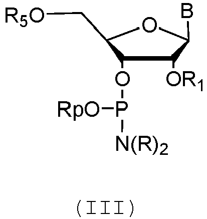
      wherein B, R₁, R₅, and Rₚ are as defined above; and
      R is lower alkyl, or the N(R)₂ moiety is a cyclic alkylamine, or a substituted cyclic alkylamine, preferably morpholine;
         with a nucleoside of formula (IV): 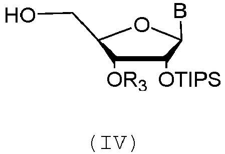
      wherein B and R₃ are as defined above; and
   b) oxidizing the product of step (a) to produce the compound of formula (II) where n is 1, and B, R₁, R₃, R₅, and Rₚ are as defined above; and
   c) where n>1, the process further comprising:
      (i) deprotecting the terminal -OR₅ group of the product of the previous step to form a free 5'-OH group;
      (ii) condensing the product of step (i) with a phosphoramidite of formula (III), wherein B, R₁, R₅, Rₚ and R are as defined above, and each B, R₁, R₅, Rₚ and R may be the same or different from any other B, R₁, R₅, Rₚ and R, respectively;
      (iii) oxidizing the product of step (ii);
      (iv) repeating steps (i)-(iii) n-2 times;
to form the compound of formula (II).

The term "lower alkyl" as used herein refert to acyclic, straight or branched chain alkyl groups containing from one to six carbons.

In yet another aspect, there is provided a process for preparing a compound of formula (V): 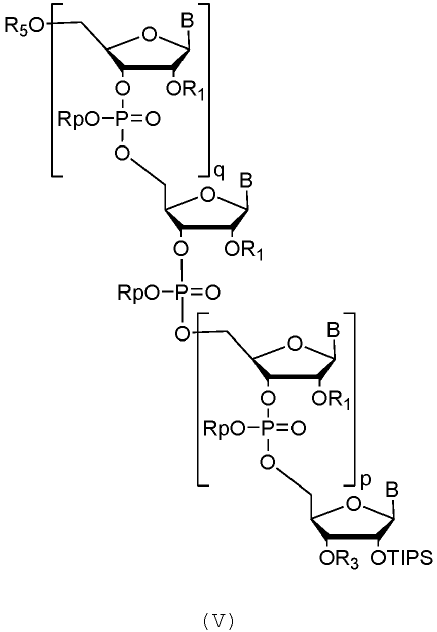 wherein
p is selected from 0, 1, or 2;
q is selected from 0, 1, or 2;
and p+q is less than or equal to 2;
R₃ is a protecting group which is a levulinyl group (Lev);
R₅ is a protecting group as defined above;
R₁, Rₚ and B are as defined above;
wherein each B, R₁ and Rₚ may be the same or different from any other B, R₁ and Rₚ, respectively;
the process comprising the steps of:
   a) condensing a phosphoramidite of formula (VI): 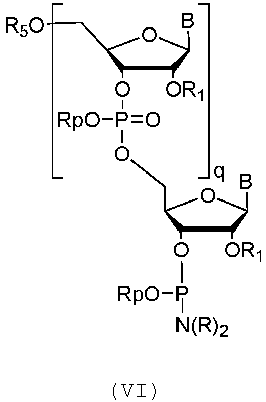
      wherein q, B, R₁, R₅ and Rₚ are as defined above, and
      R is lower alkyl, or the N(R)₂ moiety is a cyclic alkylamine, or a substituted cyclic alkylamine, preferably morpholine;
      with a compound of formula (VII): 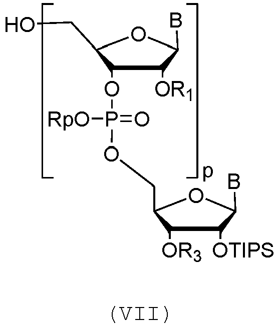 wherein p, B, R₁, R₃, and Rₚ are as defined above; and
   b) oxidizing the product of step (a) to produce the compound of formula (V).

Additionally, the present document describes a process for preparing an N-mer oligonucleotide, said process comprising:
a) condensing a phosphoramidite of formula (IIa): 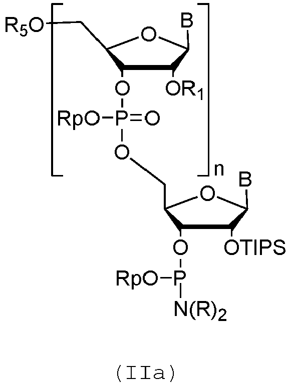 wherein
   n is an integer from 0 to 19;
   R₁ is a protecting group;
   R₅ is a protecting group;
   Rₚ is a protecting group;
   R is lower alkyl, or the N(R)₂ moiety is a cyclic alkylamine, or a substituted cyclic alkylamine, preferably morpholine;
   B is a nitrogen-containing base;
   wherein each B, R₁ and Rₚ may be the same or different from any other B, R₁ and Rₚ, respectively;
      with
      (a') a functionalized linker L bound to a support ⓢ, wherein ⓢ is selected from a solid support or an ionic support, or
      (b') a compound of formula 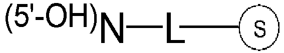 wherein ^{(5'-OH)}N is a nucleoside/oligonucleotide chain bound to the solid support or the ionic support via the functionalized linker L and having a free 5'-OH group;
b) oxidizing the product of step (a) to form a compound of formula (VIII): 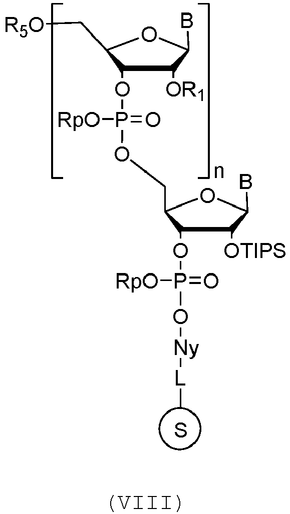 wherein
   y is 0 or 1;
   n, B, R₁, R₅ R_{p,} ⓢ, L, and N are as defined above; and
c) optionally,
   (i) deprotecting the terminal 5'-OR₅ group of the product of the previous step to form a free 5'-OH group;
   (ii) condensing the product of step (i) with a phosphoramidite of formula (IIa) 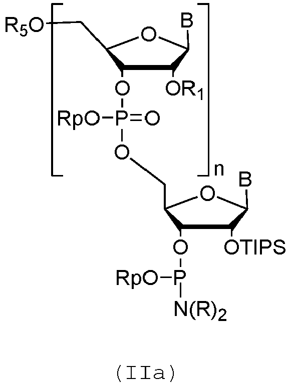 wherein n, B, R₁, R₅, Rₚ and R are as defined above, and each n, B, R₁, R₅, Rₚ and R may be the same or different from any other n, B, R₁, R₅, Rₚ and R, respectively;
   (iii) oxidizing the product of step (ii); and
   (iv) optionally repeating steps (i)-(iii);
with the proviso that if y is 0, step (c) is not optional;
to form a bound N-mer oligonucleotide.

The present document also describes a process for preparing an N-mer oligonucleotide, said process comprising:
a) condensing a compound of formula (IIb): 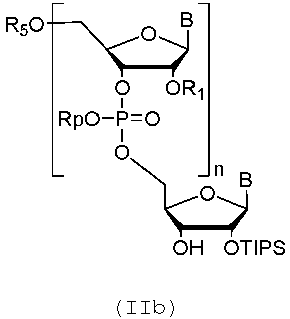 wherein
   n is an integer from 0 to 19;
   R₁ is a protecting group;
   R₅ is a protecting group;
   Rₚ is a protecting group;
   B is a nitrogen-containing base;
   wherein each B, R₁ and Rₚ may be the same or different from any other B, R₁ and Rₚ, respectively;
      with
      a functionalized linker L bound to an ionic support ⓢ,
b) oxidizing the product of step (a) to form a compound of formula (IX): 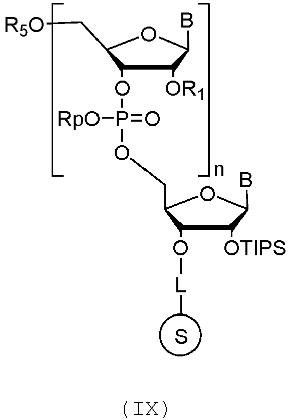 wherein
   n, B, R₁, R₅ Rₚ, ⓢ, and L, are as defined above; and
c)
   (i) deprotecting the terminal 5'-OR₅ group of the product of the previous step to form a free 5'-OH
      group;
   (ii) condensing the product of step (i) with a phosphoramidite of formula (IIa) 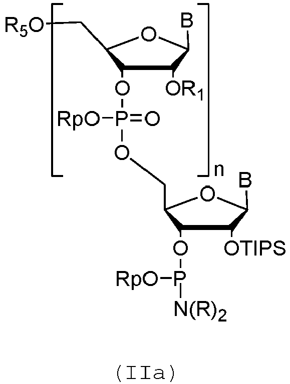 wherein n, B, R₁, R₅, and Rₚ are as defined above, and R is lower alkyl, or the N(R)₂ moiety is a cyclic alkylamine, or a substituted cyclic alkylamine, preferably morpholine; wherein each n, B, R₁, R₅, and Rₚ may be the same or different from any other n, B, R₁, R₅, and Rₚ, respectively;
   (iii) oxidizing the product of step (ii); and
   (iv) optionally repeating steps (i)-(iii);
to form a bound N-mer oligonucleotide.

### Brief Description of the Drawings

Figure 1: ³¹P-NMR of A) compound **(29)** as isolated after chromatography (δ: 0.83, 0.58 ppm), and of B) an artificial mixture of **(29)** and **(33)** (δ: 0.70, 0.60 ppm). Solvent: CD₃CN.
Figure 2: ³¹P-NMR spectrum of compound **(30)** (4 P-diastereomers) in CD₃CN.
Figure 3:
   (A) ³¹P-NMR of crude UpU_TBDMS dimer (CD₃CN) prepared as described in WO 2009/064115.
   (B) ³¹P-NMR spectrum of purified compound **(20)** in CD₃CN. Inset on the right shows the crude material before purification.

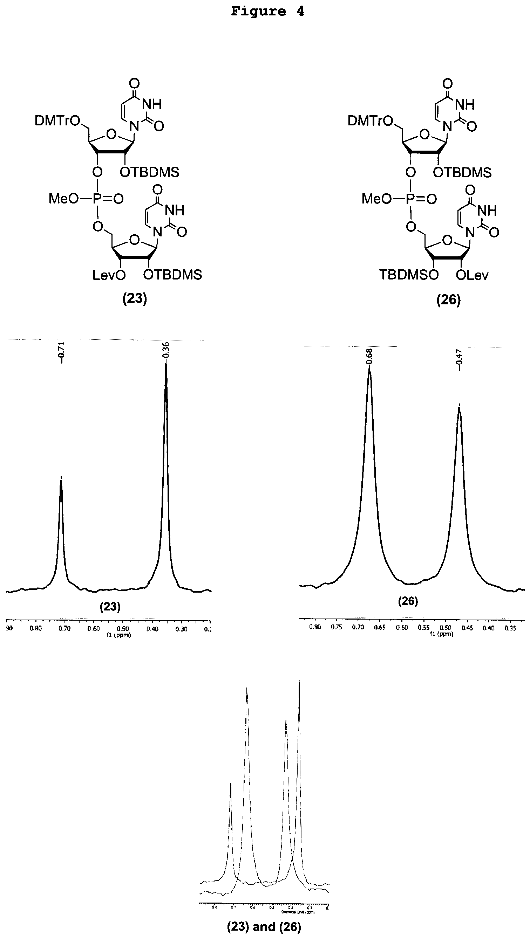

Figure 4: ³¹P-NMR spectra of **(23)** (left), **(26)** (right) in CD₃CN. The overlay of both spectra is shown in the middle.

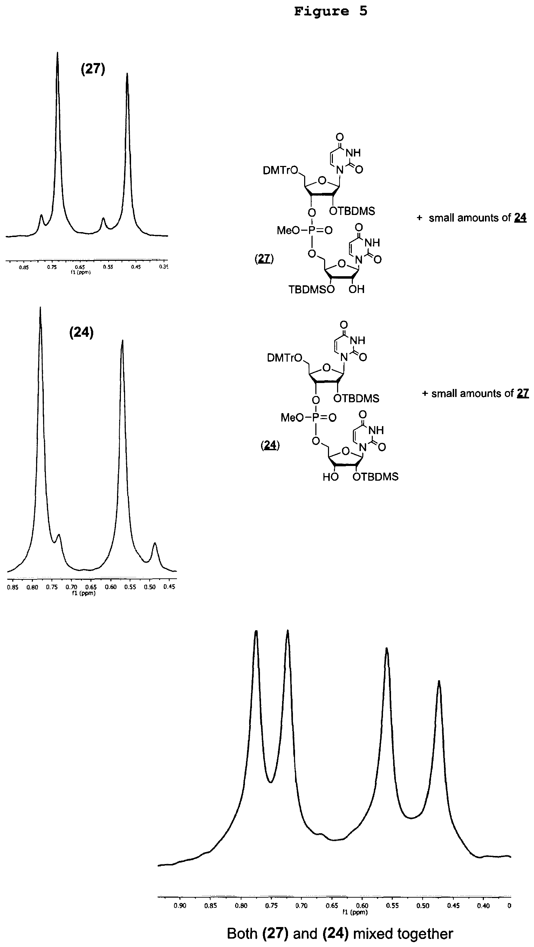

Figure 5: ³¹P-NMR spectrum of **(27)** (top) and **(24)** (bottom) in CD₃CN.

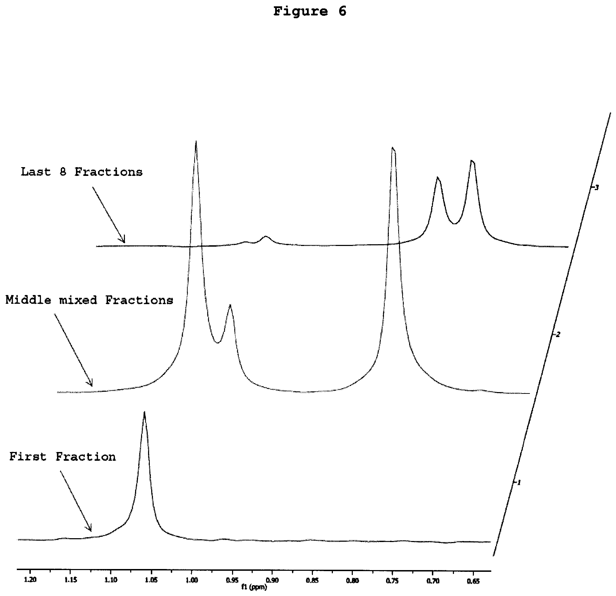

Figure 6: ³¹P-NMR spectra of fractions isolated during attempted separation of **(24)** and **(27)** by silica gel column chromatography on ethylacetate:hexanes (0425% gradient until the compounds eluted). The majority of the material eluted as **(24)** + **(27)** mixtures. Solvent: CD₃CN.

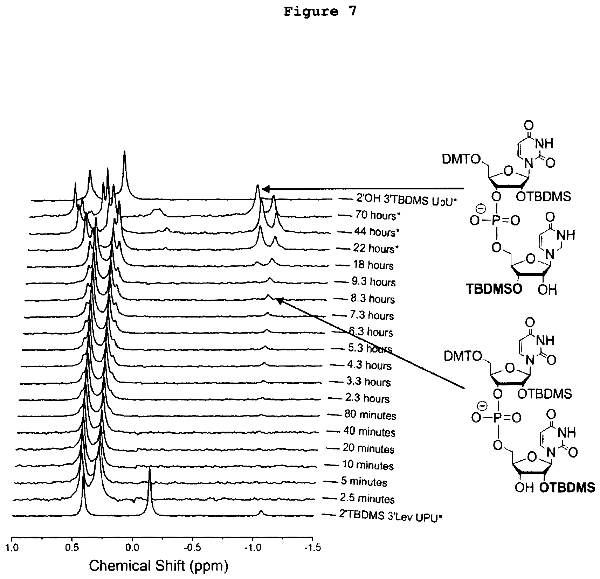

Figure 7: ³¹P NMR study of delevulinylation of 2'-TBDMS 3'-Lev UpU dinucleotide with hydrazine hydrate at 5 equivalence (see Table 2 for conditions).

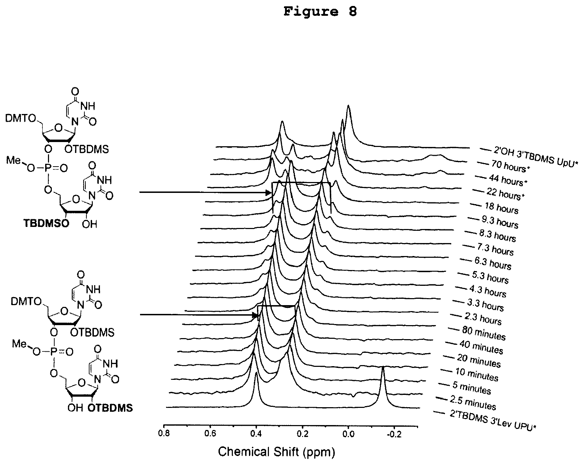

Figure 8: The expansion of the region at ca. 0.5 ppm (0.8-0.2 ppm) of the ³¹P NMR of Figure 7.

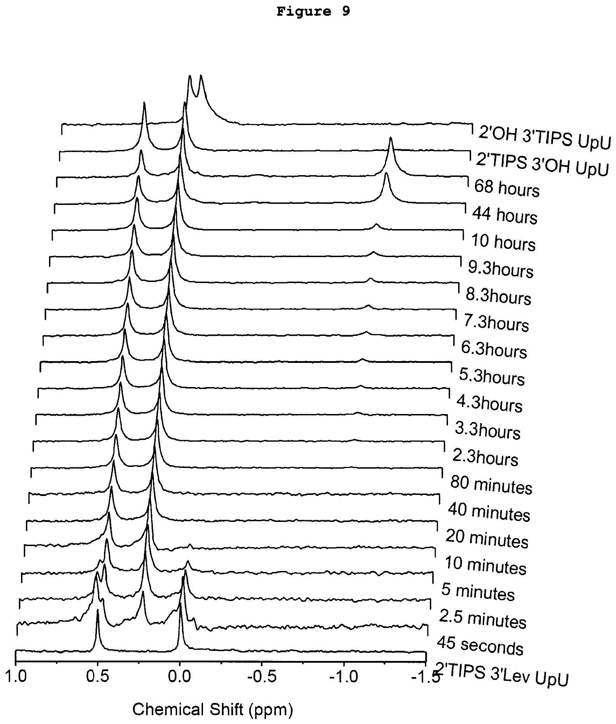

Figure 9: ³¹P NMR study of de-levulinylation of 2'-TIPS 3'-Lev UpU dinucleotide with hydrazine hydrate at 5 equivalence (see Table 2 for conditions).

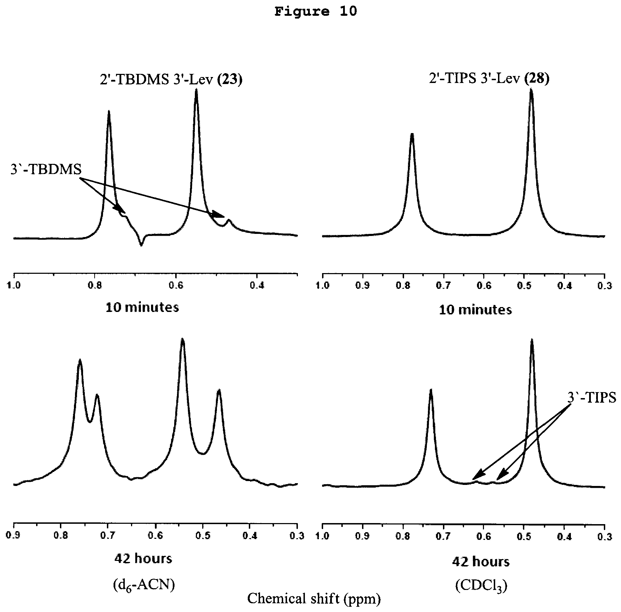

Figure 10: ³¹P-NMR Experiment that compares of de-levulinylation of UpU_TBDMS and UpU_TIPS after 10 minutes and 42 hours treatment with hydrazine solution (see detailed procedure below).
Spectra were recorded, after work up and re-purification of the dimers by silica gel column chromatography.

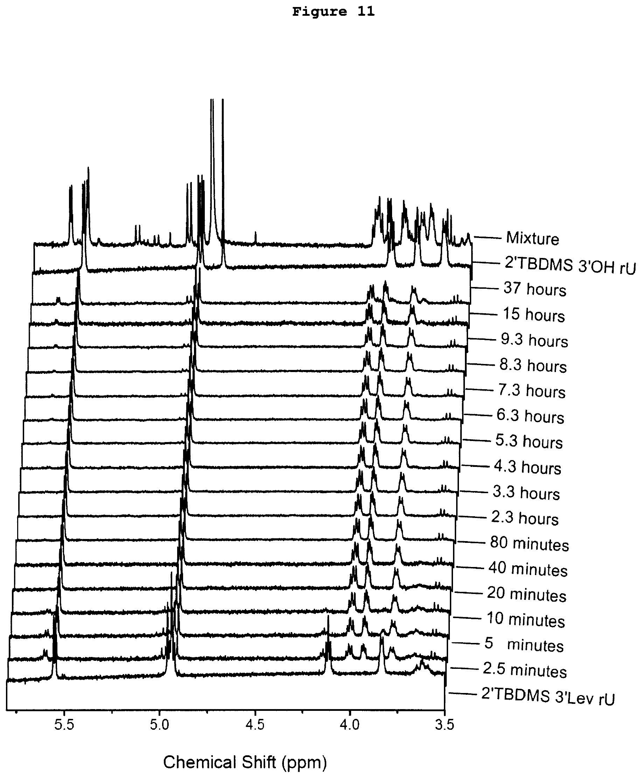

Figure 11: ¹H NMR study of de-levulinylation of 2'-TBDMS 3'-Lev uridine with hydrazine hydrate at 10 equivalence (see Table 2 for conditions).

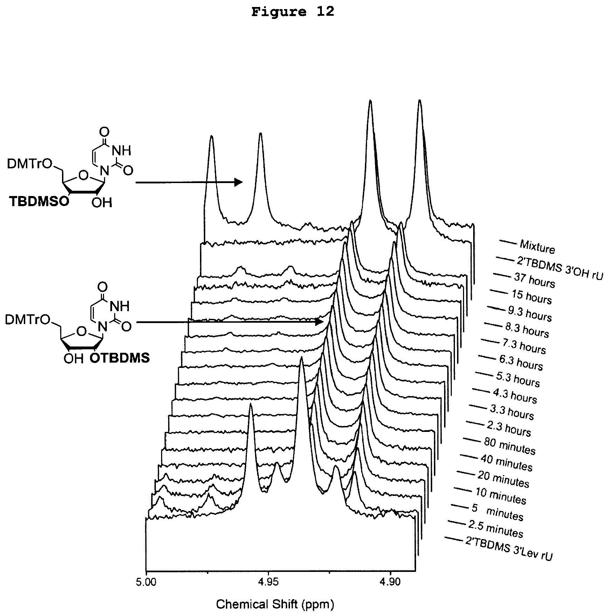

Figure 12: The expansion of the region at ca. 5 ppm (5-4.8ppm) of the ¹H NMR spectrum of Figure 11.

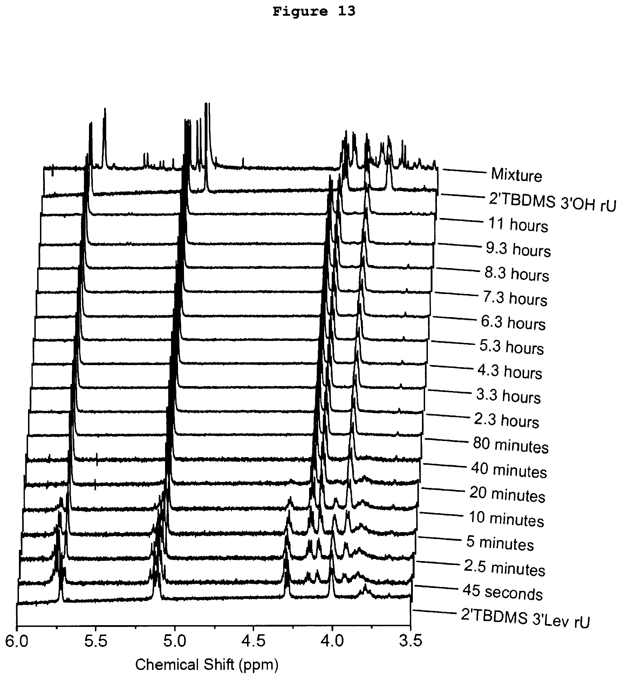

Figure 13: ¹H NMR study of de-levulinylation of 2'-TBDMS 3'-Lev uridine with hydrazine hydrate at 5 equivalence (see Table 2 for conditions).

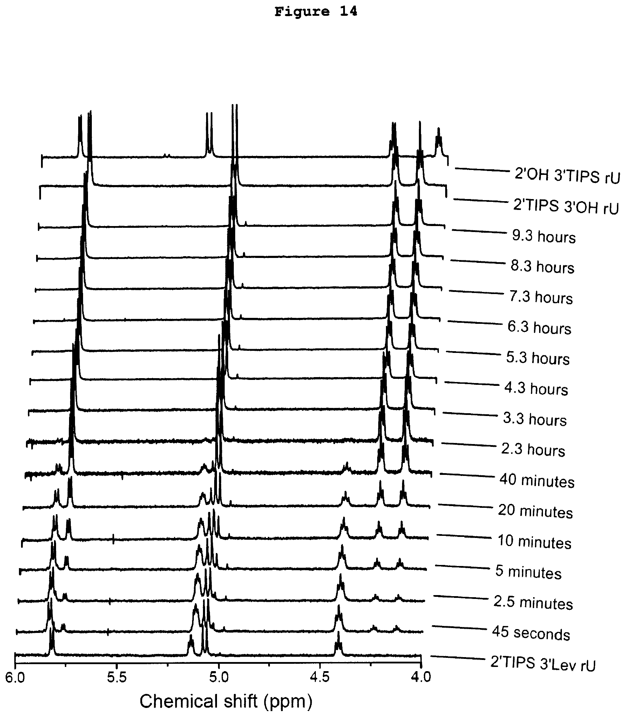

Figure 14: ¹H NMR study of de-levulinylation of 2'-TIPS 3'-Lev uridine with hydrazine hydrate at 5 equivalence (see Table 2 for conditions).

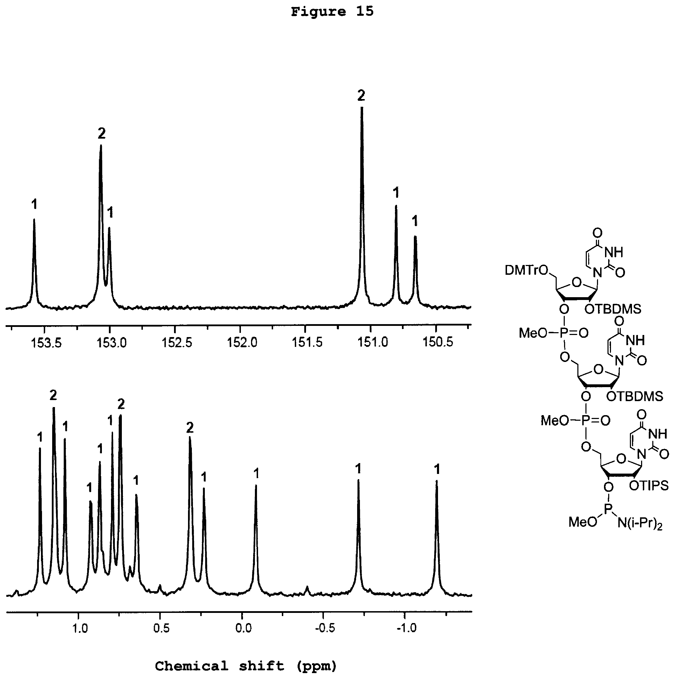

Figure 15: ³¹P-NMR spectrum of UpUpU trimer amidite **(50)** in CDCl₃.

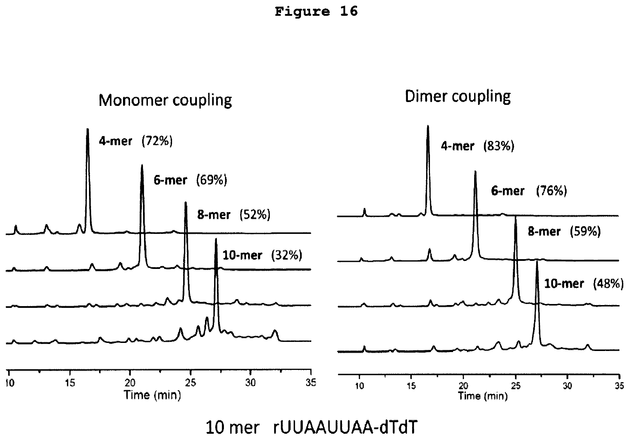

Figure 16: Anion exchange HPLC chromatograms of oligoribonucleotides made in solution with ionic tags and monomer coupling units compared to the same oligoribonucleotides made with block amidites.

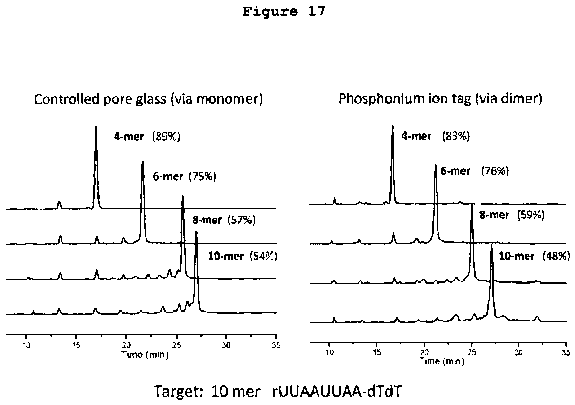

Figure 17: Anion exchange HPLC chromatograms of oligoribonucleotides made in solution with block amidites compared to oligoribonucleotides made on solid support using standard RNA synthesis conditions.
Figure 18:
   A) Anion exchange HPLC chromatograms of 5'-(rUp)₁₈-dT-3' synthesized under unoptimized couplings of monomer **(7),** dimer **(30)** and trimer **(50)** amidites to intentionally show the position of the failure couplings. Expansion of regions at 47 min is shown. Unoptimized coupling conditions: monomer concentration, 0.10 M; coupling time, 600 s.
   B) Results for dimer and trimer coupling under optimized conditions are shown. Optimized coupling conditions: monomer concentration, 0.15M; coupling time, 1200s.

### Detailed Description

The present disclosure relates generally to the field of oligonucleotide synthesis, and provides compositions and methods for the synthesis of RNA. In some embodiments, novel dimer and trimer blocks are provided for the synthesis of RNA oligonucleotides on solid supports. In another embodiment, a tetramer block is provided. In yet another embodiment, larger blockmers are provided (e.g. 5-, 6-, 7-, 8-, 9-, 10-, 11-, 12-, 13-, 14-, 15-, 16-, 17-, 18-, 19-, or 20-mers).

More particularly, in one embodiment there is provided a compound of formula (II): 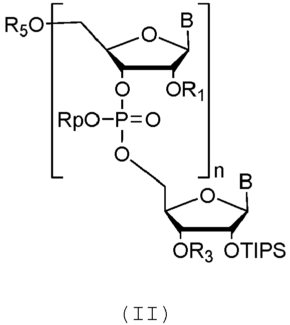 wherein
n is an integer from 1 to 19;
R₁ is a protecting group selected from the group consisting of t-butyldimethylsilyl (TBDMS), triisopropylsilyl (TIPS); triisopropyloxymethyl (TOM); cyanoethylmethyl (CEM); 2-(4-tolylsulfonyl)ethoxymethyl (TEM)2'-O-bis(2-acetoxyethoxy)methyl (ACE) orthoester; 1-(2-fluorophenyl)-4-methoxypiperidin-4-yl (Fpmp); 1-(4-chlorophenyl)-4-ethoxypiperidin-4-yl (Cpep); 4-(N-dichloroacetyl-N-methylamino) benzyloxymethyl (4-MABOM); dimethoxytrityl (DMTr), monomethoxytrityl (MMTr) 2-tert-butyldithiomethyl (DTM); allyl levulinyl (Lev) and acetal levulinyl (ALE); 2-(2-nitrophenyl)propoxycarbonyl (NPPOC), α-methylnitropiperonyloxycarbonyl (MeNPOC), thioxanthone-nitrobenzyl group conjugates, and a 5'-O-dimethoxybenzoincarbonate group (DMBOC);
R₃ is a protecting group which is a levulinyl group (Lev);
R₅ is H, or a protecting group selected from the group consisting of 9-phenylxanthyl (pixyl or Px), MMTr, and DMTr;
Rₚ is a protecting group selected from the group consisting of methyl (Me), 2-cyanoethyl (CNEt), p-nitro-phenylethyl (NPE), and para- and ortho-chloro-phenyl (p- or o-ClPh);
R is lower alkyl, or the N(R)₂ moiety is a cyclic alkylamine, or a substituted cyclic alkylamine, preferably morpholine;
B is a nitrogen-containing base;
wherein each B, R₁ and Rₚ may be the same or different from any other B, R1 and Rp, respectively
and wherein
the term "lower alkyl" as used herein refers to acyclic, straight or branched chain alkyl groups containing from one to six carbons.

In another embodiment, n is selected from 1, 2, or 3.

The term "lower alkyl" as used herein refers to acyclic, straight or branched chain alkyl groups containing from one to six carbons. Preferred lower alkyl groups include, for example, isopropyl, methyl, and ethyl.

The functional groups of the compounds disclosed herein may be protected by a variety of protecting groups specified in the claims, that are known to those of skill in the art. A "protecting group" is used in the conventional chemical sense to reference a group which reversibly renders unreactive a functional group under specified conditions of a desired reaction. Some protecting groups are well known to one skilled in the art. Examples of the protection/deprotection process as well as various protecting groups are described in Wuts and Greene, 2006, Greene's Protective Groups in Organic Synthesis, Wiley-Interscience, New York, NY. After the desired reaction, protecting groups may be removed to deprotect the protected functional group. All protecting groups should be removable (and hence, labile) under conditions which do not degrade a substantial proportion of the molecules being synthesized. In contrast to a protecting group, a "capping group" permanently binds to a segment of a molecule to prevent any further chemical transformation of that segment. It should be noted that the functionality protected by the protecting group may or may not be a part of what is referred to as the protecting group.

Protecting groups for R₁ for the compounds of formula (II) above and the various compounds below are selected from:
1. Fluoride labile protecting groups: t-butyldimethylsilyl (TBDMS), triisopropylsilyl (TIPS); triisopropyloxymethyl (TOM); cyanoethylmethyl (CEM); 2-(4-tolylsulfonyl)ethoxymethyl (TEM).
2. Acid labile groups: 2'-*O*-bis(2-acetoxyethoxy)methyl (ACE) orthoester ; 1-(2-fluorophenyl)-4-methoxypiperidin-4-yl (Fpmp); 1-(4-chlorophenyl)-4-ethoxypiperidin-4-yl (Cpep); 4-(N-dichloroacetyl-N-methylamino) benzyloxymethyl (4-MABOM); dimethoxytrityl (DMTr) and monomethoxytrityl (MMTr).
3. Reduction labile groups: 2-tert-butyldithiomethyl (DTM); allyl.
4. Base labile groups: levulinyl (Lev) and acetal levulinyl (ALE).
5. Photolabile groups: 2-(2-nitrophenyl)propoxycarbonyl (NPPOC), α-methylnitorpiperonyloxycarbonyl (MeNPOC) and thioxanthone-nitrobenzyl group conjugates and 5'-O-dimethoxybenzoincarbonate group (DMBOC).

Protecting groups for R₅ for the compounds of formula (II) above and in the various compounds below are selected from 9-phenylxanthyl (pixyl or Px), MMTr, and DMTr. Preferably, these protecting groups are used for R₅ when options 1, 3, 4, and 5 are used as protecting groups for R₁ as noted above.

The Rₚ group as shown for the compounds of formula (II) above and in the various compounds below may be methyl (Me), 2-cyanoethyl (CNEt), p-nitro-phenylethyl (NPE), and para- and ortho-chloro-phenyl (p- or o-ClPh).

Protecting groups for the 3'-hydroxyl position (R₃ for the compounds of formula (II) and for the various compounds described below) are levulinyl. Further protecting groups including ionic protecting groups, also referred to as ionic tags, are described for comparative purposes only. Ionic tags are known to those of skill in the art. These may include those described in PCT Application Publication No. WO 2006/096963 of Chan, T.-H. et al. Ionic tags may include, for example, imidazolium and phosphonium ionic moieties having linkers selected from alkyl linkers, glycol linkers, etc.

The entity B in the compounds of formula (II) above and in the compounds described below is a nitrogen-containing base, preferably a base or a protected-base (also referred to herein as a "nucleobase"). The base is preferably a purine or pyrimidine base or analog thereof. Analogs include diaminopurine and its derivatives, inosine and its derivatives, alkylated purines or pyrimidines, acylated purines or pyrimidines, thiolated purines or pyrimidines. More specific analogs include, for example, 1-methyladenine, 2-methyladenine, N6-methyladenine, N6-isopentyladenine, 2-methylthio-N6-isopentyladenine, N,N-dimethyladenine, 8-bromoadenine, 2-thiocytosine, 3-methylcytosine, 5-methylcytosine, 5-ethylcytosine, 4-acetylcytosine, 1-methylguanine, 2-methylguanine, 7-methylguanine, 2,2-dimethylguanine, 8-bromoguanine, 8-chloroguanine, 8-aminoguanine, and the like. A "protected base" is protected on at least one nitrogen by any suitable N-protecting group including levulinyl, acetyl, difluoroacetyl, trifluoroacetyl, isobutyryl, benzoyl, 9-fluorenylmethoxycarbonyl, phenoxyacetyl, dimethylformamidine, N,N-diphenyl carbamate, and the like. Preferably, the base is selected such that the compound of formula (II) is a derivative of adenine (A), cytosine (C), guanine (G), or uracil (U).

Various aspects related to the practice of the present invention are disclosed in PCT International Application Publication No. WO 2009/064115 to Samchully Pharm. Co., Ltd.; Kumar, G. and Poonian, M.S. J. Org. Chem., 49, 4905-4912 (1984); Nemer, M.J, and Ogilvie, K.K., Can. J. Chem. 58, 1389-1397 (1980). Recent advances in RNA synthesis have been summarized in a review by S. Beaucage, Curr. Opin. Drug Discov. Devel. 11, 203-261 (2008)].

The use of previously reported dimer and trimer phosphoramidite synthons were directed at the synthesis of RNA oligomers, involving the use of such dimer or trimer units only in the first coupling reaction on solid supports (WO 2009/064115). Following coupling of one such dimer unit, the ensuing steps involved the exclusive coupling of monomeric phosphoramidite units until the desired length was produced (WO 2009/064115). This discourages the use of "various kinds of dimers" for the purpose of RNA synthesis, as this would require "long-term periods of synthesis and high production costs." The disclosure further emphasizes that it "employs just one dimer or trimer species only in the first coupling step and then common inexpensive monomer units in the subsequent steps, which enable the low-cost, high purity production of the nucleotide oligomers".

In contrast to the above disclosure, the RNA dimers and trimers presently described can be used exclusively or in combination with monomeric units. In addition to applications in solution-phase RNA synthesis, their use can also be extended to routine synthesis of RNA on conventional solid supports such as controlled pore glass and polystyrene. In this case, the RNA strand is assembled by several block couplings, cleaved and released from the support after synthesis and the resulting synthetic RNA utilized in physicochemical or biological studies. There are several derivatives that may be included for such block coupling reactions [see structures **(30), (47)** and **(50)** below]. The 2'-TIPS protecting group at the 3'-termini of such structures provides unique 5'-DMTr 3'-phosphoramidite dimer and trimer synthons for RNA synthesis.

Furthermore, the 2'-TIPS protected dimer and trimer synthons described herein provide several distinct advantages over previously reported 2'-TBDMS 3'-phosphoramidite dimer synthons (WO 2009/064115). The inventors have discovered that the use of TIPS completely eliminates 2' to 3'-isomerization that occurs with 2'-TBDMS protecting groups present in previously reported synthons (WO 2009/064115); in fact, the present disclosure teaches that when synthetic procedures are followed as described in WO 2009/064115, that 2'-TBDMS dimer synthons are not isolated in pure form, but rather as mixtures of 2'+3'-TBDMS regioisomers that are difficult (if not impossible) to separate under the specifications provided.

The methods described herein provide isomerically pure dimer and trimer synthons in high yields, and their use leads to high fidelity RNA synthesis. Such dimer and trimer synthons when coupled in solution or solid phase, allow longer chain extensions at each coupling stage of RNA synthesis, significantly reducing the total number of steps required in the synthesis of target RNA oligomers, and reducing their exposure to acidic environment. Additionally, dimer and trimer synthetic routes produce crude RNA oligomers that are generally more readily separated from the products of failure couplings. Thus the dimer and trimer block approaches potentially benefit critical aspects of siRNA manufacturing: speed and purification of synthesis, and the integrity of the desired full length RNA chain.

In another embodiment, there is provided a process for preparing a compound of formula (II): 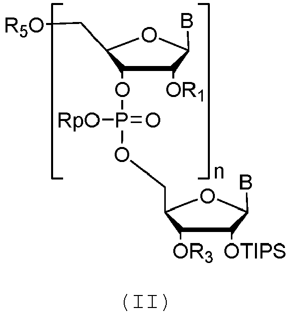 wherein
n is selected from 1, 2, or 3;
R₁ is a protecting group as defined above;
R₃ is protecting levuliny group;
R₅ is a protecting group as defined above;
Rₚ is a protecting group as defined above;
B is a nitrogen-containing base;
wherein each B, R₁ and Rₚ may be the same or different from any other B, R₁ and Rₚ, respectively;
the process comprising the steps of:
   a) condensing a phosphoramidite of formula (III): 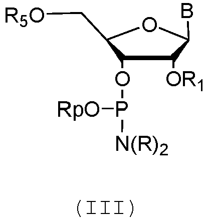
      wherein B, R₁, R₅, and Rₚ are as defined above; and
      R is lower alkyl, or the N(R)₂ moiety is a cyclic alkylamine, or a substituted cyclic alkylamine, preferably morpholine;
         with a nucleoside of formula (IV): 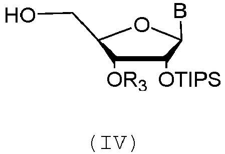
      wherein B and R₃ are as defined above; and
   b) oxidizing the product of step (a) to produce the compound of formula (II) where n is 1, and B, R₁, R₃, R₅, and Rₚ are as defined above; and
   c) where n>1, the process further comprising:
      (i) deprotecting the terminal -OR₅ group of the product of the previous step to form a free 5'-OH group;
      (ii) condensing the product of step (i) with a phosphoramidite of formula (III), wherein B, R₁, R₅, Rₚ and R are as defined above, and each B, R₁, R₅, Rₚ and R may be the same or different from any other B, R₁, R₅, Rₚ and R, respectively;
      (iii) oxidizing the product of step (ii);
      (iv) repeating steps (i)-(iii) n-2 times;
to form the compound of formula (II).

R₃ is a protecting group which is a levulinyl group. In another embodiment, the process further comprises removal of the R₃ protecting group.

In yet another embodiment, the process comprises phosphitylation of the terminal 3'-OH to form a compound of formula (IIa): 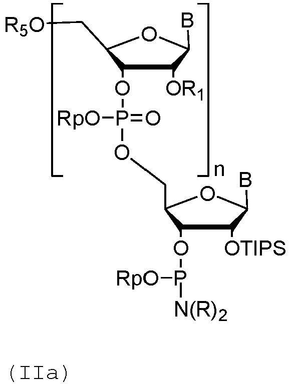 wherein n, B, R₁, R₅, Rₚ and R are as previously defined.

In yet another embodiment, there is provided a process for preparing a compound of formula (V): 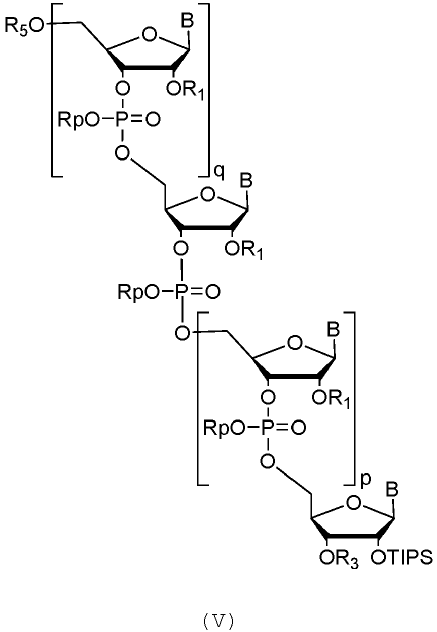 wherein
p is selected from 0, 1, or 2;
q is selected from 0, 1, or 2;
and p+q is less than or equal to 2;
R₁ is a protecting group as defined above;
R₃ is a protecting group which is a levulinyl group;
R₅ is a protecting group as defined above;
Rₚ is a protecting group as defined above;
B is a nitrogen-containing base;
wherein each B, R₁ and Rₚ may be the same or different from any other B, R₁ and Rₚ, respectively;
the process comprising the steps of:
   a) condensing a phosphoramidite of formula (VI): 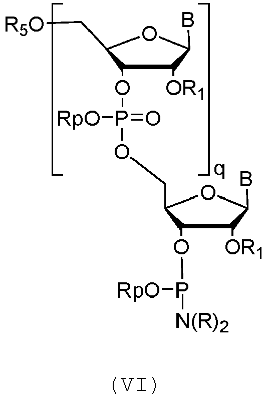
      wherein q, B, R₁, R₅ and Rₚ are as defined above, and
      R is lower alkyl, or the N(R)₂ moiety is a cyclic alkylamine, or a substituted cyclic alkylamine, preferably morpholine;
      with a compound of formula (VII): 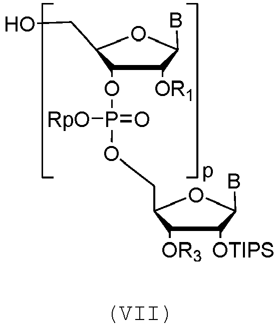 wherein p, B, R₁, R₅, and Rₚ are as defined above; and
   b) oxidizing the product of step (a) to produce the compound of formula (V).

In another embodiment, the process further comprises removal of the R₃ protecting group levulinyl.

In another embodiment of the above processes:
R₁ is TBDMS;
R₅ is DMTr or MMTr;
Rₚ methyl (Me), 2-cyanoethyl (CNEt), ortho-chlorophenyl (o-ClPh), or para-chlorophenyl (p-ClPh);
R is isopropyl, methyl, or ethyl; and
B is a nucleobase protected on at least one nitrogen by a suitable N-protecting group,
   preferably the N-protecting group is selected from levulinyl, acetyl, difluoroacetyl, trifluoroacetyl, isobutyryl, benzoyl, 9-fluorenylmethoxycarbonyl, phenoxyacetyl, dimethylformamidine, or N,N-diphenyl carbamate.

In another embodiment of the above processes, R₅ is DMTr, Rₚ is methyl, and R is iPr.

In various examples of the compositions and methods disclosed or described herein, some structures of starting materials, intermediates and products are shown below, along with their assigned compound numbers. Subject-matter of the invention are only the compounds defined in the appended claims, i.e. compounds wherein R3 is Lev.

| **#** | **R₅** | **R₃** | **R₂** |
|---|---|---|---|
| **1** | DMTr | H | H |
| **2** | DMTr | H | TBDMS |
| **3** | H | H | TBDMS |
| **4** | DMTr | Lev | TBDMS |
| **5** | H | Lev | TBDMS |
| **6** | DMTr | P(OCNEt)[N(*i*Pr)₂] | TBDMS |

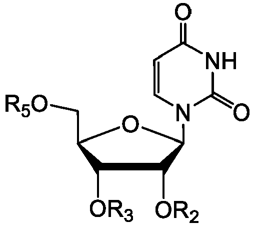

| | | | |
|---|---|---|---|
| **7** | DMTr | P(OMe))[N(*i*Pr)₂] | TBDMS |
| **8** | DMTr | TBDMS | H |
| **9** | H | TBDMS | H |
| **10** | DMTr | TBDMS | Lev |
| **11** | H | TBDMS | Lev |
| **12** | DMTr | H | TIPS |
| **13** | H | H | TIPS |
| **14** | DMTr | Lev | TIPS |
| **15** | H | Lev | TIPS |
| **16** | DMTr | TIPS | H |
| **17** | DMTr | TIPS | Lev |
| **18** | H | TIPS | Lev |

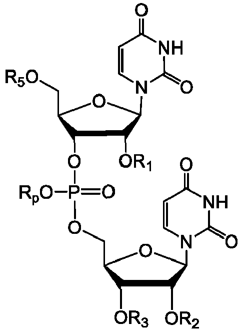

| **#** | **R₅** | **R₁** | **R₂** | **R₃** | **Rp** |
|---|---|---|---|---|---|
| **19** | DMTr | TBDMS | TBDMS | Lev | CNEt |
| **20** | DMTr | TBDMS | TBDMS | H | CNEt |
| **21** | DMTr | TBDMS | TBDMS | P(OCNEt) [N(*i*Pr)₂] | CNEt |
| **22** | DMTr | TBDMS | H | TBDMS | CNEt |
| **23** | DMTr | TBDMS | TBDMS | Lev | Me |
| **24** | DMTr | TBDMS | TBDMS | H | Me |
| **25** | DMTr | TBDMS | TBDMS | P(OMe) [N(*i*Pr)₂] | Me |
| **26** | DMTr | TBDMS | Lev | TBDMS | Me |
| **27** | DMTr | TBDMS | H | TBDMS | Me |
| **28** | DMTr | TBDMS | TIPS | Lev | Me |
| **29** | DMTr | TBDMS | TIPS | H | Me |
| **30** | DMTr | TBDMS | TIPS | P(OMe) [N(*i*Pr)₂] | Me |
| **31** | H | TBDMS | TIPS | Lev | Me |
| **32** | DMTr | TBDMS | Lev | TIPS | Me |
| **33** | DMTr | TBDMS | H | TIPS | Me |
| **34** | DMTr | TBDMS | TBDMS | H | H |
| **35** | DMTr | TBDMS | H | TBDMS | H |
| **36** | DMTr | TBDMS | TIPS | H | H |

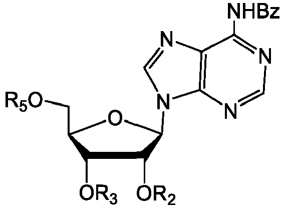

| **#** | **R₅** | **R₃** | **R₂** |
|---|---|---|---|
| **37** | DMTr | H | H |
| **38** | DMTr | H | TBDMS |
| **39** | DMTr | P(OMe) [N(*i*Pr)₂] | TBDMS |
| **40** | DMTr | H | TIPS |
| **41** | H | H | TIPS |
| **42** | DMTr | Lev | TIPS |
| **43** | H | Lev | TIPS |
| **44** | DMTr | P(OMe) [N(*i*Pr)₂] | TIPS |

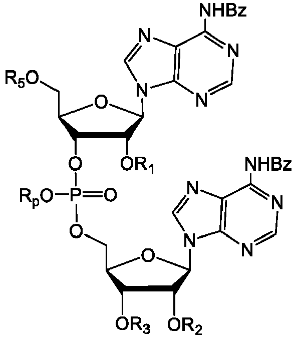

| **#** | **R₅** | **R₁** | **R₂** | **R₃** | **Rp** |
|---|---|---|---|---|---|
| **45** | DMTr | TBDMS | TIPS | Lev | Me |
| **46** | DMTr | TBDMS | TIPS | H | Me |
| **47** | DMTr | TBDMS | TIPS | P(OMe) [N(*i*Pr)₂] | Me |

| **#** | **R₃** |
|---|---|
| **48** | Lev |
| **49** | H |
| **50** | P(OMe) [N(*i*Pr)₂] |

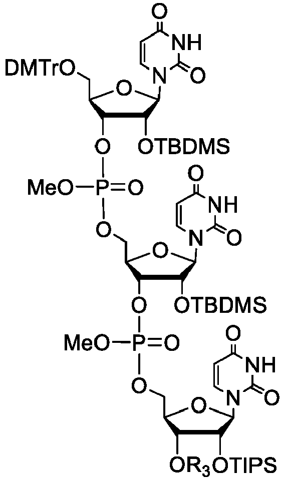

Preferred aspects include the synthesis and use of RNA dimers and trimers presented in the form of 2'-TIPS 3'-phosphoramidites. Examples of these are RNA dimers **(30)** and **(47),** and RNA trimer **(50)** shown below. Variations of these include dimers and trimer 2'-TIPS 3'-phosphoramidites of different nucleobase composition and sequence.

The blockmer (dimer, trimer, etc.) synthons described herein can be generated in a variety of ways. Non limiting examples of the synthesis of compositions useful for preparing N-mer oligoribonucleotides are illustrated in the synthesis schemes shown below.

While the Examples describe building blockmers via successive couplings of monomer units, it will be understood to those of skill in the art that such blockmers may be prepared via couplings between monomers, dimers, trimers, etc. and combinations thereof.

As well, while the present disclosure is largely directed to dimer and trimer preparations and their use in the preparation of N-mer oligoribonucleotides, it will be understood by those of skill in the art that larger blockmers (e.g., 5-, 6-, 7-, 8-, 9-, 10-, 11-, 12-, 13-, 14-, 15-, 16-, 17-, 18-, 19-, or 20-mers) useful in the preparation of N-mer oligoribonucleotides may be produced by the processes described herein.

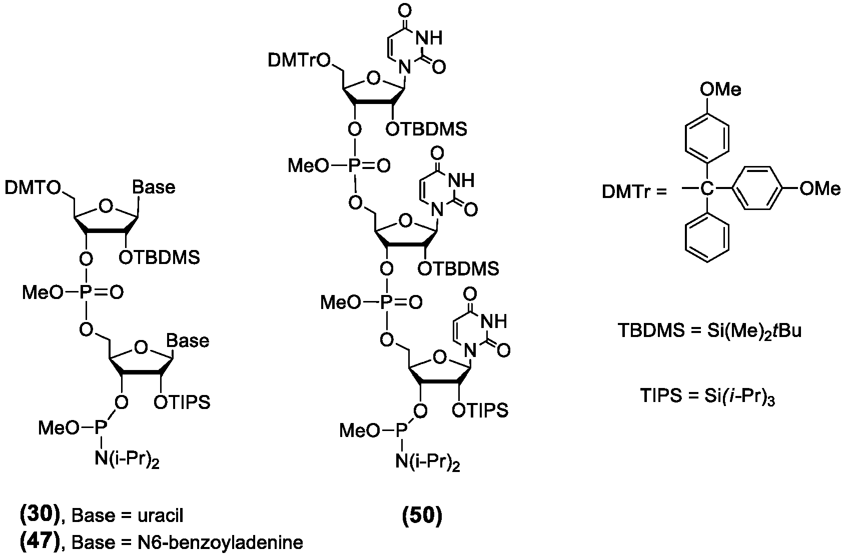

As well, the present disclosure illustrates the synthesis of a dimer block prepared from a 3'-nucleoside that is derivatized at the 3'-end with an orthogonally cleavable ionic tag such as (K) and (P).

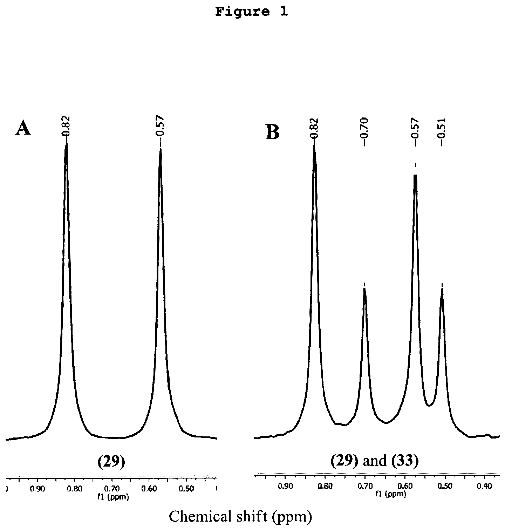

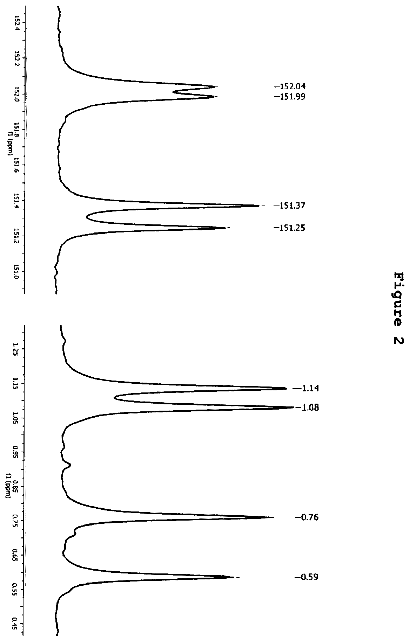

Scheme 1 illustrates a preferred synthetic method by which dimer 2'-TIPS 3'-phosphoramidite **(30)** can be generated. Detailed experimental procedures describing each of these steps are provided below. Both routes A and B (Scheme 1) produced over 80% of 2'-TIPS 3'-OH dimer **(29),** which were isolated free from its isomeric 3'-TIPS compound **(33)** (See ³¹P-NMR spectra; Figure 1). Phosphitylation of **(29)** afforded **(30)** in 67-88% yield. Again, no silyl isomerization was observed under the phosphitylation conditions. Compound **(30)** was isolated as a mixture of four P-diastereomers, and exhibited 8 peaks in its ³¹P-NMR spectrum as expected (4 diastereomers x 2 phosphorus ea = 8 peaks) (Figure 2). The chemical shifts observed are consistent with both phosphate (oxidation +5; 0-1 ppm) and phosphoramidite (oxidation +3; ca. 150-152 ppm) moieties being present in the molecule. The molecular weights of **(29)** and **(30)** were confirmed by mass spectrometry.

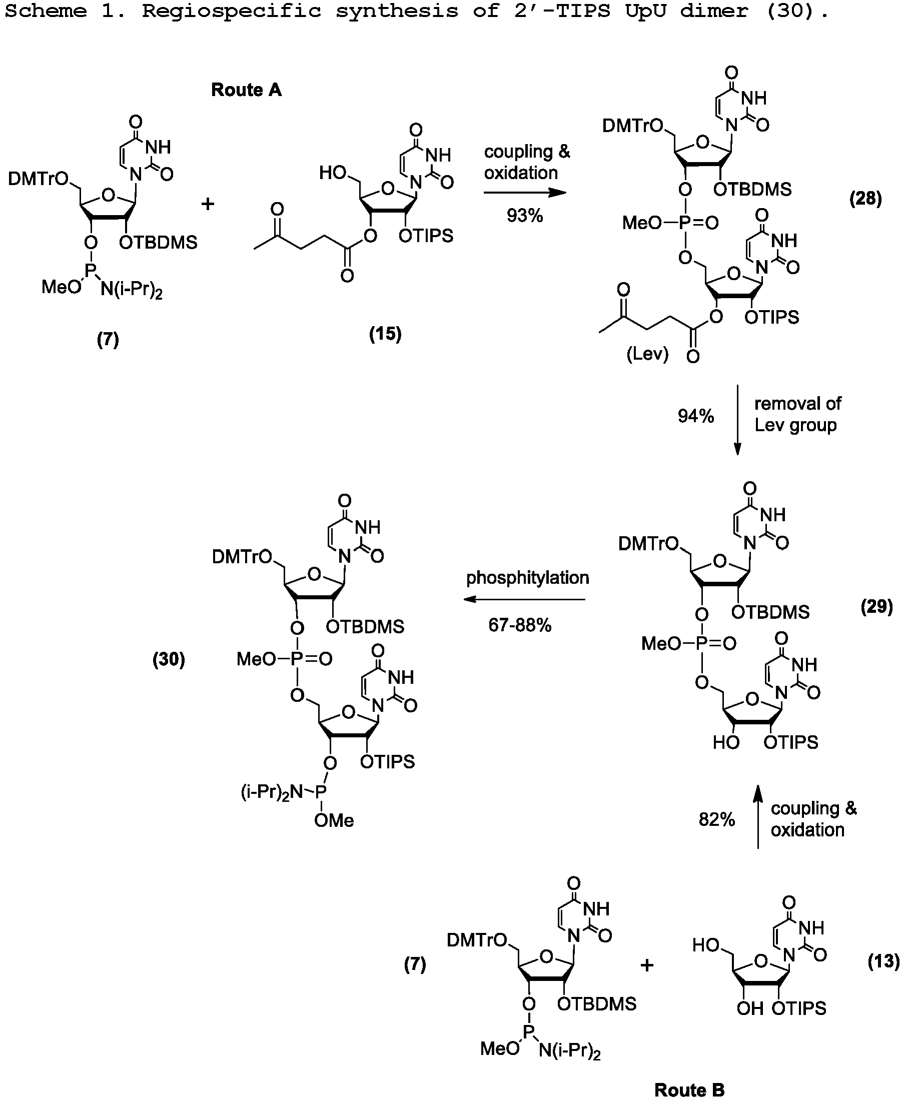

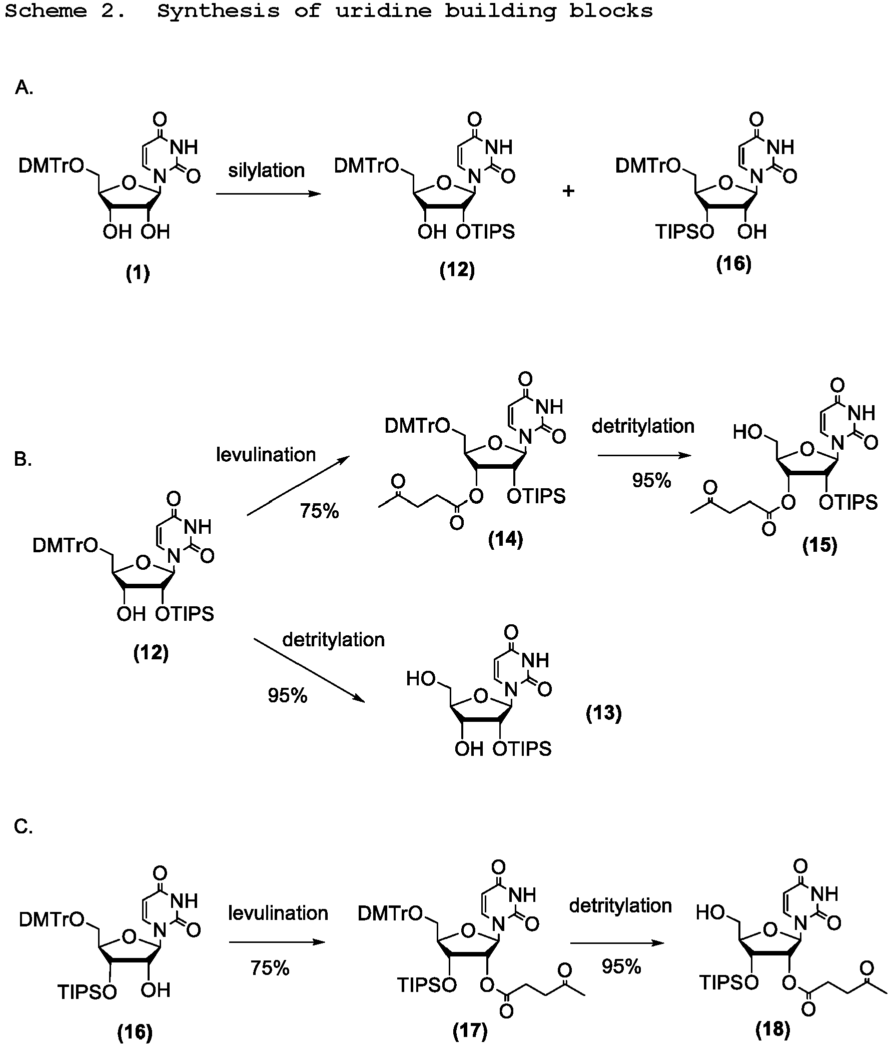

The required starting materials **(13)** and **(15)** were prepared as shown in Scheme 2. Thus, silylation of **(1)** with TIPSCl in the presence of pyridine/AgNO₃ afforded isomers **(12)** and **(16),** which were readily separated by silica gel column chromatograpy. The 2'-TIPS isomer was obtained in 66% yield, and the 3'-TIPS isomer in 20% yield. The compounds were characterized by MS and NMR. The position of silylation in isomers **(12)** and **(16)** was easily determined from 1D and 2D ¹H-NMR spectra obtained in DMSO-d6. J coupling of OH to H3' confirmed the position of the OH (C3'), and hence the site of silylation at C2'. This assignment was confirmed by ²⁹Si-¹H2' coupling observed via heteronuclear chemical shift correlation (HETCOR).

Compound **(14)** was obtained in greater than 70% yield by treating **(12)** with levulinic acid and dicyclohexylcarbodiimide (DCC) in THF (Scheme 2). As is the case for 2'-TBDMS protected nucleosides, isomerization of the TIPS group did not occur during this reaction. This was verified by the preparation of the isomeric compound **(17)** from the 3',5'-diprotected nucleoside **(16).** The 5'-dimethoxytrityl group was removed from **(12), (14)** and **(17)** using 3% trifluoroacetic acid (TFA) at room temperature for 5 min followed by addition of excess methanol. Yields on detritylation were 90-95%. The isomeric pair **(12)** and **(16)** had different chromatographic properties and were easily distinguished (see Examples).

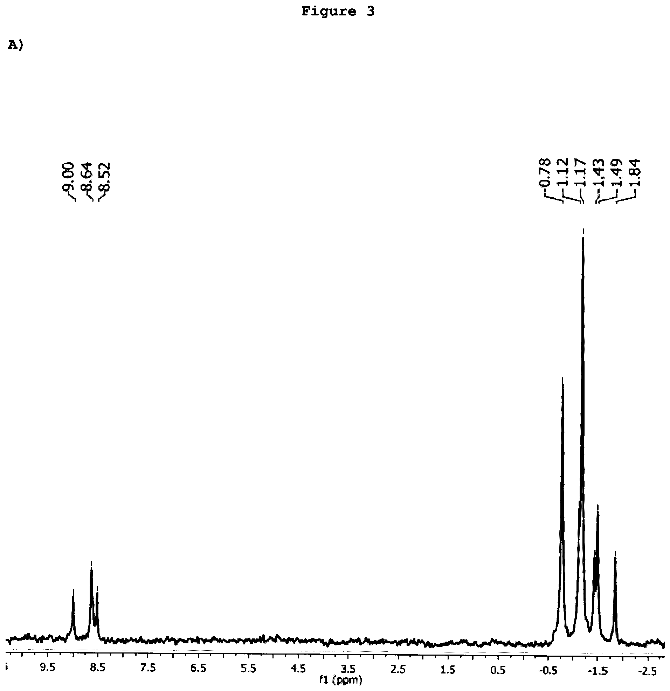

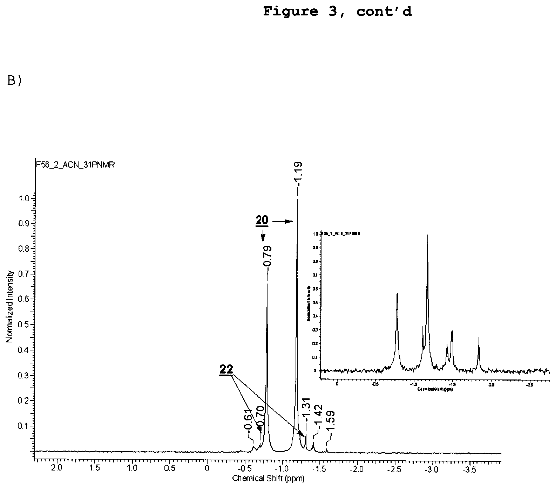

The use of TIPS instead of TBDMS is absolutely essential for obtaining dimers in isomerically pure form. We have discovered that 2'-TBDMS groups in dimers undergo O2' to O3' migration more readily those in nucleosides under various conditions. Thus, attempts to synthesize the 2'-TBDMS dimer synthon **(20)** reported in WO 2009/064115 produced material that was contaminated with small, but significant in the context of oligoribonucleotide synthesis, amounts of the 3'-TBDMS regioisomer (see Scheme 3; compound **(22);** **Figure 3A**). Furthermore, attempts to fully separate **(22)** from **(20)** by silica gel chromatography failed. Premature loss of the cyanoethyl (CNEt) phosphate protecting group from **(20)** (to give **34**) was another problem encountered during purification of this and other CNEt protected dimers. The ³¹P-NMR spectra of **(20)** before and after purification are shown in Figure 3B. It is clear that in addition to **(22),** other impurities contaminated compound **(20).** For example, the coupling of **(6)** with the 3'-hydroxyl group of **(3)** is expected to yield small amounts of 3',3'-linked UpU. As a result, isolated yields of (impure) **(20)** were moderate (67%). The contamination of **(20)** with the isomeric 3'-TBDMS dimer **(22)** will inevitably lead to 2'-+ 3'-phosphoramidite mixtures, and the introduction of both 2',5'- and 3',5'-internucleotide linkages in the final RNA sequence.

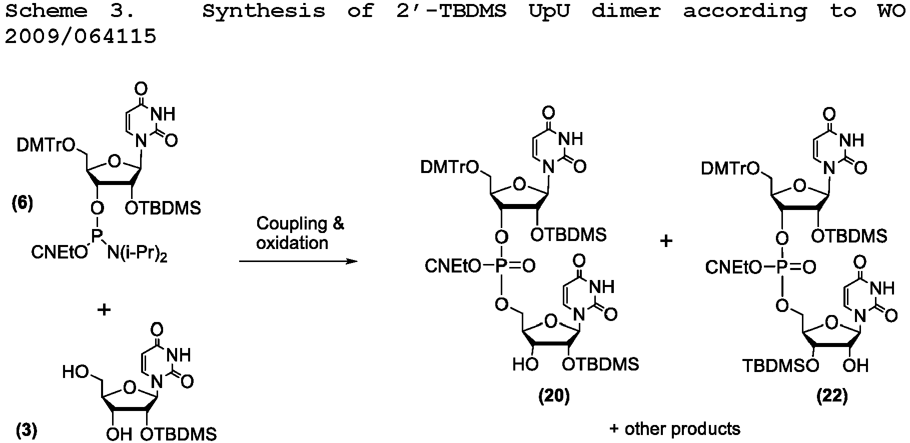

In an attempt to improve yields of TBDMS dimers while preventing silyl isomerization, dimer **(24)** was synthesized by modification of the procedures reported by Nemer and Ogilvie (Scheme 4) [Nemer, M.J, and Ogilvie, K.K., Can. J. Chem. 58, 1389-1397 (1980)]. To minimize loss of the phosphate protecting group, the methylphosphoramidite derivative **(7)** was used instead of **(6)** (Scheme 4). The starting materials **(5)** and **(11)** were prepared as previously described [Nemer, M.J. and Ogilvie, K.K., Can. J. Chem. 58, 1389-1397 (1980)].

Coupling of **(7)** with **(5)** followed by oxidation of the phosphite triester intermediate afforded the fully protected dimer **(23)** in 84% yield. ³¹P-NMR (Figure 4) and mass spectral data were consistent with its assigned structure. In the same manner, dimer **(26)** was prepared in 88% yield by coupling of **(7)** with **(11)** (Scheme 4, Figure 4).

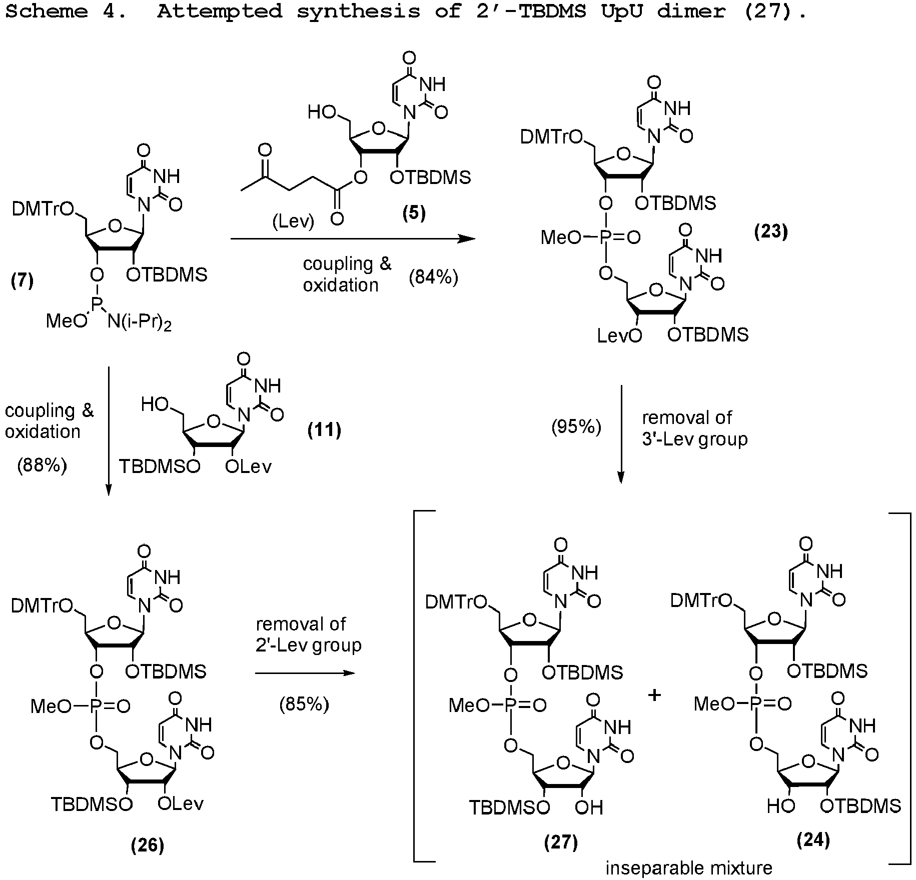

Attempts to remove the Lev group from either compound **(23)** or (**26)** without silyl migration under various conditions failed miserably (Table 1), yielding always mixtures of **(24)** + **(27)** (Figure 5). Once again, separation of these isomers by silica gel chromatography proved to be very difficult (Figure 6).

**Table 1. Conditions for removal of 3'-levulinyl group from compound (23). Experiments attempted are indicated by an "X", all of which lead to mixtures of (24) + (27) .**

| | | | | | | |
|---|---|---|---|---|---|---|
| **No. of equivalence of hydrazine4** | **1.5** | **1.5** | **2** | **2.5** | **10** | **10** |
| **Conc. of hydrazine (M)** | 0.1 | 0.5 | 0.2 | 0.2 | 0.1 | 0.5 |

| **Solvent Conditions** | | | | | | |
|---|---|---|---|---|---|---|
| Py:AcOH 3:2 | **X** | **X** | **X** | **X** | **X** | **X** |
| Py:AcOH 3:3 | | | | **X** | **X** | |
| THF | | | | **X** | **X** | |

| **Work-up Conditions** | | | | | | |
|---|---|---|---|---|---|---|
| Direct concentration of reaction mixture | **X** | **X** | **X** | **X** | **X** | **X** |
| NH₄Cl extraction | | **X** | | **X** | | **X** |
| Crude mixture placed directly on silica gel column | | | | **X** | | **X** |
| CuSO₄ extraction to remove pyridine | | | | **X** | **X** | **X** |

Reactions using hydrazine/pyridine/acetic acid were monitored by NMR (³¹P-NMR and ¹H-NMR) (Figures 7-14) to determine rates of both de-levulination and isomerization in TBDMS and TIPS dimers. The concentrations of hydrazine reagent used in these first set of experiments were half those used normally (0.5 M). Under these conditions, addition of hydrazine resulted in complete cleavage of the levulinyl group from dimer **(23)** in less than 2.5 min (³¹P-NMR experiments, Figures 7-9; Table 2). Detectable isomerization of the TBDMS group was observed within 20 min of mixing. This is high contrast to the TIPS dimer **(28),** which required 10 min for complete delevulination and was resistant to silyl isomerization for at least 68 hours under the same conditions. Neither TIPS or TBDMS monomers underwent isomerization (up to 10 h) under these conditions (Table 2).

The method of de-levulinylation of using hydrazine hydrate-pyridine-acetic acid poses an additional issue in assurance of the yield of the 2'*-O-*Silyl dimers (**23)** and (**28)**. In the process of removing 3'-*O*-Lev protecting group from the fully protected dimers, the internucleotide methyl phosphate protecting group of these compounds was labile in the presence of the potent nucleophile hydrazine hydrate. As shown in Figures 7 and 9, the emergence of the demethylated product became apparent after 20 min in the case of the 2'-TBDMS protected dimer and 80 min in the case of the 2'-TIPS protected dimer. As the internucleotide phosphate undergoes demethylation process, the resulting side product loses its original chirality around the phosphorus center, manifesting only one peak for each of the 2' and 3' isomers as opposed to two peaks for the methylated internucleotide phosphate. The theory was supported by the observation of two peaks at an upper field (~-1.00 ppm) of methylated products in TBDMS protected dimer (Figure 7), denoting two de-methylated side products with either 2'-TBDMS dimer (**34**) or the isomerized 3'-TBDMS dimer (**35**). In the case with the TIPS protected dimer, the presence of a single peak at around -1.00 ppm confirmed the previous observation that no isomerization has been detected, since only one demethylated product (**36**) corresponding to the isomerically pure product was noted (Figure 9).

**Table 2. Susceptibility of rU monomer and dimers towards hydrazine hydrate**

| | **TBDMS** | | | **TIPS** | |
|---|---|---|---|---|---|
| **Compound** | **rU (2)** | **rU (2)** | **UpU (23)** | **rU (12)** | **UpU (28)** |
| **NMR experiment** | ¹H | ¹H | ³¹P | ¹H | ³¹P |
| **Amount (mg, mmol)** | 50 (0.066) | 50 (0.066) | 53 (0.044) | 57 (0.071) | 50 (0.041) |
| **Concentration (M)** | 0.100 | 0.100 | 0.047 | 0.099 | 0.045 |
| **D-choroform (mL)** | 0.5 | 0.5 | 0.5 | 0.5 | 0.5 |
| **Hydrazine hydrate (eq.)** | 10 | 5 | 5 | 5 | 5 |
| **Hydrazine hydrate (µL,M)** | 32 (1.0) | 16(0.50) | 11 (0.24) | 17 (0.50) | 20 (0.23) |

| **Results** | | | | | |
|---|---|---|---|---|---|
| **Delevulinylation** | 5 min | 10 min | 2.5 min | 10 min | 5 min |
| **Detection of isomerization** | 2.3 hours | Non-detectable up to 11 h | 20 min | Non- detectable up to 9.3 h | Non-detectable up to 68 h |
| **Demethylation** | -- | -- | 80 min | -- | 20 min |

We then carried out a comparison between the extent of isomerization after work up and column chromatography as it was postulated that the conditions used during work up might have led to the O-2' and O-3' isomerization when using TBDMS protecting groups. This time we used the normal concentrations for the hydrazine solution (0.5 M). The experiment was performed as follows:
To a solution of UpU dimer (0.100 g, 0.0811 mmol, limiting reagent) in acetonitrile (1 mL) was added 0.811 mL of 0.5 M hydrazine hydrate in a mixture of pyridine and acetic acid (3:2). The resulting mixture was allowed to stir for the appropriate amount of time. Subsequently, the reaction was worked up by cooling the flask mixture to 0 °C in an ice bath and adding 5 equivalents of 2,4-pentanedione (0.042 mL, 0.41 mmol) to quench excess hydrazine. The mixture was allowed to return to room temperature after 10 minutes of stirring out of the ice bath. The mixture was then diluted with ethyl acetate and the organic layer was extracted four times with 5% ammonium chloride which was used to keep the aqueous layer slightly acidic, then once with brine to remove any residual ammonium chloride. The organic layer was then dried over with magnesium sulfate.

As shown in Figure 10, after 10 minutes of de-levulinylation, there is a detectable amount of isomerization in the case of TBDMS, but no measurable amount was observed for TIPS protected dimers. After 42 hours of reaction, about 40% de-levulinylated product was isomerized in the case of TBDMS, but only a miniscule amount of silyl migration occurred in TIPS protected product. The comparison has shown once again that the utilization of TIPS is significantly more promising for obtaining isomerically pure product within the time required for the completion of the de-levulination reaction under these conditions (2.5 minutes for TBDMS and 5 minutes for TIPS). Since removal of the levulinyl group of TIPS dimer requires 5 min or less, there is obviously no problem with isomerization or demethylation during the conversion of **(28)** to **(29)** (Scheme 1) and none has been detected.

In summary, the studies have established that the process described in WO 2009/064115 as outlined in Scheme 3 produces a mixture of 2'-TBDMS + 3'-TBDMS dimer synthons (i.e. **(22)** + **(23)** that were not possible to separate (Table 3). Even when alternative approaches were used to synthesize such 2'-TBDMS dimers (e.g., Scheme 4), the 2'-TBDMS dimers underwent isomerization to produce an inseparable mixture of 2'-TBDMS and 3'-TBDMS regioisomers. Our studies have also shown that TBDMS groups are significantly more prone to O2' to O3' isomerization in dimer than in monomer units (Table 2; Figures 7-14). We have discovered that while TIPS group can undergo migration (e.g. in pyridine/water; aqueous ammonia; methanol, etc), they are remarkably resistant to isomerization in a medium of pyridine/acetic acid/water containing hydrazine, and sufficiently resistant under conditions employed during removal of the levulinyl group, phosphitylation reactions, and even nucleotide condensation, affording dimer blocks of high purity.

We believe that the TIPS group constitutes a major breakthrough in the protection of 2'-hydroxyl groups in dimer and trimer units. Furthermore, the series of procedures we have developed, and in particular the use of the 2'-TIPS protecting group at the 3'-terminus of dimer and trimer blocks, allow, for the first time, the synthesis of dimer and trimer 2'-TIPS/3'-phosphoramidite synthons and their use in solution and solid phase oligoribonucleotide synthesis. As shown below, our procedures are applicable to other dimer blocks and a trimer block as exemplified below for the synthesis of ApA and UpUpU 3'-phosphoramidites **(47)** and **(50),** respectively (Schemes 5-7). Detailed synthetic schemes and experimental procedures and characterization of these compounds are provided below.

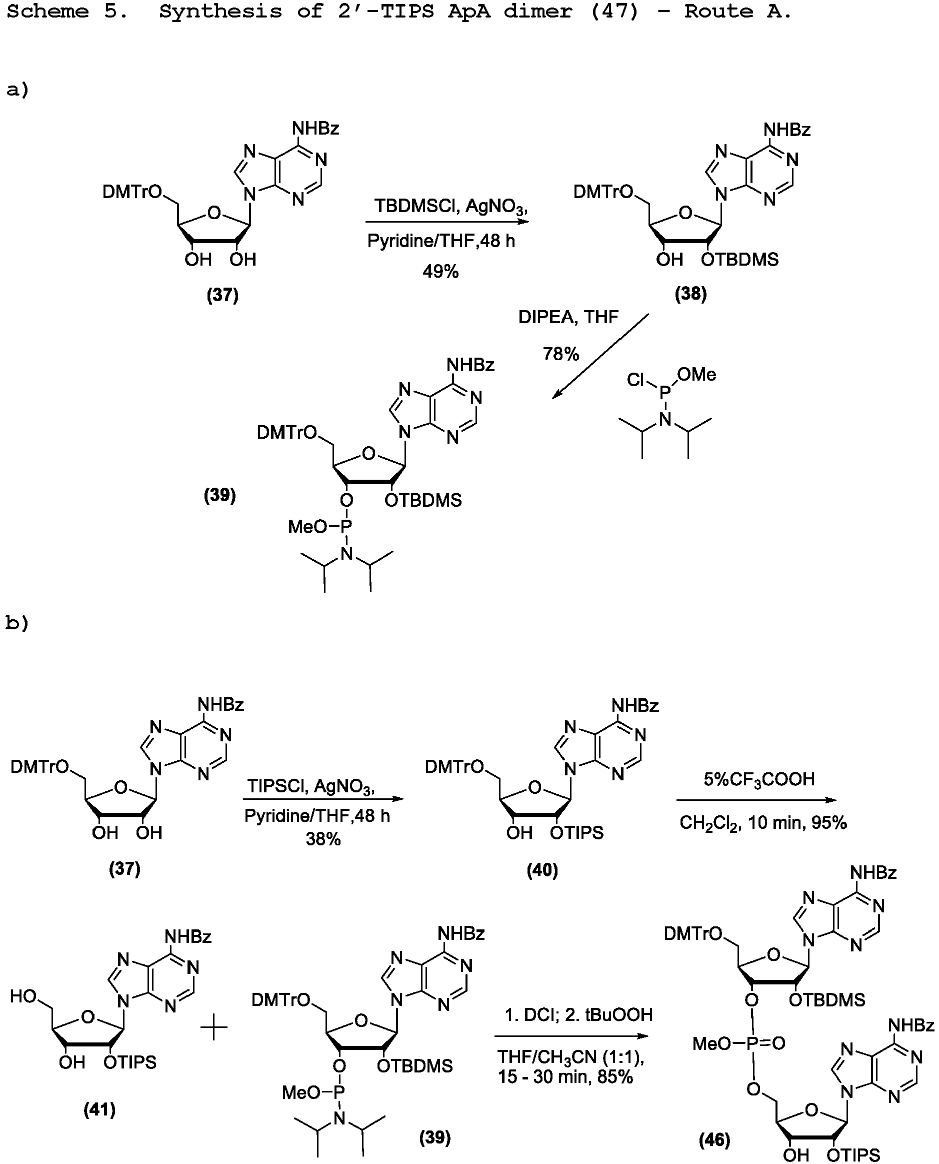

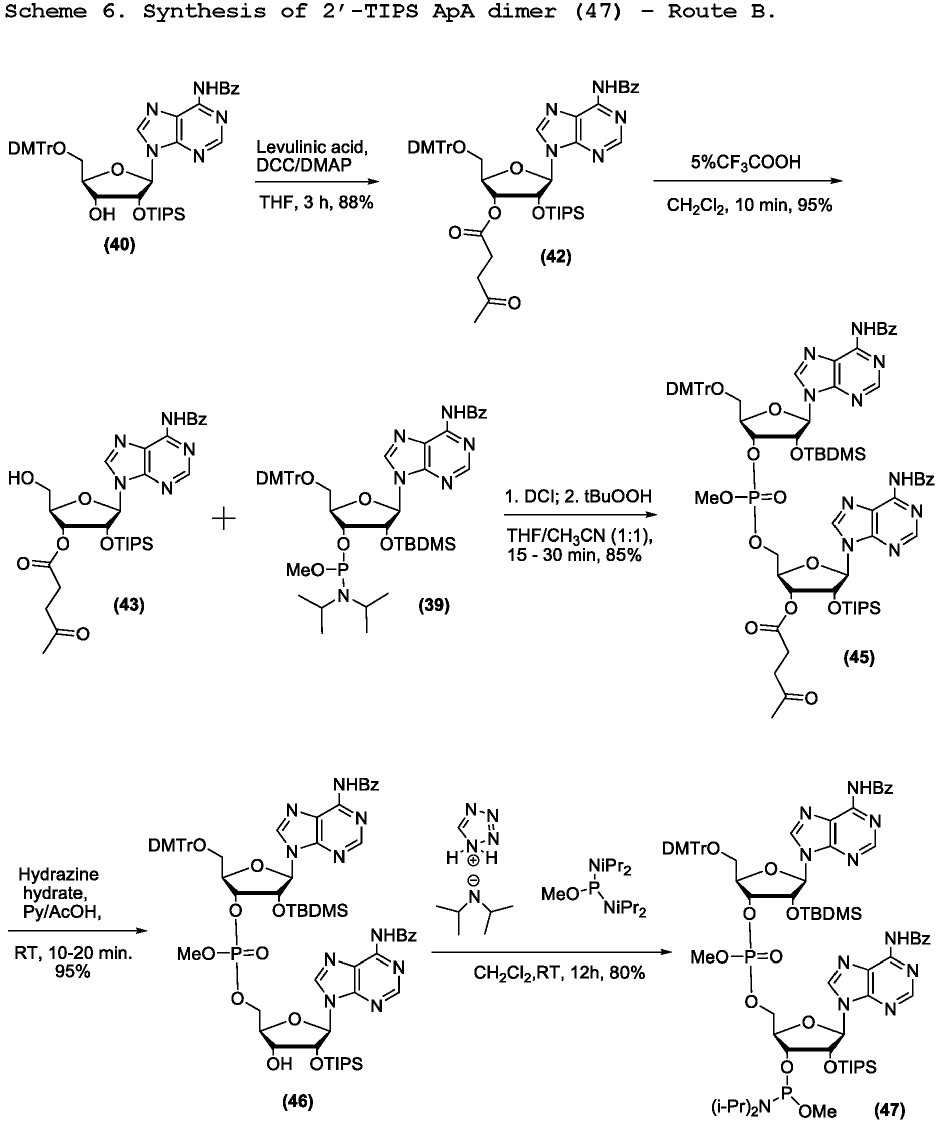

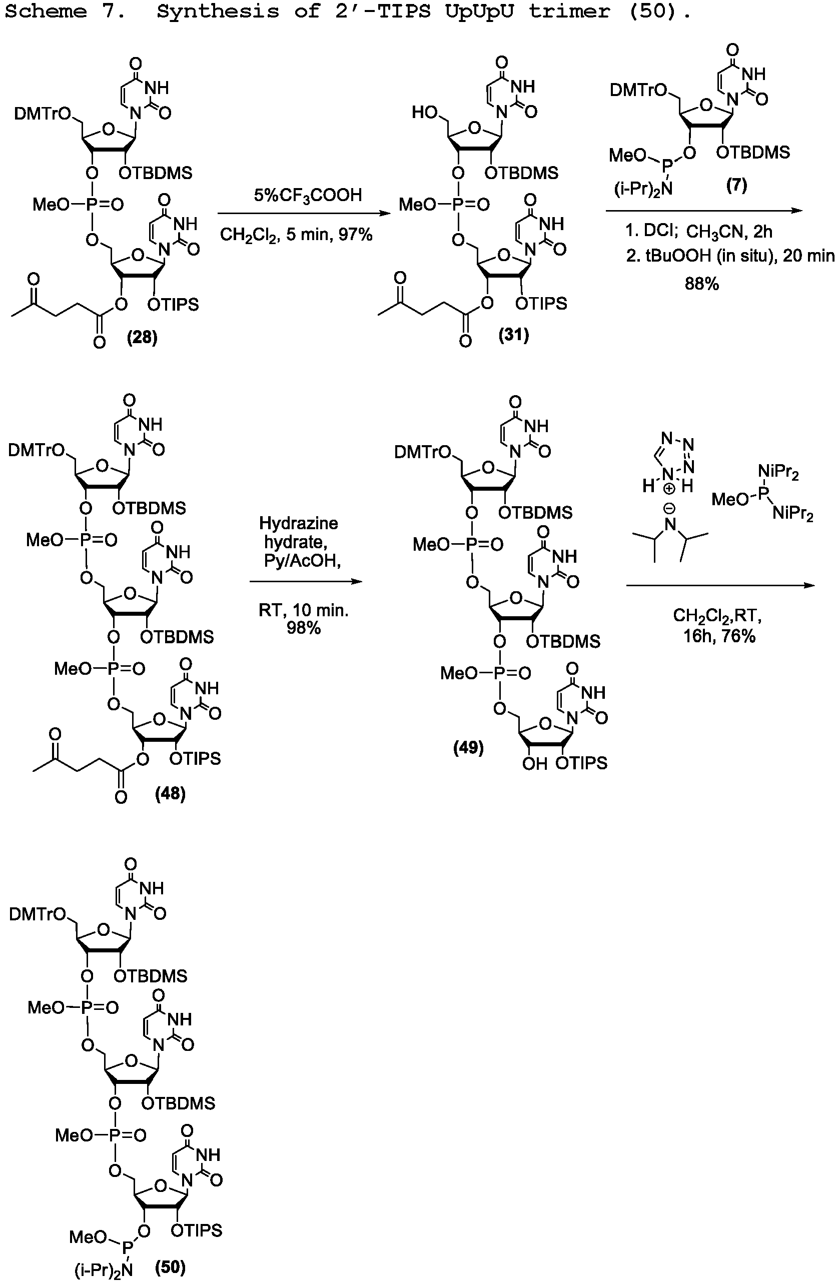

Compound **(50)** contains three chiral phosphorus centers and both Rp and Sp configurations are formed. As such, it is isolated as a mixture of 2³ = 8 P-diastereomers. Since each of these P-isomers contains three ³¹P nuclei, compound **(50)** is expected to display up to 24 peaks (3x 8-P isomers) in its ³¹P-nmr spectrum. Indeed, the spectrum shown in Figure 15 is consistent with this analysis. The fact that exactly two third of the peaks (and peak areas) appear in the P(+V)-phosphate region (ca. 0 ppm), with the remaining appearing in the P(+III)-phosphoramidite region (ca. +150 ppm), is fully consistent with the assigned structure. If migration of the TIPS group had occurred during synthesis and/or isolation of this trimer block, a mixture of 16 P-diastereomers (eight 2'-TIPS P-isomers + eight 3'-TIPS P-isomers) and hence doubling of the peaks would be expected (2x 24 = 48 peaks). As this was not observed, we conclude that the compound is isolated free from its 3'-TIPS regioisomeric form.

Scheme 1 (Route A) illustrates a preferred method for generating amidite blocks using nucleoside (**15**) as the 3'-unit. In another aspect, there is described a process for preparing amidite blocks from nucleosides containing an orthogonally cleavable ionic protecting group at C3', such as nucleosides K1, K2, K3, P4 and P5 shown below. The ionic tag in nucleoside K1, K2 and K3 contain a hydrazine cleavable gamma-ketoester moiety, whereas those in nucleosides P4 and P5 are photocleavable.

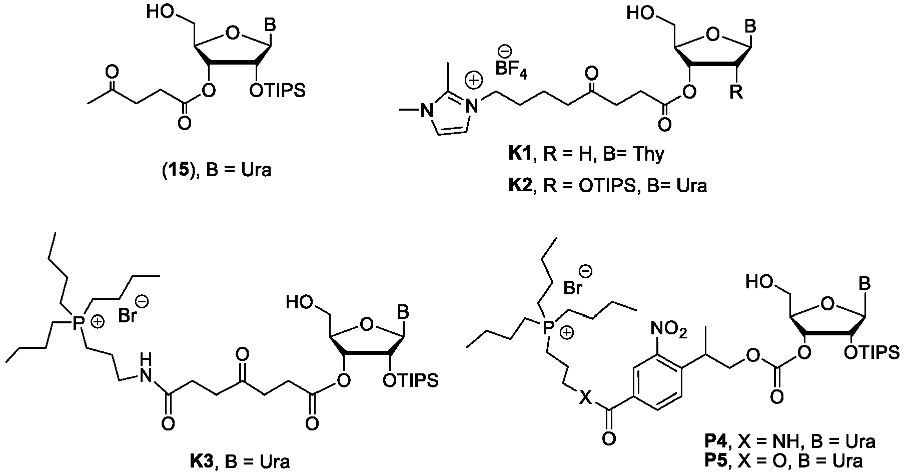

Non limiting examples of the synthesis of the ionic tags and their esterification with nucleosides are shown in Schemes 8 and 9. The ionic tags of nucleosides K1, K2 and K3, like the levulinyl group, require one of the mildest sets of cleavage conditions for selective removal from the other protecting groups used in RNA synthesis, i.e., a simple treatment with hydrazine hydrate in a pyridine and acetic acid solution. Furthermore, their ionic nature permits precipitation based purification of the nucleoside (e.g. compounds **(61)** or **(62),** Schemes 8-9), or if desired, di- and tri-nucleotides derived from such ion tagged nucleoside. Scheme 8 depicts a methodology to synthesize a 2'-deoxynucleoside with a hydrazine cleavable gamma ketoester tag, which facilitates isolation via precipitation step.

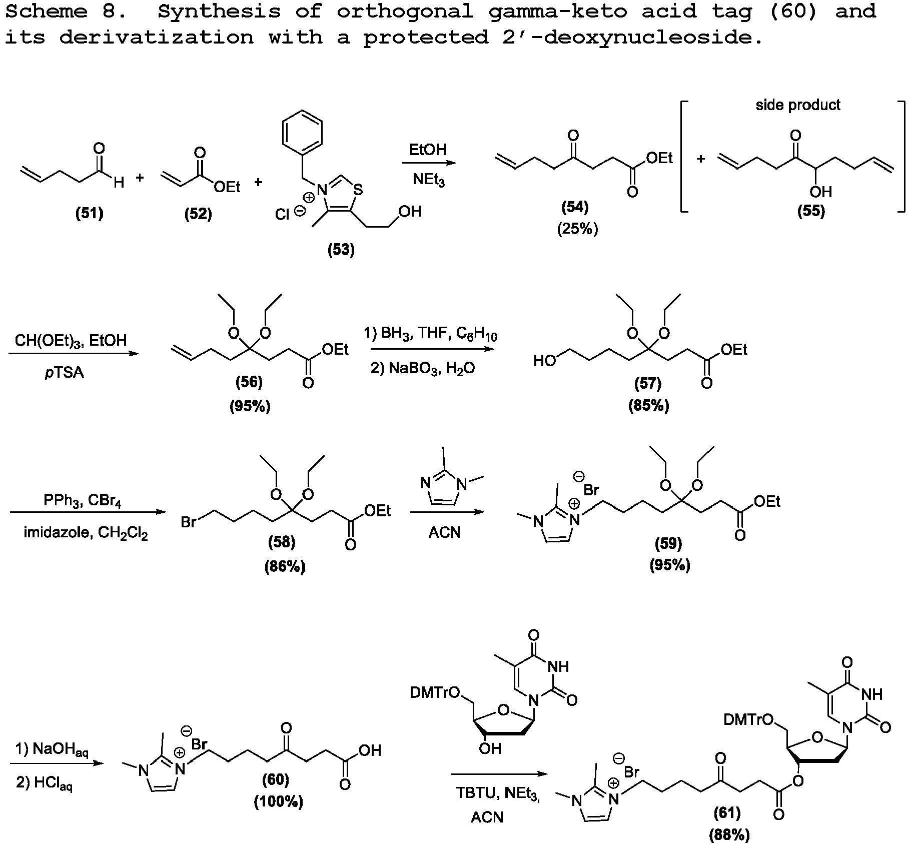

Upon selective removal of the tag, the nucleoside (or dimer or trimer block) can be converted into 3'-phosphoramidite derivatives, by the protocols described herein, that may be used in block condensations.

Several approaches can be applied to obtain the required gamma-ketoacid **(60).** For example, Scheme 8 employs a Stetter reaction as the first step [**(51)** + **(52)** → **(54)**], a reaction that transforms aldehydes into nucleophiles using catalytic amounts of a thiazolium salt, e.g. **(53),** in the presence of a mild base (Stetter, H. Angewandte Chemie, International Edition in English 15: 639-47 (1976)). Thus 4-pentenal **(51),** an aldehyde attached to an aliphatic chain terminating in an alkene, was activated as a nucleophile and allowed to undergo a Michael addition using ethyl acrylate **(52)** as the electrophile. The substrates were mixed with thiazolium salt **(53)** and dissolved in ethanol. The reaction mixture was heated and once it was refluxing gently, the reaction was initiated by the addition of triethylamine. After 18 hours the solvent was removed and the material obtained was subjected to a dichloromethane/brine extraction followed by flash chromatography. The desired product, **(54),** co-elutes with an acyloin side-product, **(55),** in all the solvent system investigated for the purification. The yield was approximately 25% (as estimated by ¹H-NMR analysis of the mixture), however, enough material can readily be obtained to continue with the synthesis, with the impurity **(55)** becoming easily separable after the subsequent step. The next step in the synthetic route was to protect ketone **(54)** as an acetal [i.e., **(56)**]. Refluxing for 4 hours was found to be adequate for this reaction to reach completion and the desired product **(56)** was obtained in 90-95% yield. The product (**56**) was easily separable from the acyloin side-product (**55**) generated in the earlier step, which did not appear to undergo any transformation in the acetal formation reaction. The identity and purity of **(56)** were confirmed by TLC, LR-MS and NMR.

With the gamma-acetal-ester, **(56),** in hand, the next step (Scheme 8) was the hydroboration of the terminal alkene. This was achieved using an *in situ* generated dicyclohexyl borohydride reagent. An appropriate amount of cyclohexene was added to borane-THF at 0 °C and allowed to react for one hour, then **(56)** was added to the resultant slurry and the reaction was allowed to proceed for 2 hours at room temperature. The oxidation of the intermediate borane was achieved by the addition of aqueous sodium perborate, a mild oxidant that left the ester intact, and the reaction was continued for another 2 hours. The reaction mixture was then extracted with ethyl acetate and the product, **(57),** was purified by flash column chromatography, providing a colourless liquid in 80-85% yield, with 15-20% of **(56)** also being recovered. This reaction never proceeded further than 85% conversion, even with longer reaction times or an increase in the amount of the borohydride reagent generated relative to substrate.

The primary alcohol, **(57),** was then mixed with triphenylphosphine and tetrabromomethane in DCM in order to generate the terminal bromide **(58).** The reaction proceeded cleanly but upon aqueous workup, acetal cleavage occurred, likely due to the formation of hydrobromic acid from the hydrolysis of the excess reagents. The reaction was performed again in the presence of imidazole, which neutralized any hydrobromic acid generated, and the desired product, **(58),** was obtained in 79-86% yield after flash chromatography. Subsequent condensation with 1,2-dimethylimidazole in acetonitrile resulted in the ionic tag, **(59),** in 95% or greater yield.

The final steps in the synthesis (Scheme 8) involved the cleavage of the acetal and ester as well as anion metathesis of the ionic moiety and finally derivatization to a nucleoside. Initially aqueous acid was used to effectuate the deprotection, followed by anion metathesis but the material thus derived decomposed rapidly. Subsequently the deprotection was performed via a two step process, the first a base mediated cleavage of the ester (59) followed by a limited acidification to protonate the carboxylic acid and cleave the acetal. The protected material, (59), was thus dissolved in aqueous sodium hydroxide and allowed to react for 16-18 hours followed by the addition of aqueous concentrated HCl to bring the solution to approximately pH 1 and finally drying under reduced pressure. The excess salt was removed by suspending the resulting solid in acetonitrile/dichloromethane and filtering off the insoluble material. The product obtained, **(60),** appeared pure by TLC, LR-MS and NMR but likely still contained a small amount of sodium chloride, which could not be detected with these techniques. Anion metathesis at this point also led to significant decomposition of the resulting product, as in the acid mediated deprotection. The bromide salt, **(60),** was stable upon long term storage, however, and was found to couple in the expected manner with a nucleoside. In fact, using O-(benzotriazol-1-yl)-N,N,N',N'-tetramethyluronium tetrafluoroborate (TBTU) as the coupling agent achieved both the coupling and anion exchange simultaneously, with the coupling proceeding quantitatively and the anion exchange confirmed by LR-MS as an absence of bromide ions and the presence of tetrafluoroborate in the negative mode. This product (**61**) was also stable upon long term storage.

With a derivatized nucleoside (**61**) in hand, the cleavage characteristics of the orthogonal gamma-ketoester ionic tag from the nucleoside were studied and compared to those of the levulinyl ester protecting group, which has been used as described above to generate oligomeric building blocks and is known to be easily removed without affecting any other protecting groups in the molecule. The rate of levulinyl ester cleavage under the conditions studied was faster than that of the ionic tag, with half lives of approximately 0.76 and 2.82 minutes respectively, corresponding to complete cleavage of the levulinyl ester in just over 5 minutes while the tag required almost 20 minutes for full cleavage. However, even though the tag cleaves more slowly than the levulinyl ester under the conditions studied, the normal reaction time employed for the cleavage of the 3'-hydroxyl protected levulinyl ester by hydrazine is 20 minutes and no degradation is observed in any of the other protecting groups in that time. This indicated that the ionic tag is suitable to the required task, and it is therefore a viable route to generating oligomeric building blocks as well.

The tag was also condensed with a 5'-O-DMTr-2'-O-TIPS Uridine nucleoside **(12)** (Scheme 9) using the same conditions employed for the derivatization of 5'-O-DMTr-Thymidine. The ¹H-NMR of the coupled ribonucleoside did not appear to show any 2'-3' silyl isomerisation.

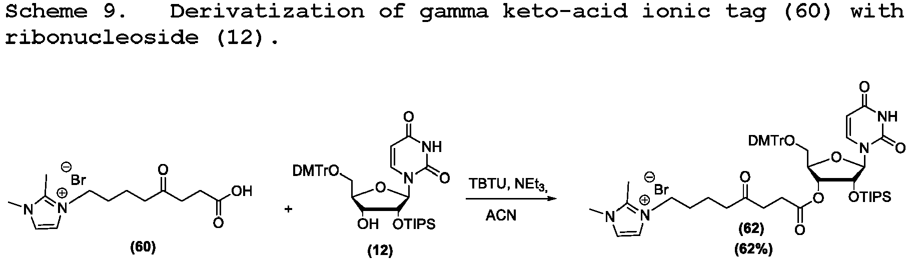

As an alternative to the gamma-ketoester linker **60,** a novel linker derived from ketopemilic acid was developed (Scheme 10).

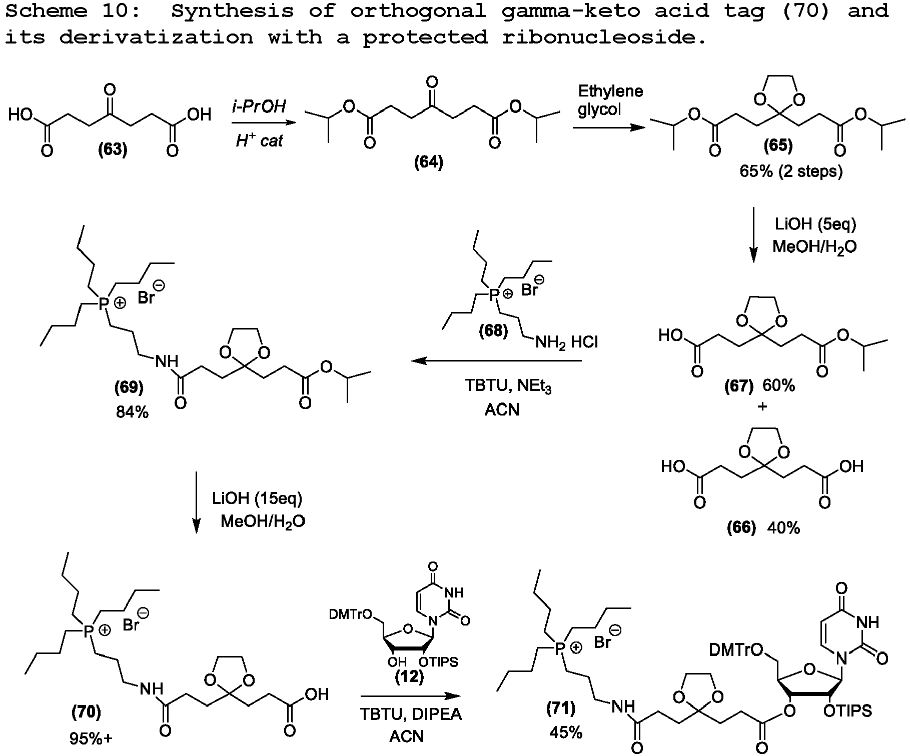

Its synthesis starts by treating ketopemilic acid (63) with *iso*propanol and catalytic para-toluenesulfonic acid using a Dean-Stark trap to esterify both terminal carboxylic acid moieties, affording **(64).** The use of methanol and ethanol (instead of isopropanol) in this reaction resulted in lactone formation as the major product, instead of the desired diester **(64).**

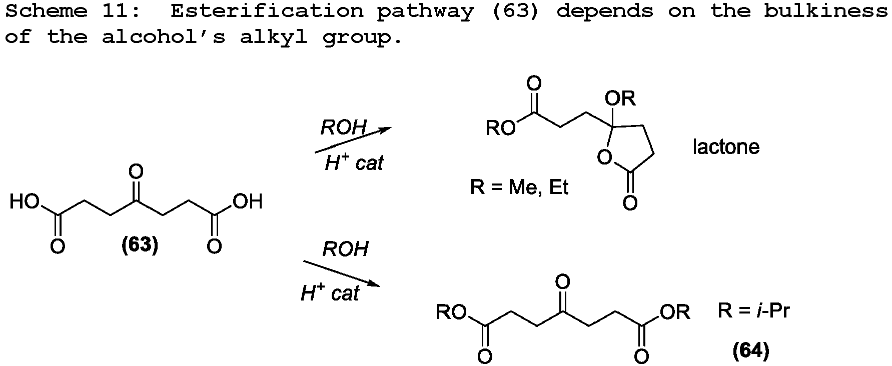

The diisopropyl ester **(64)** is isolated and then reacted with ethylene glycol and catalytic pyridinium para-toluenesulfonate by refluxing in a Dean Stark trap overnight to form the cyclic ketal **(65)** in 65% yield over two steps. Attempts to use ethylene glycol directly to form both the cyclic ketal and glycol ester resulted in the undesired lactone derivative (Scheme 11), necessitating that esterification and acetal formation be carried out in two separate steps.

Cyclic ketal diester **(65)** is taken up in methanol and 5 equivalents of aqueous lithium hydroxide is added to the reaction to preferentially hydrolyze one ester over both, producing a mixture of compounds **(66)** and **(67),** which are separated in yields of 40 and 60%, respectively. Coupling to the phosphonium ionic tag **(68)** to the cyclic ketal monoester **(67)** was accomplished using TBTU and DIPEA in acetonitrile at room temperature for 6h affording compound **(69)** in 84% yield. After isolation the isopropyl ester was hydrolyzed by LiOH in MeOH/Water to afford compound **(70)** in nearly quantitative yield. If in an alternative method, the diisopropyl ester **(64)** and **(65)** were not isolated after each step, it can be imagined that the conversion of **(63)** to **(66)** or **(67)** can be achieved in one pot.

Also, an alternative approach could be to selectively hydrolyze compound (**65**) to the di-acid (**66**), and then coupling this material with one equivalent of the phosphonium tag (**68**), potentially increasing overall yield and reducing the process by one step.

The ionic tag **(70)** is then conjugated to the 3'-hydroxy of ribonucleoside **(12)** using standard coupling conditions with TBTU and DIPEA to afford compound **(71)** in 4 h in a moderate yield of 45%. It is envisioned that mild acid hydrolysis of (**71**) would afford **K3** (Scheme 12).

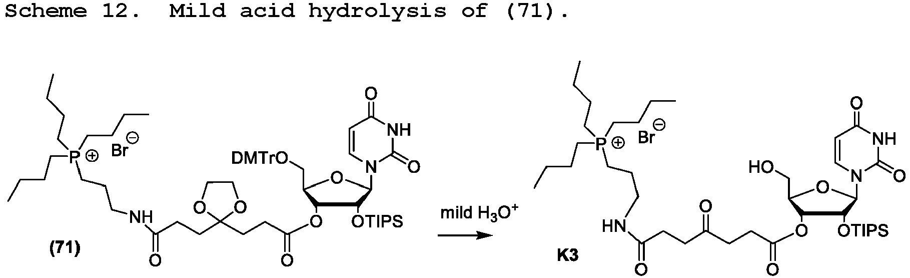

The ionic tag of nucleoside **K3** requires mild cleavage conditions for removal, that is, treatment with hydrazine hydrate in a pyridine and acetic acid solution. These conditions have been shown to be compatible with the protecting groups employed in RNA synthesis. Furthermore, and the ionic nature of the tag permits precipitation based purification of the nucleoside as carried out for compound **(71),** or if desired, of di- and tri-nucleotides derived from **(71).**

Alternatively, given that a tag containing gamma-keto ester is orthogonally cleavable, several other routes to analogous molecules are also possible. One approach to a similar molecule is shown in Scheme 13, and exploits the 5-bromolevulinyl derived ylid to attach the linker to the gamma-keto ester moiety as shown in Scheme 13 [Ronald, R. C.; Wheeler, C. J. Journal of Organic Chemistry 48: 138-9 (1983)]. This would also utilize many of the transformations already demonstrated for the Stetter approach depicted in Scheme 8. Indeed it may even be possible to link the tag in a single step with this approach, since compounds containing the ionic moiety and an aldehyde can be easily prepared.

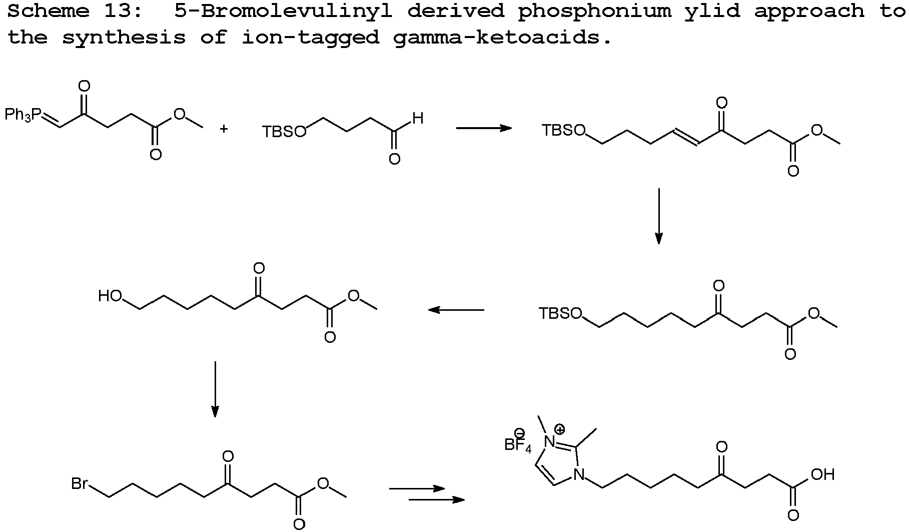

The hydrogenation of the resulting alkene would likely be necessary since it would be conjugated to the ketone and this conjugated system would likely react more slowly with hydrazine during the cleavage of the tag. It is understood that other ionic tags may replace the dimethylimidazolium tag shown, such as those described in PCT Application Publication No. WO 2006/096963 to Chan, T.-H. et al. Suitable ionic supports may include, for example, phosphonium ionic tags.

Another possible approach to the same substrate would be to use a more traditional Umpolung reaction than the Stetter approach, employing a dithiane derived from the same aldehydes as the nucleophile (Scheme 14).

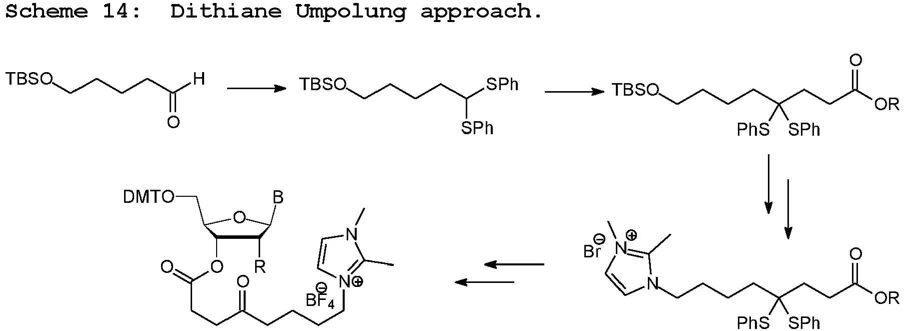

The same Michael acceptor could be used and this approach would eliminate the competing self-condensation reaction observed in the Stetter reaction [Scheme 8, product **(55)**]. The dithiane would also serve as the ketone protecting group for the bulk of the reaction and would likely be stable to the fluoride treatment employed for removal of the silyl ether (TBMS). This approach would also use many of the transformations used in the current invention, though the timing of the dithiane cleavage to liberate the ketone might be required prior to the installation of the ionic moiety, if the reagents used for this cleavage proved difficult to separate from the desired product. In that case, the dithiane could be cleaved immediately after the carbon-carbon bond formation and an acetal could then be installed.

An alternative to the orthogonal levulinyl linker described above is a light cleavable linker which can be removed in the presence of all standard ribonucleotide protecting groups. This allows for the use of extremely mild conditions to expose the 3'-hydroxyl group at anytime during the synthesis of blockers. In combination with the 2'-TIPS protecting group there is no risk of silyl migration, allowing for the production of regioisomerically pure blockmers which can be readily converted into phosphoramidies. The synthesis of the NPPOC like derivative **(80)** begins with a few short and elegant steps reported by Pfleiderer [Pfleiderer, W. et al. Helvetica Chemimica Acta, 87: 620 (2004)].

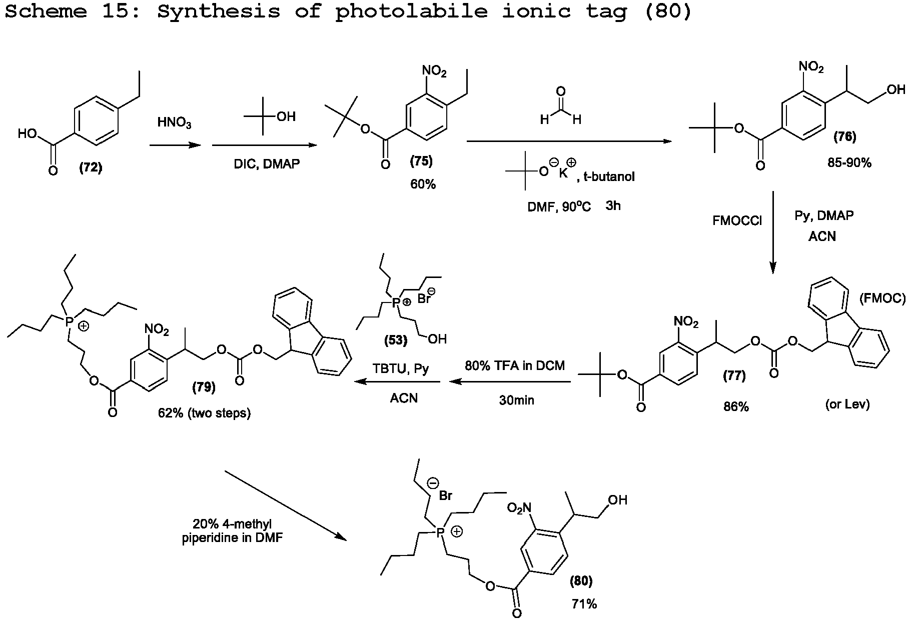

Fuming nitric acid was cooled to -10°C and 4-ethylbenzoic acid **(72)** was added over 30 min to the sitting solution, then allowed to stir for 30 min. The mixture was quenched over crushed ice and the solid precipitate of 3-nitro-4-ethyl-benzoic acid **(73)** was collected and crystallised with ethyl acetate and hexanes in good yield (95%). The tert-butyl ester was formed using DCC and DMAP with tert-butanol under standard conditions.

The formation of the 2-substituted propan-1-ol derivative was achieved as described by Pfleiderer by treating the tert-butyl ester with para-formaldehyde and a catalytic amount of potassium tert-butoxide in an aprotic dipolar solvent, such as DMF or DMSO, at 90°C for 3h [Pfleiderer, W. et al. Helvetica Chemimica Acta, 87: 620 (2004)J. The reaction is then quenched and neutralized to pH 7 with 1 M HCl, yielding 85-90% of the desired product **(76).** The newly formed primary hydroxyl is then protected with Fmoc-Cl **(77),** an acid stable protecting group. This allows cleavage of the t-butyl ester with 80% TFA in DCM without deprotection of the primary hydroxyl group. Without Fmoc protection, dehydration of the newly formed hydroxyl will occur, forming the propene derivative. As well, after the installation of the Fmoc it is imperative that the compound is not exposed to sunlight or tungsten light for long periods of time, as this compound will undergo photolytic cleavage, as per the design of the molecule.

The newly formed free acid is then coupled with phosphonium ionic tag **(53)** with TBTU in ACN and wrapped in aluminum foil for 8h which affords the tagged species **(79)** in a moderate yield of 65%, which can be easily separated from any starting material by column chromatography. Next, the Fmoc group is removed under standard conditions by treatment with 20% 4-methylpiperidine in DMF for 2h, yielding compound **(80)** in good yields. Although no protecting group can be removed at the primary alcohol, this compound should be kept in the dark at all times. This is due to the fact that it was observed that some degradation does occur over time, *albeit* much more slowly than when the Fmoc was present.

The previous synthesis of the light labile linker is somewhat long and requires the use of an expensive transient protecting group, Fmoc. In an attempt to shorten the synthesis we were able to avoid protection, deprotection, and re-protection of the carboxylic acid moiety, while increasing overall yields. This was accomplished by directly conjugating a modified phosphonium ionic tag **(68)** containing a primary amine in place of the hydroxyl group, creating an amide bond in compound **(81)** (Scheme 16). This was achieved as described for the synthesis of **(80)** (Scheme 15) using TBTU and triethylamine as coupling reagents, producing **(82)** in 35% yield (unoptimized).

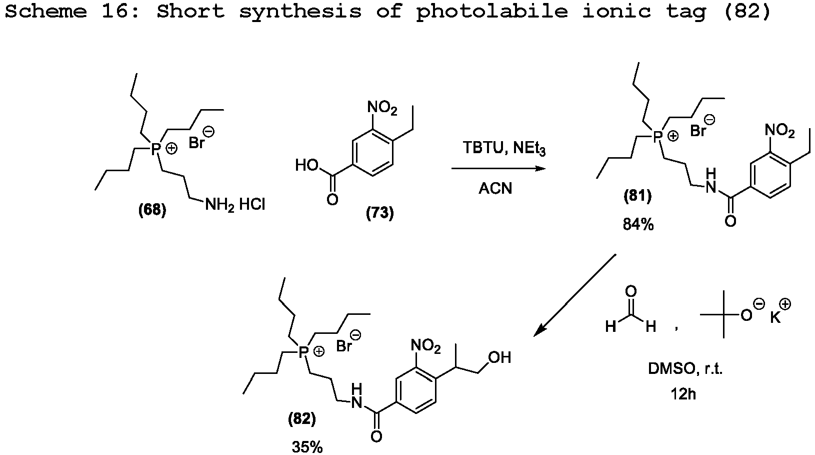

In another aspect, there is described a process for attaching ionic tag **(82)** to a ribonucleoside, affording a building block, **(85)** or (**86),** for further elaboration into oligonucleotides. The general method for carrying out the conjugation is shown in Scheme 16, and involves phosgenation of the ionic tag followed by its attachement to the 3'-hydroxyl group of the nucleoside.

Thus, phosgenation of **(80)** or **(82)** was carried out by a modified procedure from Eckert, H. Auerweck, J. Org. Process Res. Dev. 14: 1501-1505, (2010), and is outlined in Scheme 17. Detailed experimental procedures for generating phosgene from triphosgene and phenanthridine are described in the Experimental section (Examples).

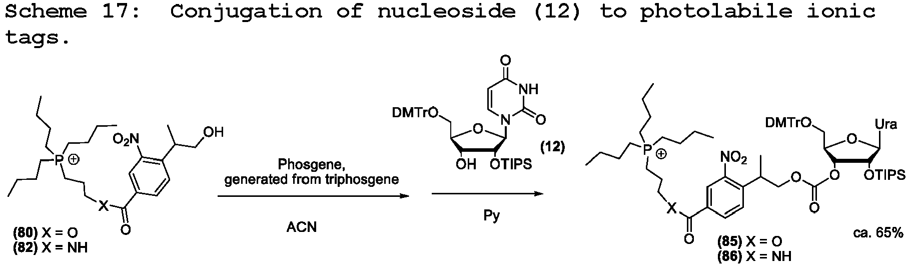

A solution of nucleoside (**12)** in acetonitrile is added directly to mixture of DIPEA and the phosgene generated above, and allowed to stir for 8 h at room temperature. After addition of ethyl acetate, the mixture is washed with sat. NaHCO₃ and brine, and precipitated in MTBE to remove excess (unreacted) nucleoside and DIPEA. The resulting precipitate is further purified by column chromatography in DCM:MeOH.

Nucleosides such as **(86)** have a number of applications. They can serve as starting materials for the synthesis of dimer or trimers or larger oligonucleotides requiring only a precipitation step for isolation by virtue of the polar ionic tag at the 3'-termini. Once the desired length has been synthesized, the 3'-tag and all protecting groups can be cleaved yielding the free (unprotected) oligonucleotide. Alternatively, because the 3'-ion tag can be selectively cleaved without deblocking all other protecting group on the heterocylic bases or sugar-phosphate backbone, it provides a novel mean for preparing protected oligonucleotide blocks containing a 3'-hydroxyl group that can be further elaborated to a 3'-phosphoramidite derivative.

These processes are exemplified in the synthesis of the tetranucleotide rAGCU starting from nucleoside **(86)** (Schemes 18 and 19). Reactions are carried out in the dark by wrapping the reactions flasks with aluminum foil, to avoid premature cleavage of its ionic photolabile tag.

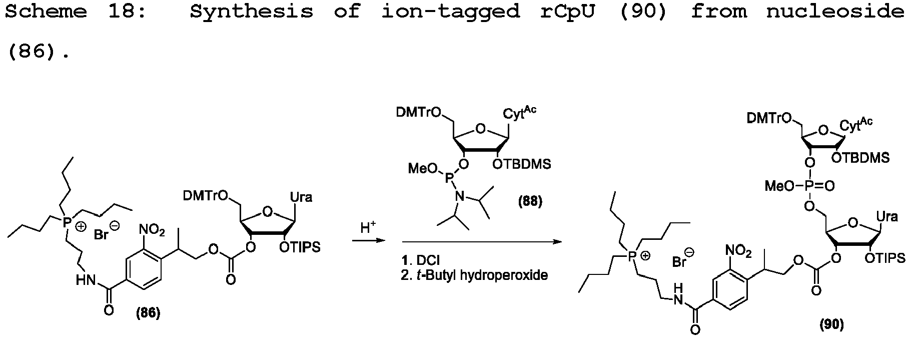

3'-Tagged-uridine **(86)** was detritylated by adding 3% trifluoroacetic acid in DCM and allowing the mixture to stir for 5 min. Addition of methanol ensures quenching of the trityl cation, preventing the re-tritylation of the 5'-hydroxyl group. The crude product is then precipitated in MTBE to remove DMTrOMe, and/or DMTrOH. The compound is then filtered over celite, collected in DCM and re-purified by column chromatography affording **P4** in 95% yield. The synthesis of dimer rCpU **(90)** from **P4** was carried out by coupling with rC phosphoramidite monomer **(88)** in the presence of 4,5-dicyanoimidazole (DCI), and the resulting solution was allowed to stir at room temperature for 3 h. Ten equivalents of tert-butanol was added to quench excess phosphoramidite, followed by 10 eq of tert-butyl hydroperoxide (1 mL of a 6 M solution in decane) to oxidize the internucleotide phosphite triester to the more stable phosphate triester. The reaction is then concentrated to an oil, taken up in minimal amounts of dichloromethane (DCM), and precipitated in MTBE to remove all excess reagents. The precipitation process is repeated if the presence of any quenched phosphoramidite is detected by TLC. Tagged rCU dimer **(90)** was isolated in 95% yield (0.63 g). The above process was repeated as described above, using the appropriate phosphoramidites till tetramer (**92)** was obtained. Full experimental procedures and characterization are provided in the Experimental section (Examples).

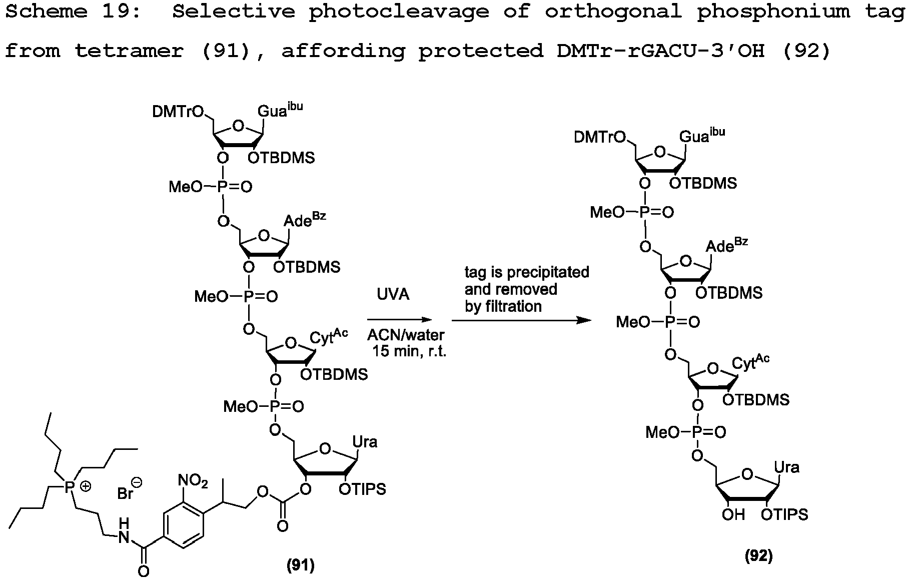

Tetramer **(91)** was dissolved in 1 mL of wet ACN (1200 ppm of H₂O), and transferred into a quartz cuvette. The cuvette was placed inside a photoreactor with stirring. The reaction was completed within 15 min (TLC analysis). The mixture was concentrated to about half volume and the cleaved tag removed by precipitation with methyl t-butyl ether (MTBE). The desired tetramer was found in the MTBE solution, which was collected after concentrating the solution to dryness. Isolated yield was 95% (92 mg). Phosphitylation of **(92),** as described previously, can provide the 3'-phosphoramidite derivative **(93)** that is suitable for RNA synthesis via block coupling.

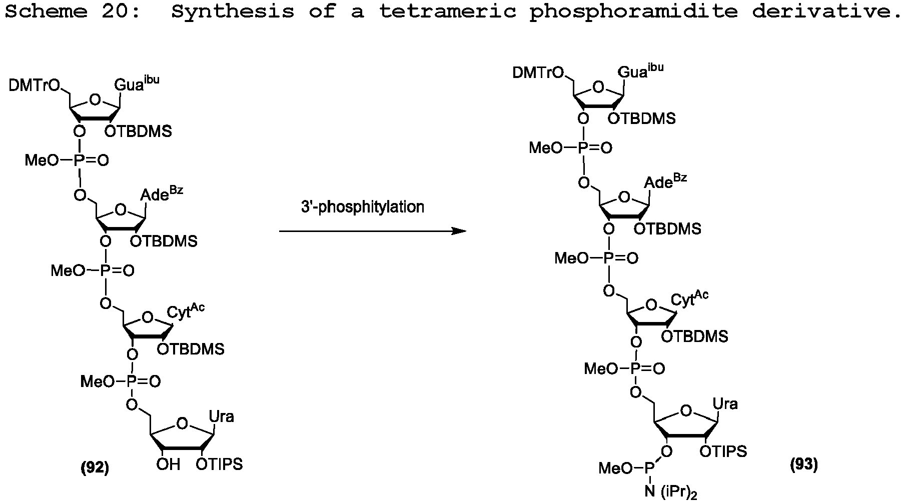

### Oligonucleotide Synthesis.

In another aspect, there is described a process for preparing an N-mer oligonucleotide, said process comprising:
a) condensing a phosphoramidite of formula (IIa): 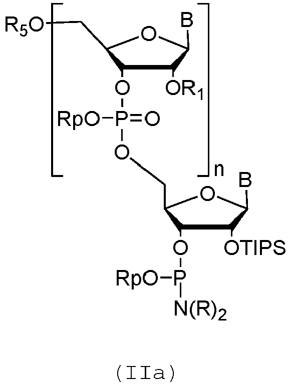 wherein
   n is an integer from 0 to 19;
   R₁ is a protecting group;
   R₅ is a protecting group;
   Rₚ is a protecting group;
   R is lower alkyl, or the N(R)₂ moiety is a cyclic alkylamine, or a substituted cyclic alkylamine, preferably morpholine;
   B is a nitrogen-containing base;
   wherein each B, R₁ and Rₚ may be the same or different from any other B, R₁ and Rₚ, respectively;
      with
      (a') a functionalized linker L bound to a support ⓢ, wherein ⓢ is selected from a solid support or an ionic support, or
      (b') a compound of formula 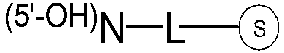 wherein ^{(5'-OH)}N is a nucleoside/oligonucleotide chain bound to the solid support or the ionic support via the functionalized linker L and having a free 5'-OH group;
b) oxidizing the product of step (a) to form a compound of formula (VIII): 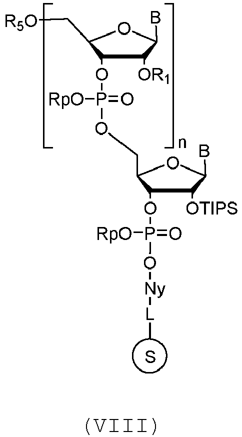 wherein
   y is 0 or 1;
   n, B, R₁, R₅ Rₚ, ⓢ, L, and N are as defined above; and
c) optionally,
   (i) deprotecting the terminal 5'-OR₅ group of the product of the previous step to form a free 5'-OH group;
   (ii) condensing the product of step (i) with a phosphoramidite of formula (IIa) 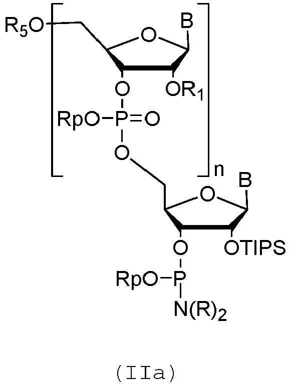 wherein n, B, R₁, R₅, Rₚ and R are as defined above, and each n, B, R₁, R₅, Rₚ and R may be the same or different from any other n, B, R₁, R₅, Rₚ and R, respectively;
   (iii) oxidizing the product of step (ii); and
   (iv) optionally repeating steps (i)-(iii);
with the proviso that if y is 0, step (c) is not optional;
to form a bound N-mer oligonucleotide.

In another aspect, y is 1 and N is a nucleoside.

In yet another aspect, 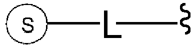 is

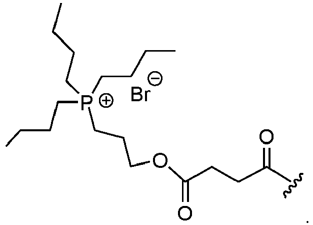

In still yet another aspect, there is described a process for preparing an N-mer oligonucleotide, said process comprising:
a) condensing a compound of formula (IIb): 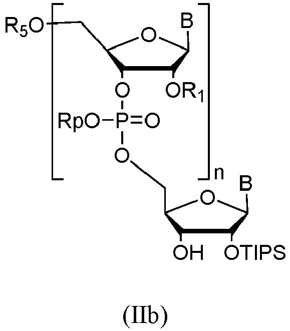 wherein
   n is an integer from 0 to 19;
   R₁ is a protecting group;
   R₅ is a protecting group;
   Rₚ is a protecting group;
   B is a nitrogen-containing base;
   wherein each B, R₁ and Rₚ may be the same or different from any other B, R₁ and Rₚ, respectively;
      with
      a functionalized linker L bound to an ionic support ⓢ,
b) oxidizing the product of step (a) to form a compound of formula (IX): 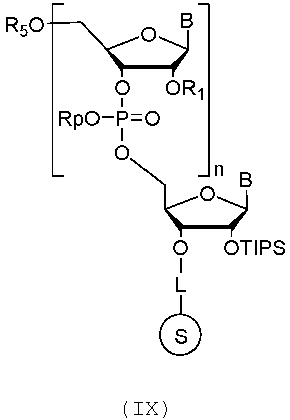 wherein
   n, B, R₁, R₅ Rₚ, ⓢ, and L, are as defined above; and
c)
   (i) deprotecting the terminal 5'-OR₅ group of the product of the previous step to form a free 5'-OH
      group;
   (ii) condensing the product of step (i) with a phosphoramidite of formula (IIa) 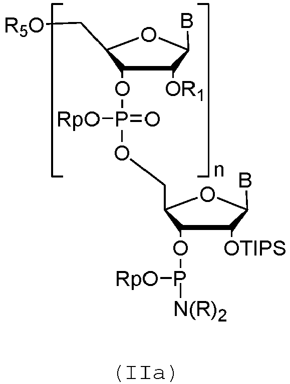 wherein n, B, R₁, R₅, and Rₚ are as defined above, and R is lower alkyl, or the N(R)₂ moiety is a cyclic alkylamine, or a substituted cyclic alkylamine, preferably morpholine; wherein each n, B, R₁, R₅, and Rₚ may be the same or different from any other n, B, R₁, R₅, and Rₚ, respectively;
   (iii) oxidizing the product of step (ii); and
   (iv) optionally repeating steps (i)-(iii);
to form a bound N-mer oligonucleotide.

In the above processes, n may be selected from 0, 1, 2, or 3.

In yet another aspect of the above processes:
R₁ is TBDMS;
R₅ is selected from DMTr or MMTr;
Rₚ is selected from methyl (Me), 2-cyanoethyl (CNEt), orthochlorophenyl (o-ClPh), or para-chlorophenyl (p-ClPh);
R is selected from isopropyl, methyl, or ethyl; and
B is a nucleobase protected on at least one nitrogen by a suitable N-protecting group,
   preferably the N-protecting group is selected from levulinyl, acetyl, difluoroacetyl, trifluoroacetyl, isobutyryl, benzoyl, 9-fluorenylmethoxycarbonyl, phenoxyacetyl, dimethylformamidine, or N,N-diphenyl carbamate.

In still yet another aspect of the above processes:
R₅ is DMTr;
Rₚ is methyl; and
R is iPr.

In another aspect of the above processes, the processes further comprise fully deprotecting the bound N-mer oligonucleotide to yield a free N-mer oligonucleotide. In another embodiment of the above processes, the N-mer oligonucleotide has a length from 4-100 ribonucleotides.

In another aspect of the above processes, 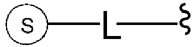 is selected from: 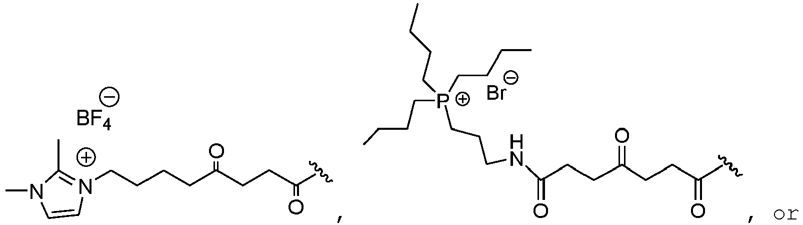 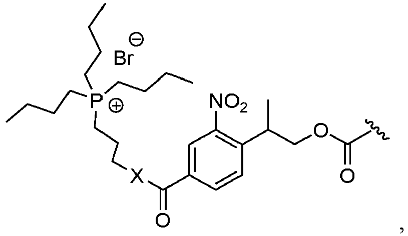 wherein X is selected from NH or O.

In yet another aspect, there is described an oligonucleotide prepared by the above processes.

Suitable solid supports for use in the above-mentioned processes are known to those of skill in the art and may include controlled pore glass (CPG) or long chain alkylamine CPG (LCAA-CPG); polystyrene, polyvinyl, and the like.

Suitable ionic supports are also known to those of skill in the art. These may include those described in PCT Application Publication No. WO 2006/096963 to Chan, T.-H. et al. Suitable ionic supports may include, for example, imidazolium and phosphonium ionic salts.

Functionalized linkers ("L" in formulae (VIII) and (IX)) are usually attached to the solid/ionic supports to space out the oligomer from the surface of the support, and suitable linkers are known to those of skill in the art. The most common ones used with CPG (controlled-pore glass) and highly cross-linked polystyrene (PS) solid supports are long-chain alkylamines that are further functionalized with a succinyl, oxalyl, hydroquinone-O,O'-diacetic acid ('Q-linker'), or universal linkers like those described in US Patent No.: 6,770,754; European Patent No.: 1404695; Guzaev, A.P. et al. J. Am. Chem. Soc., 125, 2380-2381 (2003). Other options for linkers are disclosed in Pon, R.T. et al. Nucleic Acids Research, 25, 3629-3635 (1997).

As noted above, the moiety 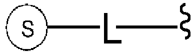 may be selected from 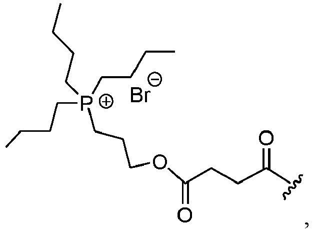 or orthogonally cleavable ionic tags such as 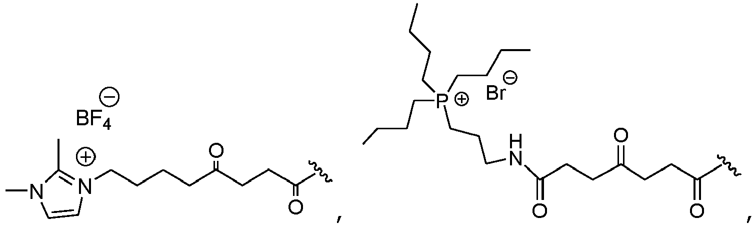 or 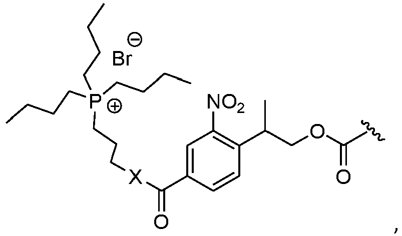 wherein X is selected from NH or O.

The utility of the dimer and trimer synthons described herein was tested through the solution phase synthesis of a decanucleotide, 5'-rUUAAUUAA-dTT-3', and the solid phase synthesis of oligonucleotides of uridine and mixed uridine/adenosine composition. The decamer was constructed by coupling of the previously synthesized dimer amidite blocks UpU (30) and ApA (47) (shown in Schemes 1 and 6) with a dTpdT dimer attached to a novel tetraalkylphosphonium ion tag (Scheme 21).

To demonstrate the general strategy in the assembly of the decamer, the synthesis of the tetramer 5'-rAprApdTpdT-3' intermediate is shown in Scheme 21.

The ionic liquid (**52)**, which was readily prepared from tributylphosphine and 3-bromo-1-propanol (Bradaric, C.J. et al. Green Chemistry, 5, 143 (2003); Huo, C. et al. J. Org. Chem. 73, 8583 (2008)) was coupled to succinylated 5'-DMTr-thymidine derivative (**95)** using dicyclohexylcarbodiimide (DCC) and catalytic amounts of 4-dimethylaminopyridine (DMAP) in methylene chloride to give the ion supported nucleoside (**96)** (Scheme 21). Compound **(96)** was isolated and purified by precipitation from methyl tert-butyl ether (MTBE). Detritylation of the ion-tagged dT **(96)** was achieved by the addition of 5% trifluoroacetic acid (TFA) in CH₃CN (10 min, r.t.), then triethylsilane to scavenge the trityl cation (5 min, r.t.), followed by precipitation in MTBE. Three trifluoroacetic acid/silane treatments were required to completely remove the trityl group, precipitating in MTBE after each treatment. Once the last detritylation step was achieved, the material is precipitated three times from MTBE to ensure complete removal of the acid, yielding compound **(97)** in 73% yield.

Structure and purity of **(96)** and **(97)** were verified by ¹H-, ¹³C-, ³¹P-NMR and ESI-MS. Under optimized conditions, the dinucleoside phosphotriester dTpT Succ IL was prepared at the 2 mmol scale by reacting dT Succ IL **(97)** with a 1.5-fold excess of thymidine phosphoramidite derivative **(98)** using 4,5-dicyanoimidazole (DCI) as the activating agent in CH₃CN/DMF and stirring for 3 hours. After the reaction had come to completion, the excess activated phosphoramidite was quenched through the addition of tert-butanol and a further 10 min of stirring. The phosphite triester TpT dimer intermediate was then dissolved in 15% v/v DMF in acetonitrile and treated with 0.5 eq each of Cap A and Cap B solution (16% N-methylimidazole in THF, and acetic anhydride:pyridine:THF, 1:2:2 ratio) for 10 min. Next, to carry out the oxidation of the internucleotide linkage, 5 eq of 6M tert-butyl hydroperoxide in decane was added and the resulting mixture allowed to stir for a further 15 min. This solution was then again precipitated and placed under a high vacuum to remove residual tert-butyl hydroperoxide or pyridine. Detritylation of the ion-tagged TpT **(99)** was achieved by the addition of 5% trifluoroacetic acid (TFA) in CH₃CN (10 min, r.t.), then triethylsilane to scavenge the trityl cation (5 min, r.t.), followed by precipitation in MTBE. Three trifluoroacetic acid/silane treatments were required to completely remove the trityl group, precipitating in MTBE after each treatment. Once the last detritylation step was achived, the material is precipitated three times from MTBE to ensure complete removal of the acid. The 5'-HO-dTT-Succ IL material **(100)** was obtained in 98% yield (characterized by ¹H NMR, MS and TLC).

Dimer dTT-Succ IL **(100)** (0.10 g, 0.098 mmol) was coupled to rAA dimer amidite (**47)** (0.293 mmol, 0.450 g, 3.0 eq.) in the presence of 4,5-dicyanoimidazole (DCI) (0.040 g, 3.5 eq.) to give after sequential precipitation, oxidation (t-BuOOH), precipitation, capping, and another precipitation the desired crude tetramer rAA-dTT Succ IL **(101)** (230 mg). Unreacted dTT-Succ IL was capped using Cap A/B solution followed by final precipitation to the tetramer rAA-dTT Succ IL. The addition of a 3-fold excess of block amidite was necessary to push the reaction to near completion. Detailed reaction conditions are described in the Experimental section (Examples) and Table 3. Following detritylation of the tetramer, the chain was extended in the 3'-to-5' direction as outlined schematically in Scheme 22 below. The reaction and deprotection conditions, as well as the product yields, are summarized in Table 3.

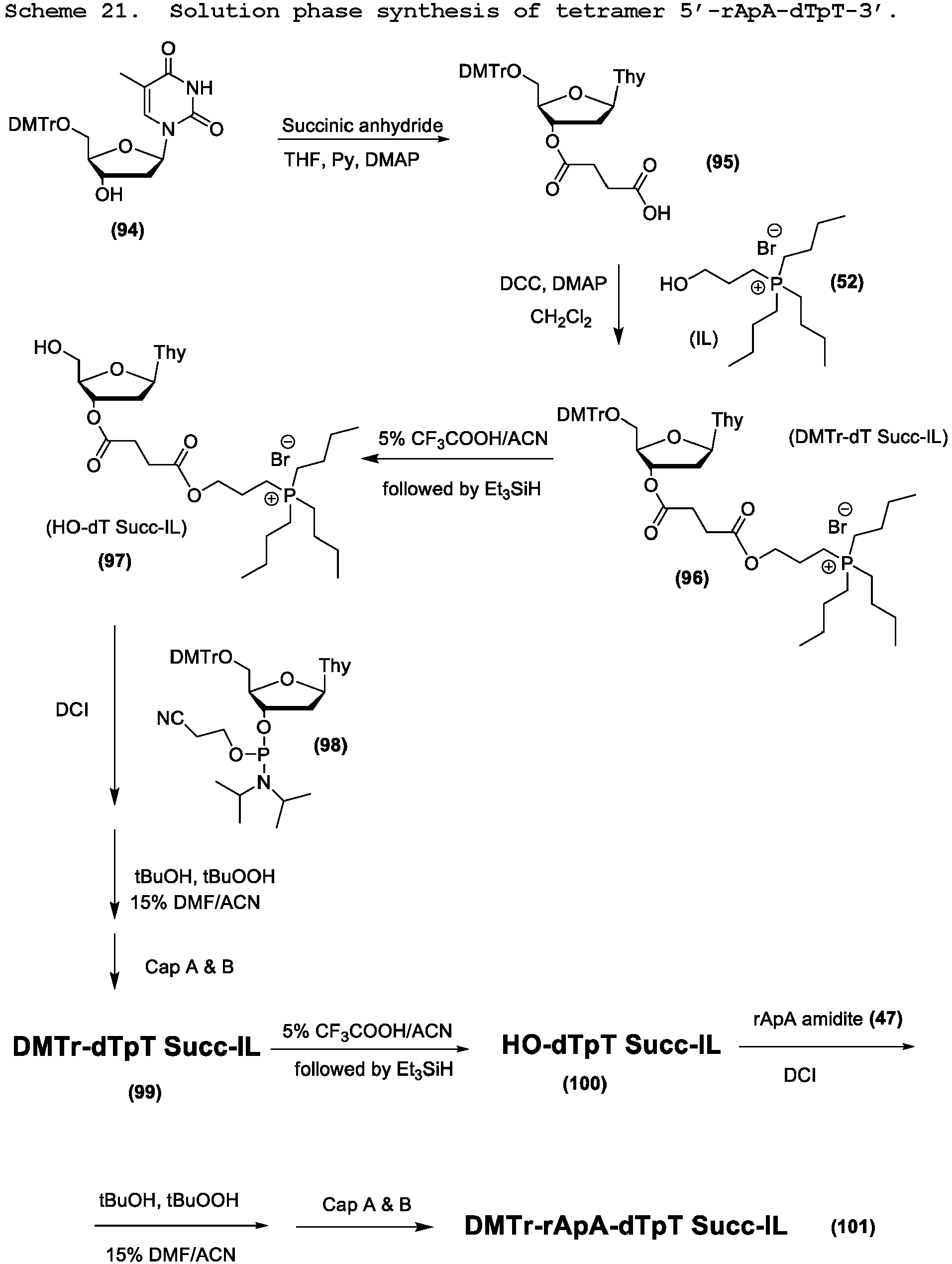

**Table 3. Reaction and deprotection conditions, and yields during the ion-tag supported synthesis of the decanucleotide 5'-rUUAAUUAA-dTT-3' via dimer coupling.**

| Entry | X+Y *^{a}* | Oligomer (X) (mmol / g) | Dimer (Y) (mmol / g) | # detritylation *^{b}* step s | Reagent *^{c}* | % Yield *^{d}* | Recovery *^{e}* (g, %) |
|---|---|---|---|---|---|---|---|
| 1 | 2+2 | dTT **(100)** | **rAAp (47)** | 2 | A | 81.0 | 0.23, 95 |
| | | 0.098/0.10 | 0.293 10.45 | | B | 78.1 | |
| 2 | 4+2 | **rAA-dTT (102)** | **rUUp (30)** | 2 | A | 72.4 | 0.24, 99 |
| | | 0.074 / 0.16 | 0.222 / 0.28 | | B | 70.0 | |
| 3 | 6+2 | **rUUAA-dTT (104)** | **rAAp (47)** | 3 | A | 58.5 | 0.18, 98 |
| | | 0.040 / 0.12 | 0.121 / 0.18 | | B | 58.5 | |
| 4 | 8+2 | **rAAUUAA-dTT (106)** | **rUUp (30)** | 3 | A | 44.5 | 0.16, 96 |
| | | 0.030 / 0.13 | 0.091 10.12 | | B | 46.0 | |
| | Repeat *^{f}* | **rAAUUAA-dTT(106)** | **rUUp (30)** | 3 | A | 45.6 | 0.127, n/a |
| | | (120 mg crude) | 0.091 / 0.12 | | B | 47.6 | |

| | | | | | | | |
|---|---|---|---|---|---|---|---|
| *^{a}* (X+Y)= type of coupling, e.g., 4+2 means coupling of tetramer rAA-dTT (Succ IL) + dimer rUUp amidite (**30**); 8+2 means coupling of 5'-rAAUUAA-dTdT-3' + rUUp dimer amidite (**30**). Coupling time was 48 hours in all cases. Concentration of limiting reactant (oligonucleotide "X") is maintained at 0.05M, amidite component at 0.15M (3 eq.) and DCI at 0.17M (3.5 eq.) Solvent of reaction is: 15% v/v dimethylformamide (DMF) in acetonitrile (ACN). ^{b} Detritylation was effected using a solution of 5% CF₃CO₂H in MeCN ( 7.71 mL/mmol of oligo; 10-15 min, r.t.), and then adding Et₃SiH (0.79 mL / mmol of oligo; 5-10 min, r.t.) followed by precipitation in MTBE. This step is repeated 2-3 times till detritylation is complete. Normally, 2 detritylation steps (at 15 min ea) is sufficient. ^{c} Reagent for removing methyl phosphate protecting group; A= 2-methyl-5-tert-butylthiophenol, ambient temperature, 2 h, or, B= tert-butylamine, 50 °C, 16 h. Following demethylation, the oligonucleotides were treated with 1:3 (ethanol: 29% *aq.* NH₄OH, v/v), r.t., 48 h, to remove N-benzoyl groups. One final step removes TIPS and TBDMS groups (NEt₃.3HF; 300 pL/pmol of oligo, r.t., 48 h). ^{d} a Yield is calculated from the HPLC profile of the isolated crude material, by calculating the area under the peak of the desired oligonucleotide. HPLC conditions are provided in the Experimental section (Examples). ^{e} Recovery of crude material after work up and precipitation in MTBE. *^{f}* Coupling of octamer with rUUp (**30**) amidite was carried twice, i.e., after the first 8+2 coupling, the material was worked up and isolated, and a portion (0.120 g) was coupled again with rUUp amidite to get the final coupled product (0.127 g). | | | | | | | |

To illustrate the general method used for deprotecting the oligonucleotides, the deprotection of the tetramer rAA-dTT-Succ IL is described here. The tetramer **(101)** was deprotected by a sequence of treatment consisting of (1) detritylation, effected using a solution of 5% CF₃CO₂H in MeCN (10-15 min, r.t.), and then adding Et₃SiH (5-10 min, r.t.) followed by precipitation in MTBE. This step was repeated 2 times till detritylation was complete obtaining **(102);** (2) removal of the cyanoethyl and methyl phosphate protecting groups [condition A: 2-methyl-5-tert-butylthiophenol: triethylamine: acetonitrile (1:1:3; v/v/v, 2 hours, r.t.); or condition B: tert-butylamine, 50 °C, 16 h]; (3) removal of the benzoyl groups (ethanolic aqueous ammonia, 16 h, 55°C), (5) removal of the silyl protecting groups (NEt₃.3HF; r.t., 48 h). HPLC analysis of the crude tetramer after deprotection revealed that the coupling of **(100)** with **(47)** had proceeded with 70-72.4% efficiency (Table 3). The identity of the tetramer was confirmed by MS and comparison to standard samples prepared via solid-phase synthesis. Detailed reaction conditions are described in the Experimental section (Examples) and Table 3.

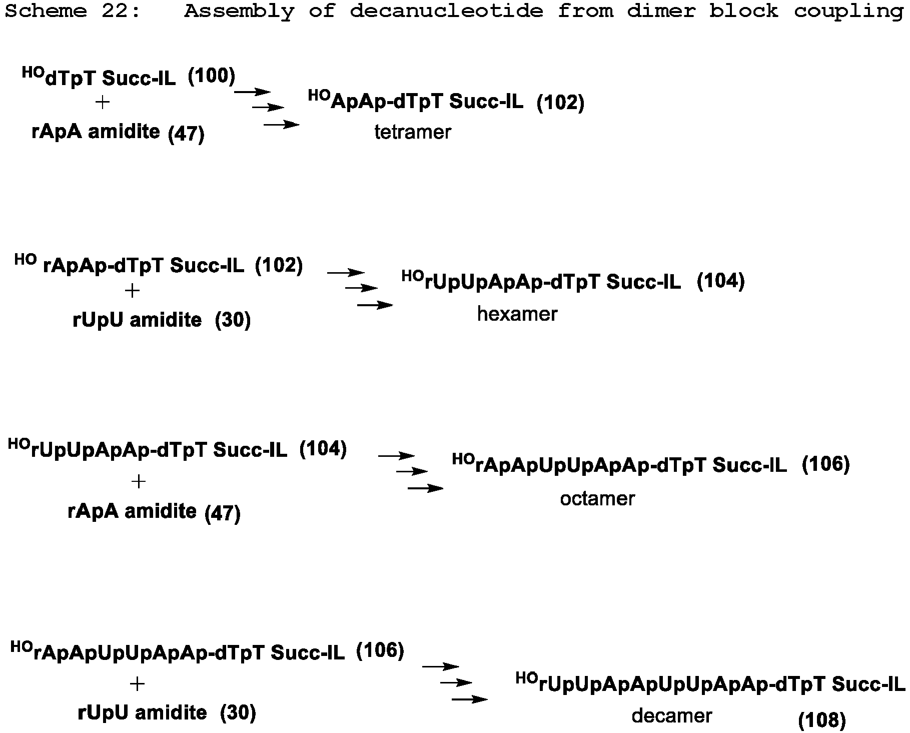

As shown in Table 3, there was no drastic decrease in the yields with increasing chain length. These range from ca. 80 to 50%, from the di- to the decamer level, which represent an apparent ave. coupling yield of 80-84% for the dimers. The addition of the dimer to the octamer was performed at a smaller scale (Table 3) and, as a result, the yield may be lower than expected because complete elimination of water from the reaction vessel is almost impossible. Remarkably, the quality of the crude oligonucleotide intermediates obtained by block coupling method was comparable to those obtained via the solid-phase method (LCAA-CPG support) using the standard monomeric phosphoramidites. Furthermore, their quality was clearly superior to those obtained in solution using monomer synthons. The comparisons (%yield) are shown in Figures 16 and 17. Overall, the results show that the combination of the ion-supported method and dimer block amidites will allow the synthesis of RNA without resorting to chromatography during chain assembly, requiring purification only after the final deprotection step of the desired target oligomer.

**Table 4. Physical data of protected oligonucleotides prepared in solution via dimer block coupling**

| Compound | Recovery (%) | Formula | ³¹P-NMR (ppm) | m/z (Calcd) | m/z (found) |
|---|---|---|---|---|---|
| (**100**) | 90 | C₄₂H₆₆N₅O₁₅P₂⁺ | 35.46,-0.72 to - 1.88 | 942.40 | 942.39 |
| (**101**) | 95 | C₁₁₄H₁₅₄Nₗ₅O₃₁P₄Si₂⁺ | 35.56, 1.67 to -0.39, -0.87 to -1.54 | 2408.94 | 2408.93 |
| (**102**) | 98 | C₉₃H₁₃₆N₁₅O₂₉P₄Si₂⁺ | 35.49, 1.68 to -0.18, -1.0 to -1.55 | 2106.81 | 2106.80 |
| (**103**) | 99 | C₁₄₉H₂₁₄N₁₉O₄₇P₆Si₄Na²⁺ | 35.56, 1.73 to -0.11, -0.87 to -1.65 | 1671.11 | 1671.11 |
| (**104**) | 94 | C₁₂₈H₁₉₆N₁₉O₄₅P₆S₁₄Na²⁺ | 35.55, 1.62 to -0.17, -0.89 to -1.59 | 1520.04 | 1520.05 |
| (**105**) | 98 | C₂₀₀H₂₈₄N₂₉O₆₁P₈Si₆⁺ | 35.6, 2.93 to -0.63, -0.89 to - 1.84 | - | - |
| (**106**) | 99 | C₁₇₉H₂₆₆N₂₉O₅₉P₈Si₆⁺ | 35.52, 4.99 to -0.62, -0.82 to - 3.54 | - | - |
| (**107**) | 96 | C₂₃₅H₃₄₄N₃₃O₇₇P₁₀Si₈⁺ | 35.57, 3.1 to - 3.76 | - | - |

**Table 5. HPLC/MS Analysis of Deprotected Oligonucleotides^{a} prepared by solid (CPG; monomer coupling) and solution phase (ion tag, dimer couplings)**

| Sequence | Synthetic Support | retention time (Min) | % total area | MS analysis | |
|---|---|---|---|---|---|
| | | | | (Calcd) | (Exptl) |
| ApApdTpdT | CPG | 17.0 | 87.3 | | |
| | IL-Monomer | 16.5 | 66.3 | 1204.2414 | 1204.2431 |
| | IL-Dimer | 16.8 | 81.0 | 1204.2414 | 1204.2421 |
| UpApApdTpdT | CPG | 19.6 | 85.2 | | |
| | IL-Monomer | 18.8 | 67.6 | 1510.2667 | 1510.2684 |
| UpUpApApdTpdT | CPG | 21.6 | 72.8 | | |
| | IL-Monomer | 21.0 | 66.2 | 1816.2920 | 1816.2935 |
| | IL-Dimer | 21.1 | 72.4 | 1816.2920 | 1816.2947 |
| ApUpUpApApdTpdT | CPG | 23.7 | 63.0 | | |
| | IL-Monomer | 22.9 | 56.8 | 2145.3445 | 2145.3472 |
| ApApUpUpApApdTpdT | CPG | 25.6 | 57.2 | | |
| | IL-Monomer | 24.6 | 49.2 | 2474.3970 | 2474.3987 |
| | IL-Dimer | 25.0 | 58.5 | | |
| UpApApUpUpApApdTpdT | CPG | 26.1 | 50.5 | | |
| | IL-Monomer | 25.5 | 41.7 | 2780.4223 | 2780.4264 |
| UpUpApApUpUpApApdTpdT | CPG | 26.9 | 55.0 | | |
| | IL-Monomer | 27.6 | 34.1 | 3086.4492 | 3086.4531 |
| | IL-Dimer | 27.1 | 47.6 | 3086.4492 | 3086.4525 |
| *a* IL = prepared with ionic liquid support; CPG = prepared with controlled pore glass with gene machine | | | | | |

### Solid-phase synthesis of 5'-(rUp)₁₈-dT-3'.

The use of dimer and trimer building blocks allow for longer chain extensions at each coupling stage of RNA synthesis, significantly reducing the total number of steps required in the synthesis of a target RNA oligomer relative the monomer coupling synthesis. The application of UpU, ApA, and UpUpU phosphoramidites in the synthesis of 19-nt oligoribonucleotide sequences has provided excellent results. Two sequences were assembled on controlled-pore glass (CPG) solid support derivatized with 5'-DMTr dT (1 micromol scale). Conditions for syntheses are outlined on Table 6.

For example, sequence 5'-(rUp)₁₈-dT-3' was assembled through monomer and block couplings as follows:
(a) CPG-dT + 18x U amidite **(7)** couplings.
(b) CPG-dT + 9x UpU amidite **(30)** couplings.
(c) CPG-dT + 6x UpUpU amidite **(50)** couplings.

Coupling efficiency of the monomer **(7),** dimer **(30)** and trimer **(50)** amidite blocks were assessed on a solid support using the following conditions: 1-µmol scale; dimer and trimer (0.15 M) blocks in MeCN, 0.25 M DCI as an activator, and 20-min coupling cycles. Oxidation of the phosphite triester intermediates was achieved using a 3 M solution of tert-butyl hydroperoxide in toluene. Following chain assembly, the fully deprotected oligomers were obtained by: (1) treatment with 5-tert-butyl-2-methylthiophenol (r.t., 120 min) to deblock the phosphate methyl groups; (2) washing the solid-support (ACN, 10 ml); (3) ammonolysis with NH₄OH/EtOH (3:1) (r.t., 60 h) to release the oligomers from the support, and (4) fluoride treatment (1ml of 1M TBAF in THF, r.t., 48h) to deblock the TBDMS and TIPS protecting groups. The three oligomers were identical as shown by HPLC and MALDI-TOF (calc 5753.3, found 5752.9 m/z).

**Table 6. Synthesis of oligonucleotides via monomer and block coupling.**

| Entry | Oligomer 5'-to-3' | Amidite | Cone. (M) | # of Couplings | Time (min) | Coupling efficiency (%) | Yield (a)a |
|---|---|---|---|---|---|---|---|
| I | (rU)₁₈dT | rU **(7)** | 0.10 | 18 | 10 | 98.5 | 76.5 |
| II | (rU)₁₈dT | rU **(7)** | 0.15 | 18 | 20 | 98.7 | 80.1 |
| III | (rU)₁₈dT | rUU **(30)** | 0.10 | 9 | 10 | 97.2 | 77.8 |
| IV | (rU)₁₈dT | rUU **(30)** | 0.15 | 9 | 20 | 98.3 | 85.9 |
| V | (rU)₁₈dT | rUUU **(50)** | 0.10 | 6 | 10 | 86.5 | 41.8 |
| VI | (rU)₁₈dT | rUUU **(50)** | 0.15 | 6 | 20 | 97.2 | 84.7 |
| VII | (rAAUU)₄dT₂ | rU **(7),** rA **(39)** | 0.15 | 16 | 20 | 98.0 | 72.5 |
| VIII | (rAAUU)₄dT₂ | rUU **(30),** rAA **(47)** | 0.15 | 8 | 20 | 98.5 | 88.8 |

| | | | | | | | |
|---|---|---|---|---|---|---|---|
| ^{a} a yield of oligomer in crude material (HPLC). Solid support: succinyl-LLAA-CPG. | | | | | | | |

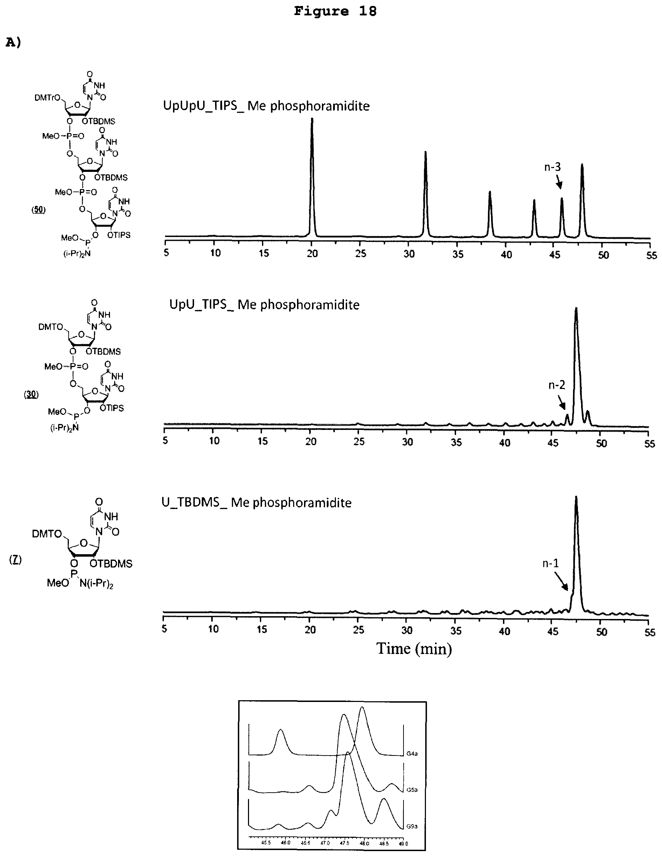

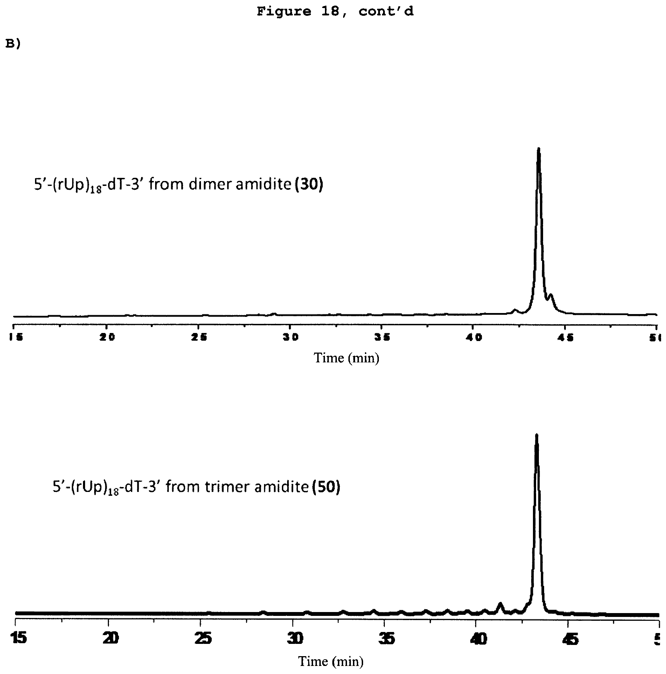

Yields of oligomers were calculated from the HPLC traces, from which coupling efficiencies of the amidites were estimated at 98.7%, 98.3%, and 97.2% for monomer, dimer and trimer amidite, respectively (Table 6; entries II, IV, VI). The overall yield of 5'-(rU)₁₈dT-3' prepared from trimer couplings (6× at 97.2% efficiency) was 84.7 %, which was superior to the 80% overall yield obtained by coupling rU monomers (18× at 98.7% efficiency) (Table 6; entries II vs VI). Reducing the trimer amidite concentration to 0.10 M and the coupling time by 50% reduced the coupling efficiency by 11% (Table 6; entry V). These conditions afforded, as expected, a mixture of the full length product and a series of well resolved n-3 "failure" sequences (Figure 18). A second poly-py/pu sequence, 5'-(rAAUU)₄dTT-3', was assembled from dimers **(30)** and **(47)** in 88.8% overall yield (98.5% per coupling). This contrasts the 72.5% overall yield obtained when this sequence was assembled via monomer coupling (98.0 % per coupling; Table 6, entry VII vs VIII). Thus in this case, the block coupling strategy yielded ca. 16% more product than the standard method.

In summary, the present invention describes a viable route for the synthesis of regioisomerically pure dimer and trimer RNA phosphoramidites that couple with similar efficiency as monomeric phosphoramidite units. The method increases the overall yield of the target oligoribonucleotide sequence by decreasing the number of coupling steps required for chain assembly and has the potential of significantly simplifying the final purification of RNA sequences. The present invention also demonstrates that dimer and trimer synthons can be utilized either in solution or solid-phase in conjunction with monomer synthons in the final stages of chain assembly, affording n-2 or n-3 failure sequences that are more readily resolved. Dimer and trimer amidite blocks will likely find use in the large scale solution (or solid)-phase synthesis of siRNA drugs.

### EXAMPLES

### General Methods

¹H-NMR were acquired on either a 400- and 500-Varian NMR Spectrometers. ¹³C- and ³¹P-NMR spectra were recorded on 300- and 202-Varian NMR spectrometers. Mass spectra were taken on a ThermoQuest Finnigan LCQ Duo octopole mass spectrometer using electrospray ionization in either positive or negative mode. Thin layer chromatography was done on aluminum backed 200µm thick silica with fluorescent indicator UV₂₅₄ Macherey-Nagel. All N-protected nucleosides and phosphitylating reagents were purchased from ChemGenes (33 Industrial Way, Wilmington, MA). All other reagents were purchased from Aldrich. Pyridine was distilled over calcium hydride and all other solvents were dried on a M-Braun SPS solvent drying machine.

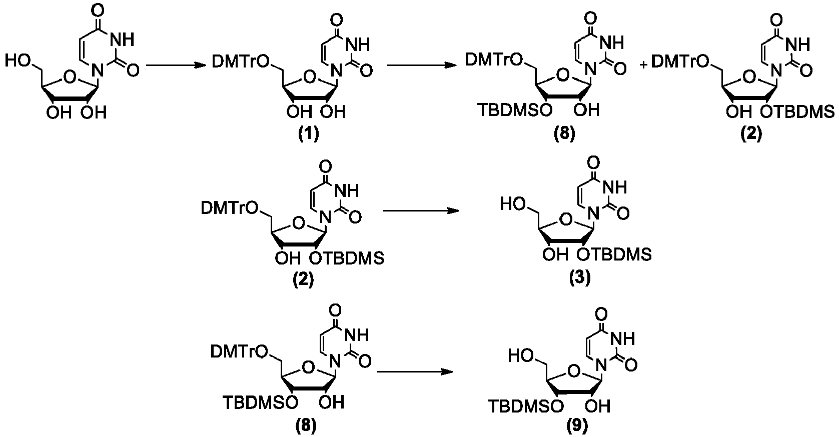

### 5'-DMTr-uridine (1)

Uridine (20.3 g, 83.3 mmol) was suspended in anhydrous pyridine (Py) and evaporated to dryness on a rotovap (x3). Uridine was again suspended in 125ml of Py and 125ml of tetrahydrofuran (THF). To this suspension was added 4,4'-dimethoxytrityl chloride (DMTr-Cl) (28.4 g, 84.1 mmol). The mixture began to go into solution after 30min and by 5h the reaction was almost complete. More DMTr-Cl could be added to drive the reaction to completion, but increased amounts of side products (e.g. ditritylated rU) may form. The solution was then condensed slightly removing approximately half volume on a rotoevaporator. This mixture was then diluted with ethyl acetate and extracted with sat. NaHCO₃ (x2) and brine (x2). The organic layer was dried with MgSO₄ and purified by column chromatography 0→4% MeOH: dichloromethane (DCM). Yield: 80%.
Mass Calc. 546.2 Mass found: 596.1 (M+Na⁺)

### 5'-DMTr-2'-TBDMS-uridine (2) and 5'-DMTr-3'-TBDMS-uridine (8)

Compound **(1)** (27.88 g, 51.0 mmol) was dissolved in dry THF (340 mL). Dry pyridine (16.2 mL, 0.20 mol) and AgNO₃ (9.53 g, 56.1 mmol) were added and stirring was continued for 15 min. Once almost all AgNO₃ dissolved, tert-butyldimethylsilyl chloride (TBDMS-Cl) (8.46 g, 56.1 mmol) was added all at once and the resulting cloudy milky solution was stirred at room temperature for 12h. The reaction mixture was filtered into 5% NaHCO₃ solution to avoid deprotection of DMTr. This mixture was condensed on the rotovap to remove approx half the solvent (200ml) . This mixture was then diluted with ethyl acetate and extracted with sat. NaHCO₃ (x3). The organic layer was dried with MgSO₄ and condensed to dryness. This mixture was purified by column chromatography in tert-butyl-methyl ether: Hexanes 0→50%. Yield of 5'-DMTr-2'-TBDMS-uridine **(2)** 21.92g (65%) and 5'-DMTr-3'-TBDMS-uridine **(8)** 8.52g (26%).

### 5'-DMTr-2'-TBDMS-uridine (2):

¹H NMR (400 MHz, DMSO-d₆) δ ppm -0.14 (s, 5 H) -0.04 (s, 6 H) 0.74 (s, 15 H) 1.07 (t, J=7.04 Hz, 2 H) 3.26 (d, J=4.30 Hz, 9 H) 3.70 (s, 10 H) 4.12 (d, J--4.70 Hz, 3 H) 4.26 (d, J=5.48 Hz, 2 H) 4.88 (t, J=5.09 Hz, 2 H) 5.19 (d, J=6.26 Hz, 2 H) 5.74 (s, 2 H) 6.06 (d, J=5.09 Hz, 1 H) 6.83 (dd, J=9.00, 2.74 Hz, 7 H) 7.03 - 7.29 (m, 12 H) 7.37 (d, J=7.43 Hz, 4 H) 7.45 - 7.57 (m, 4 H) 7.62 (d, *J*=7.43 Hz, 2 H) 8.02 (d, J=7.04 Hz, 4 H) 8.58 (s, 2 H) 8.65 (s, 2 H) 11.20 (br. s., 3 H) Mass calc. 546.20 Mass found: 569.32 (M+Na⁺) .

### 5'-DMTr-3'-TBDMS-uridine (8)

¹H NMR (400 MHz, DMSO-d₆) δ ppm -0.04 (s, 4 H) 0.76 (s, 6 H) 3.16 (dd, J=10.78, 4.35 Hz, 1 H) 3.25 - 3.40 (m, 2 H) 3.71 (s, 5 H) 3.84 - 3.98 (m, 1 H) 4.13 (m, 2 H) 5.24 - 5.44 (m, 2 H) 5.62 - 5.83 (m, 1 H) 6.76 - 7.02 (m, 3 H) 7.07 - 7.49 (m, 7 H) 7.79 (s, 1 H) 11.39 (s, 1 H) Mass calc. 546.20 Mass found: 569.30 (M+Na⁺) .

### 2'-TBDMS-uridine (3)

A solution of 3% triflouroacetic acid (TFA) in DCM (15mL) was added directly to compound **(2)** (1.26 g, 1.91 mmol). The solution was quenched with 5 mL of MeOH after 5 min and condensed to dryness on a rotovap. By TLC there was a very small amount of starting material so the process was repeated again. The compound was purified by column chromatography. 0 →75% Ethyl acetate: hexanes. Yield: 0.65 g (94%).

¹H NMR (500 MHz, DMSO-d₆) δ ppm 0.02 (s, 10 H) 0.00 (s, 10 H) 0.82 (s, 30 H) 3.55 (d, J=11.98 Hz, 4 H) 3.63 (d, J=11.25 Hz, 4 H) 3.86 (br. s., 4 H) 3.92 (d, J=4.16 Hz, 4 H) 4.01 (d, J=7.34 Hz, 3 H) 4.12 (br. s., 4 H) 4.96 (d, J=5.14 Hz, 4 H) 5.12 (br. s., 5 H) 5.63 (d, J=8.07 Hz, 4 H) 5.76 (d, J=4.40 Hz, 4 H) 7.92 (d, J=8.07 Hz, 1 H) 11.30 (br. s., 1 H) Mass calc. 358.16 Mass found: 381.20 (M+Na⁺) .

### 3'-TBDMS-uridine (9)

A solution of 3% triflouroacetic acid (TFA) in DCM (20mL) was added directly to compound **(8)** (2.0 g, 3.03 mmol). The solution was quenched with 5 mL of MeOH after 5 min and condensed to dryness on a rotovap. By TLC there was a very small amount of starting material so the process was repeated again. The compound was purified by column chromatography. 0 →75% Ethyl acetate: hexanes. Yield: 1.05g (96%).

¹H NMR (400 MHz, DMSO-d₆) δ ppm 0.07 (s, 6 H) 0.87 (s, 9 H) 3.54 (d, J=17.19 Hz, 2 H) 3.83 (d, J=2.74 Hz, 1 H) 4.02 - 4.17 (m, 2 H) 5.21 (br. s., 1 H) 5.28 (br. s., 1 H) 5.64 (d, J=8.21 Hz, 1 H) 5.77 (d, J=6.64 Hz, 1 H) 7.85 (d, J=8.21 Hz, 2 H) 8.31 (s, 1 H); Mass calc. 358.16 Mass found: 381.20 (M+Na⁺).

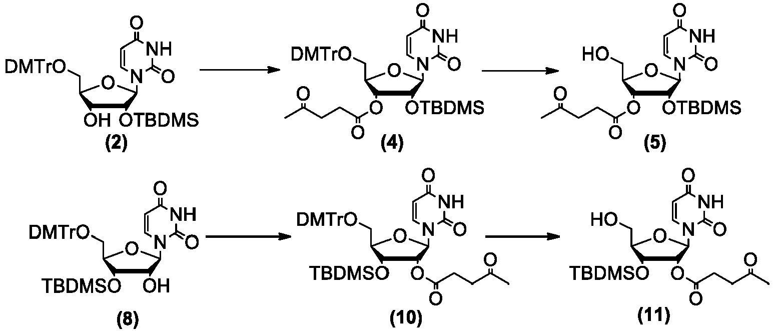

### 5'-DMTr-2'-TBDMS-3'-levulinyl-uridine (4)

Levulinic acid (1.70g, 14.6 mmol), DCC (3.03 g, 14.5 mmol) and DMAP (0.23 g, 1.85mmol) were dissolved in 50mL of THF and stirred for 15 min. Compound **(2)** (4.83 g, 7.30 mmol) was then added directly to the above solution. After 5h the reaction was completely brown/red and complete by TLC (50:50 ethyl acetate:hexanes). The reaction mixture was filtered over Celite^{™}. The organic layer was washed with 5% NaHCO₃, and saturated NaCl. The compound was dried with MgSO₄ and purified by column chromatography 0 →30% ethyl acetate:hexanes. Yield: 4.88g (87%)

¹H NMR (500 MHz, ACETONITRILE-d₃) δ ppm 0.09 (d, J=3.00 Hz, 6 H) 0.85 - 0.94 (m, 9 H) 2.50 - 2.67 (m, 2 H) 2.68 - 2.83 (m, 2 H) 3.40 (d, J=3.00 Hz, 1 H) 3.53 - 3.66 (m, 1 H) 3.80 (s, 6 H) 3.92 - 4.05 (m, 1 H) 4.19 (d, J=3.86 Hz, 1 H) 4.50 (s, 1 H) 5.30 (d, J=0.86 Hz, 1 H) 5.40 (d, J=8.14 Hz, 1 H) 5.87 (d, J=5.57 Hz, 1 H) 6.87 - 7.01 (m, 2 H) 7.25 - 7.40 (m, 4 H) 7.41 - 7.49 (m, 1 H) 7.70 (d, J=8.14 Hz, 1 H) ESI MS, Calc. 758.32 found: 781.32 (M+Na⁺)

### 2'-TBDMS-3'-levulinyl-uridine (5)

5'-DMTr-2'-TBDMS-3'-levulilnyl uridine **(4)** (10g, 13.2 mmol) was dissolved in 100 mL of 3% trifloroacetic acid (TFA) in DCM (vol/vol) and allowed to stir for 10 min followed by 10 mL of MeOH. The solution was immediately condensed to an oil and the process was repeated with another 100 mL of DCM/TFA. The sample was then re-suspended in DCM and loaded on a column. The DCM was washed off with hexanes and the tritanol was eluted with hexanes:ethyl acetate (50:50) the product was eluted with 75% ethyl acetate. Yield: 5.68 g (94%).

¹H NMR (500 MHz, ACETONITRILE-d₃) δ ppm 0.10 (s, 8 H) 0.13 (s, 6 H) 0.80 - 0.97 (m, 9 H) 2.12 (s, 2 H) 2.48 - 2.64 (m, 2 H) 2.67 - 2.82 (m, 2 H) 3.38 (br. s., 1 H) 3.65 - 3.86 (m, 2 H) 3.98 - 4.04 (m, 1 H) 4.38 - 4.49 (m, 1 H) 5.25 (t, *J*=5.32 Hz, 1 H) 5.48 (s, 1 H) 5.66 (d, *J*=8.44 Hz, 1 H) 5.95 (d, *J*=5.50 Hz, 1 H) 7.82 (d, *J*=8.07 Hz, 1 H) Calc. Mass: 456.19 Mass found: 479.29 (M+Na⁺).

### 5'-DMTr-2'-levulinyl-3'-TBDMS-uridine (10)

Levulinic acid (4.44 g, 6.72 mmol), DCC (2.64 g, 12.8 mmol) and DMAP (0.1g, 0.82 mmol) were dissolved in 45mL of THF and stirred for 15 min. Compound **(8)** (4.44 g, 7.30 mmol) was then added directly to the above solution. After 5h the reaction was completely brown/red and complete by TLC (50:50 ethyl acetate:hexanes). The reaction mixture was filtered over Celite^{™}. The organic layer was washed with 5% NaHCO₃, and saturated NaCl. The compound was dried with MgSO₄ and purified by column chromatography 0 →30% ethyl acetate:hexanes. Yield: 3.11 g (70%). Mass calc. 758.32 Mass found: 781.32 (M+Na⁺).

### 2'-Levulinyl-3'-TBDMS uridine (11)

5'-DMTr-2'-levulinyl-3'-TBDMS-uridine **(10)** (2.40g, 3.16 mmol) was dissolved in 25ml of 3% trifloroacetic acid (TFA) in DCM (vol/vol) and allowed to stir for 5min followed by 10ml of MeOH. The solution was immediately condensed to a oil and the process was repeated with another 15ml of DCM/TFA. The sample was then re- suspended in DCM and loaded on a column. The DCM was washed off with hexanes and the tritanol was eluted with hexanes:ethyl acetate 50:50, the product was eluted with 80% ethyl acetate. Yield: 1.36g (94%).

¹H NMR (500 MHz, ACETONITRILE-d₃) δ ppm 0.04 (d, *J*=3.67 Hz, 15 H) 0.74 - 0.91 (m, 23 H) 1.97 (dt, *J*=4.86, 2.52 Hz, 9 H) 2.15 (s, 10 H) 2.54 - 2.68 (m, 6 H) 2.72 - 2.82 (m, 6 H) 3.64 - 3.79 (m, 7 H) 4.07 - 4.19 (m, 3 H) 4.45 (dd, *J*=6.05, 5.32 Hz, 4 H) 5.19 (dd, *J*=5.14, 3.30 Hz, 4 H) 5.70 (dd, *J*=8.07, 2.20 Hz, 3 H) 5.86 (d, *J*=6.24 Hz, 3 H) 7.84 (d, *J*=8.44 Hz, 3 H) 9.13 (br. s., 4 H). Mass calc. 456.19 Mass found: 479.29 (M+Na⁺).

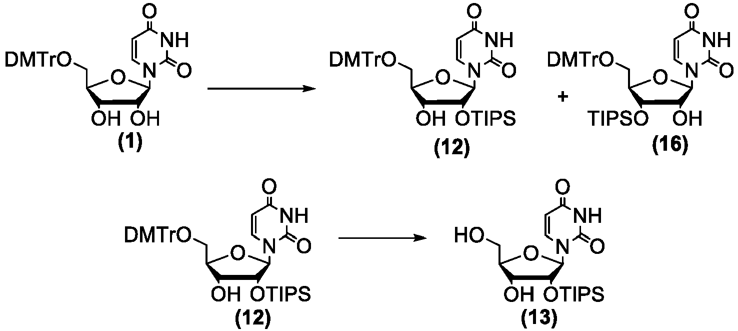

### 5'-DMTr-2'-TIPS-uridine (12) and 5'-DMTr-3'-TIPS-uridine (16)

Compound **(1)** (8.55 g, 15.6 mmol) was dissolved in dry THF 156 mL. Dry pyridine (5.10 mL, 62.5 mmol) and AgNO₃ (2.92 g, 17.2 mmol) were added and stirring was continued for 5 min, until almost all AgNO₃ dissolved. TIPSCl (3.6 mL, 17.2 mmol) was added all at once and the resulting cloudy milky solution was stirred at room temperature. After 12h TLC was analysed and there was still a lot of starting material in the reaction mixture. So, one half more equivalent of AgNO₃ and TIPSCl were added and the mixture was sonicated for another 6 h. The reaction mixture was filtered into 5% NaHCO₃ solution to avoid deprotection of DMTr. The reaction mixture was extracted with DCM, evaporated and purified through column chromatography. The unreacted starting material was recovered. Yield: 2'-TIPS nucleoside: 7.25g (66%); 3'-TIPS nucleoside 2.19 g (20%).

¹H NMR (300 MHz, ACETONITRILE-d₃) of 2'-TIPS nucleoside **(12):** δ ppm 1.05 - 1.22 (m, 21 H) 3.17 (d, *J*=5.22 Hz, 1 H) 3.36 (dd, *J*=10.89, 2.97 Hz, 2 H) 3.79 (s, 6 H) 4.09 (d, *J*=3.96 Hz, 1 H) 4.30 (d, *J*=4.68 Hz, 1 H) 4.51 (t, *J*=4.95 Hz, 1 H) 5.34 (d, *J*=8.10 Hz, 1 H) 5.91 (d, *J*=4.86 Hz, 1 H) 6.90 (dd, J=9.00, 0.90 Hz, 2 H) 7.26 - 7.37 (m, 7 H) 7.41 - 7.46 (m, 2 H) 7.75 (d, *J*=8.28 Hz, 1 H). Mass calc. 702.33, found 725.36.

### 2'-TIPS-uridine (13)

A solution of 3% triflouroacetic acid (TFA) in DCM (30mL) was added directly to compound **(12)** (3.54 g, 5.04 mmol). The solution was quenched with 10 mL of MeOH after 5 min and condensed to dryness on a rotovap. By TLC there was a very small amount of starting material so the process was repeated again. The compound was purified by column chromatography, 0 →75% ethyl acetate: hexanes. Yield: 1.96 g (97%).

Mass calc. 702.33, found 725.38.

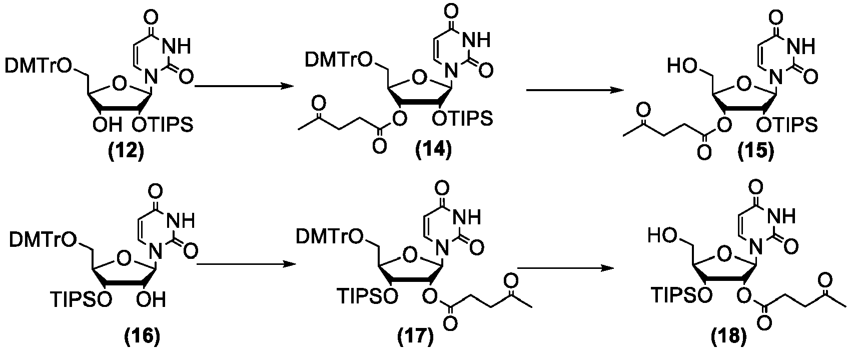

### 5'-DMTr-2'-TIPS-3'-levulinyl-uridine (14)

Levulinic acid (1.05 g, 9.05 mmol), DCC (1.601 g, 7.66 mmol) and DMAP (0.23 g, 1.85 mmol) were dissolved in 26 mL of THF and stirred for 10 min. Compound **(12)** (0.5 g, 0.613 mmol) was then added directly to the above solution. After 5h the reaction was completely brown/red and complete by TLC (50:50 ethyl acetate:hexanes). The reaction mixture was filtered over Celite^{™}. The organic layer was washed with 5% NaHCO₃, and saturated NaCl. The compound was dried with MgSO₄ and purified by column chromatography 0 →30% ethyl acetate:hexanes. Yield: 1.79 g (86%).

¹H NMR (300 MHz, ACETONITRILE-d₃) δ ppm 1.02 - 1.36 (m, 21 H) 2.13 (s, 3 H) 2.54 - 2.62 (m, 2 H) 2.71 - 2.78 (m, 2 H) 3.37 - 3.40 (m, 2 H) 3.78 (s, 6 H) 4.15 (d, *J*=2.77 Hz, 1 H) 4.63 - 4.67 (m, 1 H) 5.31 (dd, *J*=5.14, 2.96 Hz, 1 H) 5.40 - 5.46 (m, 1 H) 5.42 (s, 1 H) 5.99 (d, *J*=6.32 Hz, 1 H) 6.87 - 6.93 (m, 4 H) 7.27 - 7.37 (m, 7 H) 7.41 - 7.45 (m, 2 H) 7.75 (d, *J*=8.30 Hz, 1 H).

### 2'-TIPS-3'-levulinyl-uridine (15)

A solution of 3% triflouroacetic acid (TFA) in DCM (20mL) was added directly to compound **(14).** The solution was quenched with 5mL of MeOH after 5 min and condensed to dryness on a rotovap. By TLC there was a very small amount of starting material so the process was repeated again. The compound was purified by column chromatography. 0 →75% ethyl acetate: hexanes. Yield: 0.92 g (95%) .

¹H NMR (300 MHz, CHLOROFORM-d) δ ppm 0.96 - 1.11 (m, 20 H) 2.20 (s, 3 H) 2.52 - 2.88 (m, 5 H) 3.82 (d, *J*=2.05 Hz, 1 H) 3.88 - 3.95 (m, 1 H) 4.16 - 4.21 (m, 1 H) 4.82 (t, *J*=5.27 Hz, 1 H) 5.17 (dd, *J*=4.98, 3.52 Hz, 1 H) 5.68 (d, *J*=5.57 Hz, 1 H) 5.75 (dd, *J*=7.91, 2.34 Hz, 1 H) 7.71 (d, *J*=8.21 Hz, 1 H) 8.43 (s, 1 H).

### 5'-DMTr-2'-levulinyl-3'-TIPS-uridine (17)

Levulinic acid (0.49 g, 4.24 mmol), DCC (0.834 g, 4.04 mmol) and DMAP (0.09 g, 0.73 mmol) were dissolved in 13 mL of THF and stirred for 10 min. Compound **(16)** (1.42 g, 2.02 mmol) was then added directly to the above solution. After 5h the reaction was completely brown/red and complete by TLC (50:50 ethyl acetate:hexanes). The reaction mixture was filtered over Celite^{™}. The organic layer was washed with 5% NaHCO₃, and saturated NaCl. The compound was purified by column chromatography, 0 →30% ethyl acetate:hexanes. Yield: 1.36g (84%) .

¹H NMR (300 MHz, CHLOROFORM-d) δ ppm 0.72 - 1.13 (m, 21 H) 2.14 - 2.19 (m, 3 H) 2.60 - 2.69 (m, 2 H) 2.69 - 2.84 (m, 2 H) 3.38 (dd, *J*=10.84, 2.93 Hz, 1 H) 3.47 - 3.56 (m, 1 H) 3.79 (s, 6 H) 4.08 - 4.16 (m, 1 H) 4.58 (dd, *J*=4.98, 2.93 Hz, 1 H) 5.38 - 5.47 (m, 2 H) 6.16 (d, *J*=6.15 Hz, 1 H) 6.83 (d, *J*=8.50 Hz, 4 H) 7.21 - 7.53 (m, 10 H) 7.74 (d, *J*=8.21 Hz, 1 H) 8.53 - 8.64 (m, 1 H). Mass calc. 800.37, found 823.24

### 2'-Levulinyl-3'-TIPS-uridine (18)

A solution of 3% triflouroacetic acid (TFA) in DCM (20mL) was added directly to compound **(17)** (1.10 g, 1.37 mmol). The solution was quenched with 5ml of MeOH after 5 min and condensed to dryness on a rotovap. By TLC there was a very small amount of starting material so the process was repeated again. The compound was purified by column chromatography, 0 →75% ethyl acetate: hexanes. Yield: 0.65g (95%).

¹H NMR (300 MHz, CHLOROFORM-d) δ ppm 0.96 - 1.11 (m, 21 H) 2.20 (s, 3 H) 2.52 - 2.88 (m, 5 H) 3.82 (d, J=2.05 Hz, 1 H) 3.88 - 3.95 (m, 1 H) 4.16 - 4.21 (m, 1 H) 4.82 (t, *J*=5.27 Hz, 1 H) 5.17 (dd, *J*=4.98, 3.52 Hz, 1 H) 5.68 (d, *J*=5.57 Hz, 1 H) 5.75 (dd, *J*=7.91, 2.34 Hz, 1 H) 7.71 (d, *J*=8.21 Hz, 1 H) 8.43 (s, 1 H).

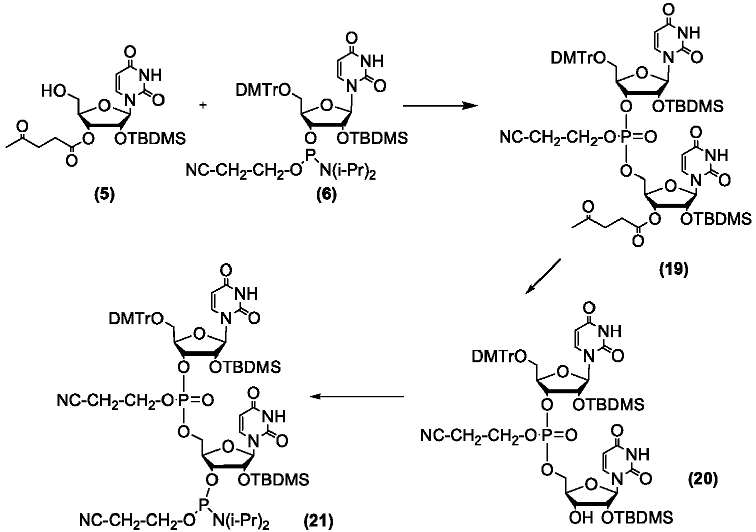

### [5'-DMTr-2'TBDMS-rU]-[3'-p(CNEt)-5']-[rU-2'TBDMS-3'-Lev] dimer (19)

2'-TBDMS-3'-levulinyl-uridine **(5)** (2.85g, 6.25mmol) was dissolved in 60mL of THF with 2eq of DCI (1.48g, 12). To this stirring solution 1.2eq (6.46g 7.50mmol) 5'-DMTr-2'TBDMS-uridine 3'-cyanoethylphosphoramidite **(6)** was added and allowed to stir at room temperature for 2h. The reaction was monitored by TLC (5%MeOH in DCM) and after 2 hours a small amount of compound (1) remained and another 0.2 eq (0.11 g, 0.124 mmol)of amidite were added and allowed to stir for one hour until the starting material had been consumed. At this point 10 eq (10 mL of a 6 M solution in decane) of *tert*-butyl hydroperoxide was added and allowed to stir for 20 min, until the phosphite triester was converted to the phosphate, as monitored by TLC (5% MeOH in DCM). The solution was condensed to approximately 10 mL and then dissolved in ethyl acetate and extracted with 5% sodium sulfite (x2) and brine (x1). The organic layer was dried and condensed and put on a short column (0→5% MeOH in DCM) yielding 70 % pure material. The column was not treated with triethylamine to avoid loss of the cyanoethyl phosphate protecting group.

¹H NMR (400 MHz, ACETONITRILE-d₃) δ ppm -0.04 - 0.22 (m, 5 H) 0.77 - 0.97 (m, 7 H) 1.96 (dt, *J*=4.94, 2.47 Hz, 1 H) 2.07 - 2.18 (m, 2 H) 2.66 - 2.90 (m, 2 H) 3.44 (d, *J*=2.74 Hz, 2 H) 3.79 (d, *J*=1.37 Hz, 4 H) 4.15 - 4.56 (m, 6 H) 4.88 (dd, *J*=7.95, 3.84 Hz, 1 H) 5.03 - 5.15 (m, 1 H) 5.36 (dd, *J*=8.23, 5.21 Hz, 1 H) 5.43 - 5.50 (m, 3 H) 5.64 (d, *J*=8.23 Hz, 1 H) 5.72 - 5.93 (m, 2 H) 6.91 (dd, *J*=8.91, 2.88 Hz, 3 H) 7.20 - 7.56 (m, 9 H) 7.69 (dd, *J*=8.23, 1.92 Hz, 1 H) 9.29 (s, 2 H); ³¹P NMR (81 MHz, Acetone) δ ppm 0.58 (s, 1 P) 0.96 (s, 1 P).

### [5'-DMTr-2'TBDMS-rU]-[3'-p(CNEt)-5']-[rU-2'TBDMS] (20)

Compound **(19)** (2.79 g 2.22 mmol) was dissolved in minimal amounts of ACN, just enough to get it into solution. It was then treated with ten equivalents (45 mL, 22.2 mmol) of a 0.5 M hydrazine hydrate solution in 3:2 pyridine:acetic acid that was prepared immediately before the reaction. The reaction mixture was allowed to stir for 10 min before it was chilled to 0°C in an ice bath and ten equivalents (2.31 mL, 22.2 mmol) of 2,4-pentanedione which was allowed to stir for an additional 5 min before the reaction was diluted with MTBE and extracted x3 with ammonium chloride (5%w/v) and once with cupric sulfate, and once with brine. The sample was then dried over MgSO₄ and condensed to dryness. The sample was put on a short column and run with MeOH 0→4 % in chloroform. The column was not treated with triethylamine to avoid loss of the cyanoethyl phosphate protecting group. Yield approx 70 % contained impurities of other regioisomer and some loss due to decyanoethylation.

¹H NMR (400 MHz, ACETONITRILE-d₃) δ ppm -0.05 - 0.17 (m, 12 H) 0.76 - 0.95 (m, 19 H) 1.29 (s, 1 H) 1.88 - 2.04 (m, 6 H) 2.09 - 2.28 (m, 8 H) 2.66 - 2.84 (m, 3 H) 3.44 (br. s., 2 H) 3.79 (d, J=1.17 Hz, 7 H) 3.99 - 4.03 (m, 1 H) 4.06 - 4.30 (m, 7 H) 4.33 - 4.39 (m, 2 H) 4.42 - 4.55 (m, 1 H) 4.66 - 4.93 (m, 2 H) 5.30 - 5.41 (m, 1 H) 5.62 (d, J=8.22 Hz, 2 H) 5.74 (dd, J=4.30, 1.57 Hz, 1 H) 5.80 (s, 1 H) 5.84 - 5.92 (m, 1 H) 6.80 - 7.02 (m, 5 H) 7.26

- 7.48 (m, 11 H) 7.61 (s, 1 H) 7.64 - 7.82 (m, 2 H) 9.29 (br. s., 3 H).

### [5'-DMTr-2'TBDMS-rU]-[3'-p(CNEt)-5']-[rU-2'TBDMS]-3'-cyanoethyl phosphoramidite (21)

Compound with impurities **(20)** (0.61 g, 0.530 mmol) was dissolved in 2.0 mL of dry THF. The nucleotide solution was added drop wise over 10min to a solution of (N,N-diisopropylamino) methylphosphonamidic chloride 1.3 eq (0.153 mL) and diisopropylethylamine (2 eq, 0.147 mL). The reaction was monitored by TLC (5 % MeOH in DCM). Excess amidite ran close to the newly formed phosphitylated dimer, but once the reaction was worked up the quenched product had a much lower polarity. Once the reaction was complete, 1.5 h, the reaction mixture was diluted with ethyl acetate pre washed with sat. NaHCO₃. It was then extracted once with a solution of 5% NaHCO₃ and x2 with brine. The organic phase was dried with MgSO₄, condensed to dryness, coevaporated with toluene (x2). The cyanoethyl phosphate protecting group was base labile as was the condition of phosphitylation which posed a problem for the synthesis of the dimer. This fact must be kept in mind throughout the synthesis and purification. To overcome these problems, very little excess of diisopropylethylamine was used during the synthesis, just enough to quench the HCl formed from the chlorophosphite. As well, the reaction mixtures were kept reasonably concentrated and reaction times were kept short. Even if there was a small amount of starting material left after 1.5 hours the reaction was worked up. For the purification process, the silica was first dried in a 500°C oven for a few days, then it was allowed to cool in a desiccator. The silica was then loaded on the column with dry DCM and 3% dry pyridine. Once packed, 300ml of 0.05 % pyridine in DCM was run through the column washing the excess pyridine away. At all times there was 0.05 % pyridine in the elution solvent. As an extra precaution a layer, 2 inches, of sodium sulphate was added above the silica to remove any excess water from the solvent. Once the fractions were collected and ready to be condensed, 10 mL of dry toluene or benzene was added to the solution to aid in removal of residual pyridine. Once the sample was near dryness, toluene or benzene was again added to aid in the co evaporation of pyridine. To remove any decyanoethylated product from the amidite, one can solvate the amidite in MTBE and the deprotected sample will precipitate out. Clearly the other isomer was present in the phosphorus NMR as well as the decyanoethylated product. Yield: 0.14 g (20 %).

³¹P NMR (81 MHz, Acetone) δ ppm -0.99 (s, 1 P) -0.80 (s, 1 P) - 0.42 (s, 1 P) -0.30 (s, 1 P) 9.32 (s, 1 P) 14.57 (s, 1 P) 150.19 (s, 1 P) 150.49 (s, 1 P) 150.85 (s, 1 P) 151.40 (s, 1 P) 151.43 (s, 1 P) ; HRMS (ESI) for C₆₃H₈₉N₇O₁₇P₂Si₂ Mass calc. 1333.53, found: 1356.67 (M+Na⁺).

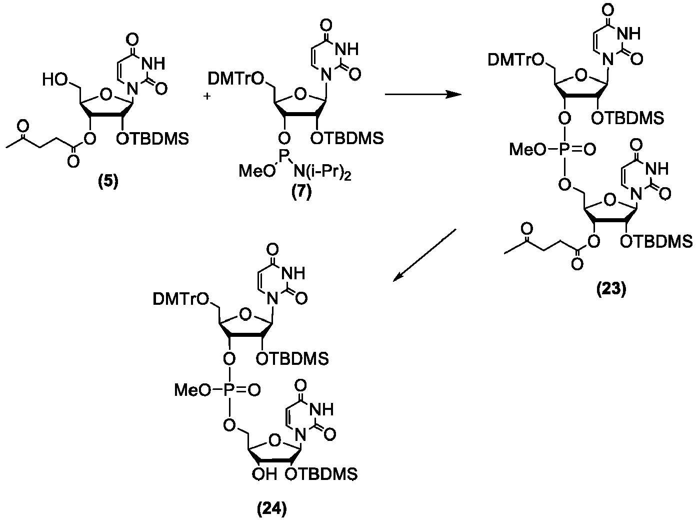

### [5'-DMTr-2'TBDMS-rU]-[3'-p(OMe)-5']-[rU-2'TBDMS-3'-Lev] (23)

Compound **(5)** (1.55 g, 3.39 mmol) was dissolved in 26 mL of THF (0.15M) with 2eq of DCI. To this stirring solution (3.21 g, 3.90 mmol) of phoshoramidite **(7)** was added all at once and allowed to stir at room temperature for 2h. The reaction was monitored by TLC (5%MeOH in DCM) and after 2 hours the reaction was complete. At this point 10eq of tert-butyl hydroperoxide was added and allowed to stir for 20min, until all the phosphite triester was converted to the phosphate, as monitored by TLC (5% MeOH in DCM) observing a more polar spot appear. The solution was condensed to approximately 10ml and then dissolved in ethyl acetate and extracted with 5% sodium sulfite (x2) and brine (x1). The organic layer was dried and condensed and put on a short column (0→5% MeOH in DCM). Yield: 3.12 g (84%).

¹H NMR (500 MHz, ACETONITRILE-*d*₃) δ ppm 0.05 - 0.17 (m, 6 H) 0.88 - 0.97 (m, 11 H) 1.97 (dt, *J*=4.95, 2.54 Hz, 1 H) 2.03 (s, 1 H) 2.12 (d, *J*=2.45 Hz, 2 H) 2.53 - 2.64 (m, 2 H) 2.72 - 2.79 (m, 1 H) 3.39 - 3.48 (m, 2 H) 3.51 - 3.55 (m, 1 H) 3.56 - 3.61 (m, 1 H) 3.63 - 3.66 (m, 1 H) 3.70 (d, *J*=11.25 Hz, 1 H) 3.77 (s, 1 H) 3.78 - 3.82 (m, 4 H) 4.03 - 4.13 (m, 1 H) 4.13 - 4.26 (m, 2 H) 4.29 - 4.36 (m, 1 H) 4.41 (dt, *J*=13.88, 5.53 Hz, 1 H) 4.47 (t, *J*=4.89 Hz, 1 H) 4.78 - 4.84 (m, 1 H) 4.85 - 4.91 (m, 1 H) 5.24 (t, *J*=4.89 Hz, 1 H) 5.37 (dd, *J*=8.19, 5.26 Hz, 1 H) 5.59 (dd, *J*=12.23, 8.07 Hz, 1 H) 5.77 (d, *J*=4.40 Hz, 1 H) 5.81 (d, *J*=4.65 Hz, 1 H) 5.87 (dd, *J*=13.94, 5.38 Hz, 2 H) 6.88 - 6.94 (m, 2 H) 7.26 - 7.42 (m, 11 H) 7.42 - 7.49 (m, 1 H) 7.69 (dd, *J*=10.64, 8.19 Hz, 1 H) 9.20 - 9.36 (m, 2 H); ³¹P NMR (81 MHz, ACETONITRILE-d₃) δ ppm 0.47 (s, 1 P) 0.67 (s, 1 P); HRMS (ESI) for C₅₇H₇₇N₄O₁₈PSi₂ calcd: 1192.4509, found: 1215.32 (M + Na⁺).

### [5'-DMTr-2'TBDMS-rU]-[3'-p(OMe)-5']-[rU-2'TBDMS] (24)

Compound **(23)** (1.26g, 1.06mmol) was dissolved in minimal amounts of ACN, just enough to get it into solution. It was then treated with ten equivalents (21ml, 10.6mmol) of a 0.5M hydrazine hydrate solution in 3:2 pyridine:acetic acid that was prepared immediately before the reaction. The reaction mixture was allowed to stir for 10min before it was chilled to 0°C in an ice bath and ten equivalents (0.728ml, 10.6mmol) of 2,4-pentanedione which was allowed to stir for an additional 5min before the reaction was diluted with MTBE and extracted x3 with ammonium chloride (5%w/v) and once with cupric sulfate, and once with brine. The sample was then dried over MgSO₄ and condensed to dryness. The sample was put on a short column and run with MeOH 0→5% in chloroform. Yield: 1.20g (95%) (impure).

¹H NMR (400 MHz, ACETONITRILE-d₃) of major component δ ppm -0.11 - 0.07 (m, 7 H) 0.07 - 0.25 (m, 7 H) 0.80 - 0.94 (m, 20 H) 1.84 - 2.05 (m, 3 H) 2.07 - 2.31 (m, 6 H) 2.48 - 2.70 (m, 3 H) 2.75 (m, 3 H) 3.33 - 3.54 (m, 3 H) 3.66 - 3.85 (m, 10 H) 4.15 - 4.41 (m, 6 H) 4.47 (t, *J*=4.89 Hz, 2 H) 4.73 - 4.99 (m, 2 H) 5.00 - 5.24 (m, 2 H) 5.37 (dd, *J*=8.22, 1.57 Hz, 2 H) 5.42 - 5.54 (m, 1 H) 5.64 (d, *J*=7.83 Hz, 1 H) 5.72 - 5.91 (m, 3 H) 6.80 - 6.98 (m, 5 H) 7.24 - 7.40 (m, 8 H) 7.40 - 7.57 (m, 4 H) 7.68 (dd, *J*=8.22, 5.09 Hz, 2 H) 9.30 (br. s., 3 H); ³¹P NMR (81 MHz, ACETONITRILE-d₃) δ ppm 0.49 (s, 1 P) 0.58 (s, 1 P) 0.74 (s, 1 P) 0.78 (s, 1 P) ); Mass calcd: 1094.41, found: 1117.42 (M + Na⁺).

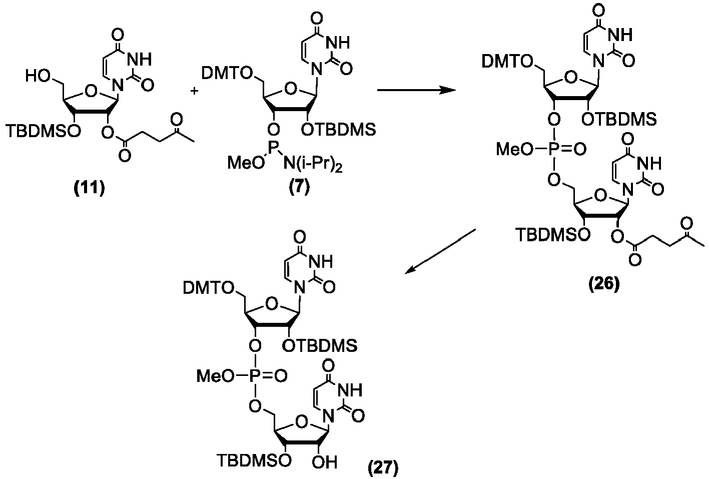

Synthesis steps for **(26)** and **(27)** were identical to those previously described for compounds **(23)** and **(24).**

### [5'-DMTr-2'-TBDMS-rU]-[3'-p(OMe)-5']-[rU-2'-Lev-3'-TBDMS] (26)

Compound **(11)** (0.23 g, 0.50 mmol) was dissolved in 5.0 mL of THF (0.15M) with 2 eq of DCI (0.12 g, 1.0 mmol). To this stirring solution (0.476 g, 0.579 mmol) of phoshoramidite **(7)** was added all at once and allowed to stir at room temperature for 2h. The reaction was monitored by TLC (5%MeOH in DCM) and after 2 hours the reaction was complete. At this point 10eq of tert-butyl hydroperoxide was added and allowed to stir for 20min, until all the phosphite triester was converted to the phosphate, as monitored by TLC (5% MeOH in DCM) observing a more polar spot appear. The solution was condensed to approximately 10ml and then dissolved in ethyl acetate and extracted with 5% sodium sulfite (x2) and brine (x1). The organic layer was dried and condensed and put on a short column (0→5% MeOH in DCM). Yield: 0.50 g (88%).

¹H NMR (500 MHz, acetone) δ ppm 0.04 - 0.23 (m, 13 H) 0.80 - 0.96 (m, 19 H) 1.99 - 2.14 (m, 7 H) 2.54 - 2.70 (m, 3 H) 2.72 - 2.94 (m, 4 H) 3.46 - 3.63 (m, 3 H) 3.73 (d, *J*=11.25 Hz, 2 H) 3.78 - 3.95 (m, 8 H) 4.13 (d, *J*=3.67 Hz, 1 H) 4.16 - 4.22 (m, 1 H) 4.23 - 4.34 (m, 2 H) 4.35 - 4.50 (m, 2 H) 4.51 - 4.70 (m, 3 H) 4.92 - 5.07 (m, 2 H) 5.19 - 5.40 (m, 3 H) 5.48 - 5.64 (m, 2 H) 5.86 - 6.00 (m, 3 H) 6.83 - 6.96 (m, 5 H) 7.20 - 7.39 (m, 8 H) 7.44 - 7.53 (m, 2 H) 7.57 (d, *J*=8.07 Hz, 1 H) 7.65 (d, *J*=8.07 Hz, 1 H) 7.86 (d, *J*=8.31 Hz, 1 H) 7.82 (d, *J*=8.07 Hz, 1 H) 10.13 (br. s., 3 H); ³¹P NMR (81 MHz, ACETONITRILE-d₃) δ ppm 0.48 (s, 1 P) 0.68 (s, 1 P).

### [5'-DMTr-2'-TBDMS-rU]-[3'-p(OMe)-5']-[rU-3'-TBDMS] (27)

Compound **(26)** (0.210g, 0.176 mmol) was dissolved in minimal amounts of ACN, just enough to get it into solution. It was then treated with ten equivalents (3.52 mL) of a 0.5 M hydrazine hydrate solution in 3:2 pyridine:acetic acid that was prepared immediately before the reaction. The reaction mixture was allowed to stir for 10 min before it was chilled to 0°C in an ice bath and ten equivalents (0.18 mL, 1.75 mmol) of 2,4-pentanedione which was allowed to stir for an additional 5 min before the reaction was diluted with MTBE and extracted x3 with ammonium chloride (5%w/v) and once with cupric sulfate, and once with brine. The sample was then dried over MgSO₄ and condensed to dryness. The sample was put on a short column and run with MeOH 0 → 4 % in chloroform. Yield: 0.162g (85%).

¹H NMR (500 MHz, ACETONITRILE-*d*₃) δ ppm -1.93 - 0.51 (m, 13 H) 0.88 - 0.96 (m, 11 H) 1.89 (s, 3 H) 1.97 (dt, *J*=5.07, 2.48 Hz, 1 H) 2.03 (s, 1 H) 2.21 (s, 3 H) 3.41 - 3.48 (m, 1 H) 3.50 - 3.67 (m, 2 H) 3.78 (m, *J*=1.47 Hz, 4 H) 3.98 - 4.29 (m, 4 H) 4.34 (dd, *J*=5.62, 3.18 Hz, 1 H) 4.48 (t, *J*=5.01 Hz, 1 H) 4.67 - 5.15 (m, 2 H) 5.39 (dd, *J*=8.19, 1.83 Hz, 2 H) 5.62 (t, *J*=8.19 Hz, 1 H) 5.76 (dd, *J*=12.84, 4.28 Hz, 1 H) 5.85 (br. s., 1 H) 5.89 (t, *J*=5.87 Hz, 1 H) 6.23 (br. s., 2 H) 6.91 (dd, *J*=8.93, 3.30 Hz, 4 H) 7.13 - 7.41 (m, 5 H) 7.41 - 7.59 (m, 2 H) 7.71 (t, *J*=8.07 Hz, 4 H) 9.78 (br. s., 3 H); ³¹P NMR (81 MHz, ACETONITRILE-d₃) δ ppm 0.48 (s, 1 P) 0.57 (s, 1 P) 0.73 (s, 1 P) 0.79 (s, 1 P).

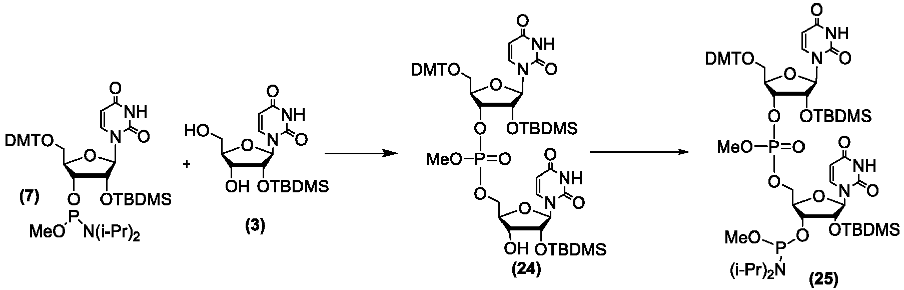

### [5'-DMTr-2'-TBDMS-rU]-[3'-p(OMe)-5']-[rU-2'-TBDMS] (24)

A concentrated solution of phosphoramidite **(7)** (4.40g, 5.35mmol, 1.01 eq), DCI (1.25 g, 10.6 mmol, 2 eq) in acetonitrile (ACN, 26.5mL) were added to a flamed dried flask and allowed to stir until all reagents were dissolved. In a separate vessel the 2'-TBDMS nucleoside **(3)** (1.9 g, 5.30 mmol, 1.0 eq) was dissolved in 20 mL of ACN. The solution of activated amidite was then cannulated slowly (drop-wise) into the flask with the nucleoside. This mixture was allowed to stir for 20 min before a TLC was run (10% MeOH in DCM) showing complete consumption of the amidite. tert-Butyl hydroperoxide (6M in decane, 5.3 mL, 5 eq) was then added to the solution and allowed to stir for another 20 min. The sample was then diluted with MTBE and extracted x3 with a 5% aqueous solution of sodium sulphite and once with brine. The mixture was then dried over magnesium sulphate and evaporated to dryness. The sample was then purified by silica gel chromatography using 0-4% MeOH in DCM as eluting solvent to give the pure product in reasonable yield (Note this was a difficult column as the starting nucleoside had similar polarity to the oxidised dimer): 3.7 g (68%) (impure).

¹H NMR (400 MHz, ACETONITRILE-d₃) δ ppm -0.11 - 0.07 (m, 7 H) 0.07 - 0.25 (m, 7 H) 0.80 - 0.94 (m, 20 H) 1.84 - 2.05 (m, 3 H) 2.07 - 2.31 (m, 6 H) 2.48 - 2.70 (m, 3 H) 2.75 (q, *J*=6.26 Hz, 3 H) 3.33 - 3.54 (m, 3 H) 3.66 - 3.85 (m, 10 H) 4.15 - 4.41 (m, 6 H) 4.47 (t, *J*=4.89 Hz, 2 H) 4.73 - 4.99 (m, 2 H) 5.00 - 5.24 (m, 2 H) 5.37 (dd, *J*=8.22, 1.57 Hz, 2 H) 5.42 - 5.54 (m, 1 H) 5.64 (d, *J*=7.83 Hz, 1 H) 5.72 - 5.91 (m, 3 H) 6.80 - 6.98 (m, 5 H) 7.24 - 7.40 (m, 8 H) 7.40 - 7.57 (m, 4 H) 7.68 (dd, J=8.22, 5.09 Hz, 2 H) 9.30 (br. s., 3 H); ³¹P NMR (81 MHz, ACETONITRILE-d₃) δ ppm 0.56 (s, 1 P) 0.77 (s, 1 P).

### [5'-DMTr-2'-TBDMS-rU]-[3'-p(OMe)-5']-[rU-2'-TBDMS]-3'-methylphosphoramidite (25)

Compound **(24)** (3.70 g 3.43 mmol) was dissolved in 10.0ml of dry THF. The nucleotide solution was added drop wise over 20 min to a solution of N,N-diisopropylamino)methylphosphonamidic chloride 2.5eq (1.694 g, 1.729 ml, 8.57 mmol) and diisopropylethylamine (3 eq, 1.867 ml). The reaction was monitored by TLC (5% MeOH in DCM). Excess amidite ran close to the newly formed phosphitylated dimer, but once the reaction was worked up the quenched product had a much lower polarity. Once the reaction was complete, 1.5h, the reaction mixture was diluted with ethyl acetate pre washed with sat. NaHCO₃, it was then extracted once with a solution of 5% NaHCO₃ and x2 with brine. The organic phase was dried with MgSO₄, condensed to dryness, co evaporated with toluene (x2). With the methoxy phosphate protecting group there was no worry of internucleotide phosphate deprotection on column under basic conditions therefore triethylamine can be used to quench the silica as a typical phosphoramidite purification. The compound was purified by 1% triethylamine 49% DCM 50% ethyl acetate. Yield 3.701 g (87%)

¹H NMR (500 MHz, ACETONITRILE-d₃) of major component. δ ppm 0.01 - 0.20 (m, 10 H) 0.78 - 0.96 (m, 15 H) 1.11 - 1.32 (m, 12 H) 1.83 - 2.01 (m, 6 H) 2.20 (s, 4 H) 2.59 - 2.74 (m, 2 H) 2.78 (s, 1 H) 3.37 (d, J=7.34 Hz, 2 H) 3.41 - 3.54 (m, 3 H) 3.63 - 3.75 (m, 4 H) 3.76 - 3.88 (m, 7 H) 4.01 - 4.24 (m, 3 H) 4.25 - 4.41 (m, 4 H) 4.41 - 4.63 (m, 2 H) 4.73 - 4.88 (m, 2 H) 4.95 (s, 1 H) 5.32 - 5.39 (m, 1 H) 5.48 - 5.70 (m, 2 H) 5.77 - 5.97 (m, 3 H) 6.75 - 6.94 (m, 4 H) 7.17 - 7.38 (m, 7 H) 7.42 - 7.49 (m, 2 H) 7.49 - 7.58 (m, 1 H) 7.60 - 7.76 (m, 2 H); ³¹P NMR (81 MHz, ACETONITRILE-*d*₃) δ ppm 0.50 (s, 1 P) 0.64 (s, 1 P) 0.83 (s, 1 P) 0.86 (s, 1 P) 151.14 (s, 1 P) 151.18 (s, 1 P) 152.08 (s, 1 P) 152.11 (s, 1 P).

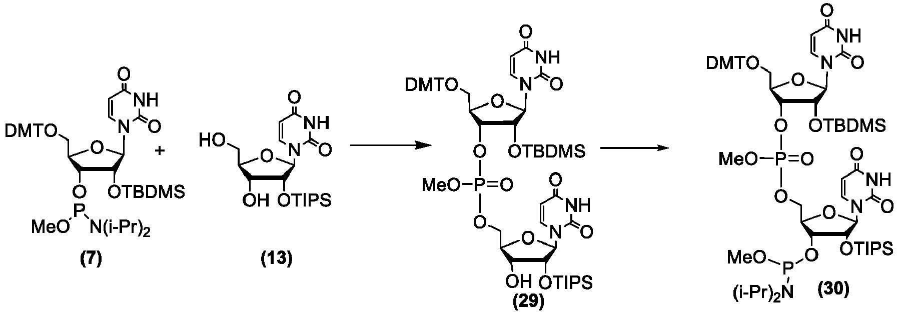

### [5'-DMTr-2'-TBDMS-rU]-[3'-p(OMe)-5']-[rU-2'-TIPS] (29)

A concentrated solution of phosphoramidite **(7)** (4.40 g, 5.35 mmol, 1.01 eq), DCI (1.25 g, 10.6 mmol, 2 eq) in acetonitrile (ACN, 26.5mL) were added to a flamed dried flask and allowed to stir until all reagents were dissolved. In a separate vessel the 2'-TIPS nucleoside **(13)** (1.9g, 5.30mmol, 1.0eq) was dissolved in 20 mL of ACN (note: for rA nucleoside a small amount of THF was added due to solubility issues). The solution of activated amidite was then cannulated slowly (drop-wise) into the flask with the nucleoside. This mixture was allowed to stir for 20 min before a TLC was run (10% MeOH in DCM) showing complete consumption of the amidite. *tert*-Butyl hydroperoxide (6M in decane, 5.3 mL, 5 eq) was then added to the solution and allowed to stir for another 20 min. The sample was then diluted with MTBE and extracted x3 with a 5% aqueous solution of sodium sulphite and once with brine. The mixture was then dried over magnesium sulphate and evaporated to dryness. The sample was then purified by silica gel chromatography using 0-4% MeOH in DCM as eluting solvent to give the pure product in 82% yield (3.7 g).

³¹P NMR (81 MHz, ACETONITRILE-d₃) δ ppm 0.57 (s, 1 P) 0.81 (s, 1 P) .

Mass calc. 1137.36 Mass found. 1159.53 (M+Na⁺).

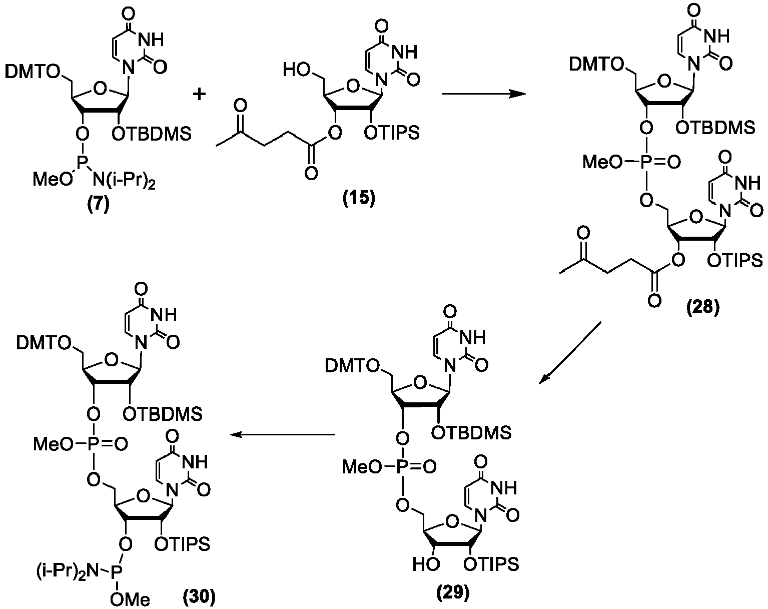

### [5'-DMTr-2'TBDMS-rU]-[3'-p(OMe)-5']-[rU-2'-TIPS-3'-Lev] (28)

DCI (1.08 g, 9.18 mmol, 2 eq) was added to a 30 mL ACN solution of the phosphoramidite **(7)** (5.66 g, 6.89 mmol, 1.5 eq) and the nucleoside **(15)** (2.289 g, 4.59 mmol, 1.0 eq). The mixture was allowed to stir for 20-30 min until the starting nucleoside was completely consumed. One equivalent of tert-butanol (0.5 mL) was added to quench any remaining active amidite (this helps with the next purification step as the quenched amidite becomes less polar and easier to separate from the desired dimer). Five equivalents of *tert*-butyl hydroperoxide (6 M; 3.06 mL) were added and the resulting mixture allowed to stir for an additional 15 min until all of the phosphite triester was consumed (TLC analysis with 10% MeOH in DCM). The sample was then diluted with MTBE and extracted x3 with a 5% solution of sodium bisulphite and once with brine. The mixture was then dried over magnesium sulphate and condensed to dryness. The sample was then purified by column chromatography (0-75% gradient of ethyl acetate in hexane). Yield: 5.29 g (93%).

³¹P NMR (81 MHz, ACETONITRILE-*d*₃) δ ppm 0.39 (s, 1 P) 0.81 (s, 1 P) .

### [5'-DMTr-2'-TBDMS-rU]-[3'-p(OMe)-5']-[rU-2'-TBDMS] (29)

To a solution of **(28)** 9.53g (7.71 mmol) in ACN (10 mL) was added 77 mL (5 eq) of 0.5 M hydrazine hydrate in pyridine/acetic acid (3:2) and the resulting mixture allowed to stir for 20 min. The reaction was worked up by cooling the reaction mixture to 0°C in an ice bath and adding 5 eq of 2,4-pentanedione (3.96 mL, 38.6 mmol) to quench excess hydrazine. At this point, the mixture was removed from the ice bath and allowed to stir for an additional 10 min at room temperature. The mixture was diluted with MTBE or ethyl acetate and extracted x4 with 5% ammonium chloride to keep the aqueous medium slightly acidic, then once with brine to remove any residual ammonium chloride. Then the mixture was extracted twice with 3% aqueous CuSO₄ solution (to mop up excess pyridine) then once with 1% EDTA and 10% NaCl to remove any residual copper from the organic layer. The organic layer was then dried with magnesium sulphate and condensed to dryness. This mixture was then purified by column in ethyl acetate/hexanes (0-75% gradient). Yield: 8.25 g (94%).

¹H NMR (300 MHz, ACETONITRILE-*d*₃) δ ppm 0.09 - 0.15 (m, 6 H) 0.87 - 1.20 (m, 40 H) 1.94 - 2.00 (m, 1 H) 2.10 - 2.20 (m, 2 H) 3.44 (br. s., 3 H) 3.67 - 3.79 (m, 12 H) 4.04 - 4.23 (m, 5 H) 4.28 - 4.35 (m, 2 H) 4.38 - 4.48 (m, 2 H) 4.81 - 4.90 (m, 1 H) 5.38 (d, J=8.21 Hz, 1 H) 5.64 (d, *J*=8.20 Hz, 1 H) 5.81 - 5.91 (m, 2 H) 6.90 (d, *J*=7.91 Hz, 5 H) 7.24 - 7.37 (m, 9 H) 7.41 - 7.52 (m, 4 H) 7.71 (dd, *J*=8.20, 2.64 Hz, 1 H) 9.62 (br. s., 1 H) ³¹P NMR (81 MHz, ACETONITRILE-*d*₃) δ ppm 0.58 (s, 1 P) 0.83 (s, 1 P).

### [5'-DMTr-2'-TBDMS-rU]-[3'-p(OMe)-5']-[rU-2'-TIPS]-3'-methylphosphoramidite (30)

N,N-Diisopropylammonium tetrazolide (14.1 mmol, 2.41 g) and bis (N,N-diisopropylamino)methylphosphine (14.1 mmol, 3.69 g) were added to 26.8mL of ACN. After stirring for a few minutes, the mixture was sonicated for 0.5h until the majority of the tetrazolide salt had dissolved. The dimer **(29)** (4.01 mmol, 4.566 g) was then added directly to this solution and sonicated for an additional 8h; finally the mixture was allowed to stir overnight at room temperature. The mixture was then diluted with either MTBE or ethyl acetate (the ethyl acetate must be prewashed with saturated sodium bicarbonate to remove residual acetic acid) and extracted with a 10% NaCl solution x2 then once with brine. The organic layer was then dried with magnesium sulphate and condensed to dryness. The mixture was then taken up in minimal amounts of DCM and precipitated in petroleum ether to remove amines (-78°C) . The ppt was filtered over Celite^{™} and collected with DCM. The solution was evaporated and the resulting solid purified by column chromatography. The column was first loaded with 3% triethylamine in hexanes. The sample was then dissolved with 17% hexanes, 3% triethylamine, 80% DCM and loaded on the column. The compound was eluted with 0-10% ethyl acetate/DCM/3%triethylamine. Yield: 3.5 g (67%).

³¹P NMR (81 MHz, CHLOROFORM*-d*) δ ppm 0.59 (s, 1 P) 0.77 (s, 1 P) 1.09 (s, 1 P) 1.14 (s, 1 P) 151.25 (s, 1 P) 151.38 (s, 1 P) 151.99 (s, 1 P) 152.05 (s, 1 P).

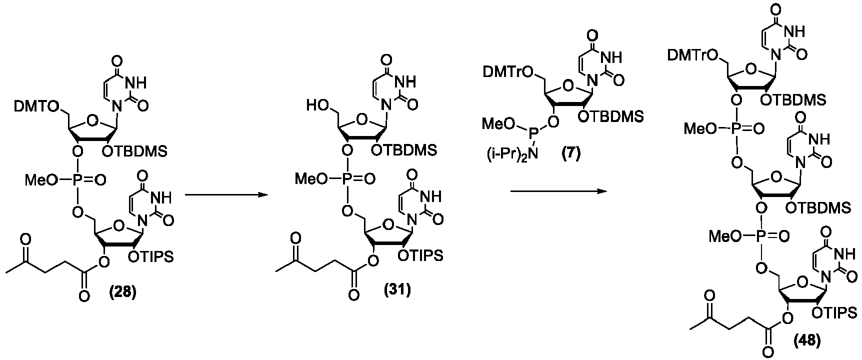

### [2'-TBDMS-rU]-[3'-p(OMe)-5']-[rU-2'-TIPS-3'-Lev] (31)

Compound **(28)** (5.29g, 4.28 mmol) was dissolved in 75ml of DCM and 3% trifluoroacetic acid (TFA) (vol/vol) and allowed to stir for 5min then 20ml of MeOH was added. The solution was immediately condensed to an oil and the process was repeated with another 25ml of DCM/TFA. The sample was then re- suspended in DCM and loaded on a column. The DCM was washed off with hexanes and the tritanol was eluted with hexanes:ethyl acetate 50:50, the product was eluted with 80% ethyl acetate. Yield: 3.88 g (97%).

¹H NMR (400 MHz, ACETONITRILE-*d*₃) δ ppm -0.08 - 0.13 (m, 8 H) 0.75 - 0.95 (m, 13 H) 0.96 - 1.10 (m, 27 H) 1.20 (s, 1 H) 1.78 - 1.91 (m, 1 H) 1.91 - 2.04 (m, 2 H) 2.07 - 2.32 (m, 5 H) 2.52 - 2.71 (m, 3 H) 2.71 - 2.97 (m, 4 H) 3.70 - 3.87 (m, 7 H) 4.22 - 4.40 (m, 6 H) 4.41 - 4.63 (m, 4 H) 4.70 - 4.87 (m, 2 H) 5.15 (ddd, *J*=14.18, 5.38, 3.13 Hz, 2 H) 5.23 - 6.21 (m, 4 H) 5.67 - 5.95 (m, 6 H) 7.59 (dd, *J*=8.22, 5.09 Hz, 2 H) 7.85 (dd, *J*=8.22, 2.74 Hz, 3 H) 9.50 (s, 1 H) 9.46 (s, 2 H).

### [5'-DMTr-2'-TBDMS-rU]-[3'-p(OMe)-5']-[rU-2'-TBDMS]-[3'-p(OMe)-5']-[rU-2'-TIPS-3'-Lev] (48)

Compound **(31)** (3.88g, 4.16mmol) was dissolved in 15ml of THF with 2eq of DCI (0.982g, 8.32mmol). Immediately phoshoramidite **(7)** (4.10g 4.99mmol) was added to the solution and allowed to stir at room temperature for 2h. The reaction was monitored by TLC (5% MeOH in DCM) and after 2 hours the reaction was usually complete.

At this point 10 eq (2.5 ml of a 6 M solution in decane) of tert-butyl hydroperoxide was added to solution and allowed to stir for 20min until all the phosphite triester was converted to the phosphate, as monitored by TLC (5% MeOH in DCM) observing a more polar spot appear. The solution was condensed to approximately 10ml and then dissolved in ethyl acetate and extracted with 5% sodium sulfite (x2) and brine (x1). The organic layer was dried and condensed and put on a short column (0→5% MeOH in DCM) yielding 6.10 g of **(48)** (88%).

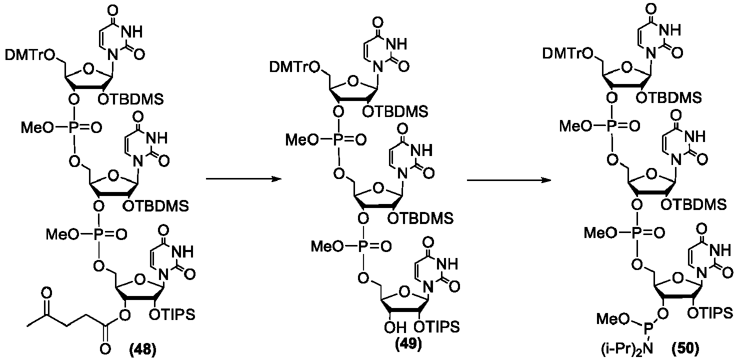

### [5'-DMTr-2'-TBDMS-rU]-[3'-p(OMe)-5']-[rU-2'-TBDMS]-[3'-p(OMe)-5']-[rU-(2'-TIPS] (49)

The levulinyl protected sample **(48)** (6.01 g 3.64 mmol) was dissolved in minimal amounts of ACN, just enough to get it into solution. It was then treated with 5 equivalents (36.4 ml, 18.2 mmol) of a 0.5 M hydrazine hydrate solution in 3:2 pyridine:acetic acid that was prepared immediately before the reaction. The reaction mixture was allowed to stir for 10min before it was chilled to 0°C in an ice bath and 5 equivalents (1.87ml, 18.2mmol) of 2, 4-pentanedione was added which was allowed to stir for an additional 5min before the reaction was diluted with MTBE and extracted x3 with ammonium chloride (5%w/v) and once with cupric sulfate, then once with a 1% solution of EDTA in 10% NaCl (w/vol) and lastly once with brine. The sample was then dried over MgSO₄ and condensed to dryness. The sample was re-suspended in chloroform, loaded on a column and eluted with ethyl acetate:hexanes 0→80%. Yield: 5.58 g (98%).

³¹P NMR (81 MHz, CHLOROFORM-*d*) δ ppm -0.52 (s, 1 P) 0.27 (s, 1 P) 0.40 (s, 1 P) 0.44 (s, 1 P) 0.64 (s, 1 P) 0.71 (s, 1 P) 0.83 (s, 1 P) 0.94 (s, 1 P).

### [5'-DMTr-2'-TBDMS-rU]-[3'-p(OMe)-5']-[rU-2'-TBDMS]-[3'-p(OMe)-5']-[rU-2'-TIPS]-3'-methylphosphoramidite (50)

N,N-Diisopropylammonium tetrazolide 3.6eq (2.52 g, 14.7 mmol) and bis-(N,N-diisopropylamino)methylphosphine 3.5 eq (3.76 g, 4.11ml, 14.3 mmol) were added to 27.3 mL of DCM. After stirring for a few minutes, the mixture was sonicated for 0.5 h until the majority of the tetrazolide salt had dissolved. The trimer **(49)** (6.433 g, 4.09 mmol) was then added directly to this solution and sonicated for an additional 8 h; finally the mixture was allowed to stir overnight at room temperature. The mixture was then diluted with either MTBE or ethyl acetate (the ethyl acetate must be prewashed with saturated sodium bicarbonate to remove residual acetic acid) and extracted with a 10% NaCl solution x2 then once with brine. The organic layer was then dried with magnesium sulphate and condensed to dryness. The mixture was then taken up in minimal amounts of DCM and precipitated in petroleum ether to remove amines (-78°C) . The precipitate was filtered over Celite^{™} and collected with DCM. The solution was evaporated and the resulting solid purified by column chromatography. The column was first loaded with 3% triethylamine in hexanes. The sample was then dissolved with 17% hexanes, 3% triethylamine, 80% DCM and loaded on the column. The compound was eluted with 0-10% ethyl acetate/DCM/3%triethylamine. Yield: 5.433 g (76%).

³¹P NMR (81 MHz, CHLOROFORM*-d*) δ ppm -1.19 (s, 1 P) -0.71 (s, 1 P) -0.09 (s, 1 P) 0.23 (s, 1 P) 0.32 (s, 2 P) 0.64 (s, 1 P) 0.74 (s, 2 P) 0.79 (s, 1 P) 0.86 (s, 1 P) 0.93 (s, 1 P) 1.08 (s, 1 P) 1.15 (s, 2 P) 1.23 (s, 1 P) 150.66 (s, 1 P) 150.80 (s, 1 P) 151.06 (s, 2 P) 153.00 (s, 1 P) 153.06 (s, 2 P) 153.57 (s, 1 P).

### Synthesis of UpU dimers by the procedure described in WO 2009/064115

Phosphoramidite **(6)** (1.38 g, 1.60 mmol) was added to a solution of **(3)**(0.50 g, 1.39 mmol) in 10 mL of dry ACN. This mixture was then concentrated to dryness on the rotovap. This mixture was then re-dissolved in 10 mL of ACN, to which a solution of 5-benzylthiotetrazole (0.34 g, 1.74 mmol) in 5 mL of ACN was added. This was immediately condensed on the rotovap and concentrated to approximately 3 mL. This mixture was allowed to stir for an hour. This solution was cooled to 0°C in an ice bath and 15 mL of a 0.1M iodine:pyridine:water mixture was added and stirred for 5min. The iodine mixture was quenched by the addition of 5 mL of a 2M Na₂S₂O₃ solution. This mixture was then put on the rotovap and concentrated to a gum. The gum was re-suspended in ethyl acetate and extracted with NaHCO₃ (x3). The organic layer was dried with MgSO₄ and concentrated to dryness.

Spectral data was taken at this point, before purification.

The sample was then purified by column, ethyl acetate:hexanes 0→60% to yield 1.04 g (65%) of material that contained a mixture of inseparable 3'/2' regioisomers and other phosphorus containing impurities, presumably the 3'-3' linked moiety.

³¹P-NMR of crude material (see Figure 3A):
³¹P NMR (81 MHz, ACETONITRILE-*d*₃) δ ppm -1.83 (s, 1 P) -1.49 (s, 1 P) -1.42 (s, 1 P) -1.17 (s, 1 P) -1.11 (s, 1 P) -0.77 (s, 1 P) 8.53 (s, 1 P) 8.61 (s, 1 P) 8.65 (s, 1 P) 9.00 (s, 1 P).

³¹P-NMR of purified dimer **(20)** (see Figure **3B****):**
³¹P NMR (81 MHz, ACETONITRILE-*d*₃) δ ppm -1.59 (s, 1 P) -1.42 (s, 1 P) -1.31 (s, 1 P) -1.19 (s, 1 P) -0.79 (s, 1 P) -0.70 (s, 1 P) - 0.61 (s, 1 P).

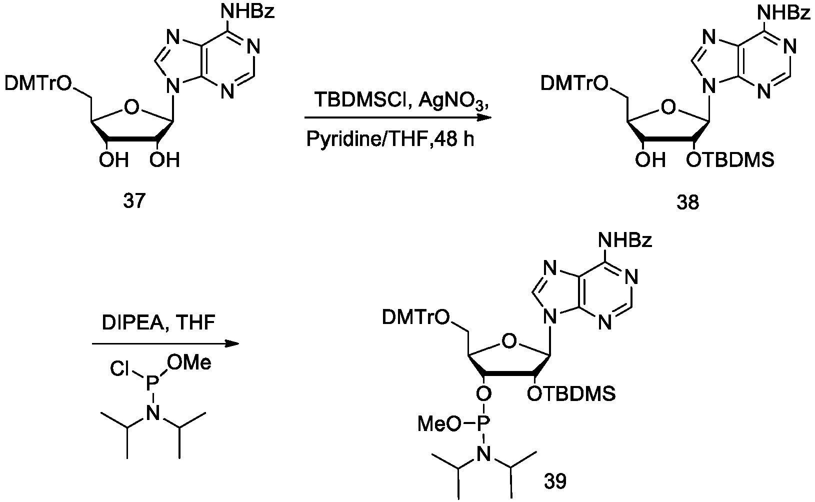

### 5'-DMTr-2'-TBDMS-N-benzoyl-adenosine (38)

5'-DMTr-N-benzoyl-adenosine **(37)** (12.3 g, 18.25 mmol) was dissolved in dry THF (183 mL). Dry pyridine (5.92 mL, 73 mmol) and AgNO₃ (4.03 g, 23.73 mmol) were added and stirring was continued for 5 min. Once almost all AgNO₃ dissolved, TBDMSCl (3.57 g, 23.73 mmol) was added all at once and the resulting cloudy milky solution was stirred at room temperature for 12h. The reaction mixture was filtered into 5% NaHCO₃ solution to avoid deprotection of DMT. The reaction mixture was extracted with DCM, evaporated and purified through column chromatography to give the product (7 g, 49%).

¹H NMR (400 MHz, CDCl₃) δ 8.97 (s, 1H), 8.74 (s, 1H), 8.23 (s, 1H), 8.03 (d, *J* = 7.8, 2H), 7.65-7.18 (m, 12H), 6.82 (d, *J* = 8.7, 4H), 6.11 (d, *J* = 5.4, 1H), 5.03 (t, *J* = 5, 1H), 4.36 (d, *J* = 3.8, 1H), 4.29 (d, *J* = 3.1, 1H), 3.78 (s, 6H), 3.54 (m, 2H), 1.61 (br s, 1H), 0.84 (s, 9H), -0.01 (s, 3H), -0.15 (s, 3H).

¹³C NMR (300 MHz, acetone) δ 158.64, 145, 142.56, 135.92, 132.74, 130.14,128.55, 128.3, 128.1, 127.71, 126.74, 113.01, 88.64, 86.34, 83.73, 75.63, 70.45, 63.35, 54.26, 25.2, 17.8, -5.71, - 5.51.

ESI-HRMS for C₄₄H₄₉N₅O₇SiNa⁺: Calcd 810.32925, found 810.32986.

### 5'-DMTr-2'-TBDMS-N-benzoyl-adenosine 3'-methylphosphoramidite (39)

To a stirred THF solution of compound **(38)** (7.3 g, 9.31 mmol) was added to the (N, N-diisopropylamino) methylphosphonamidic chloride (5.58 mL, 27.93 mmol) and diisopropylethylamine (8 mL, 46.55 mmol) slowly over 10 min. The reaction was stirred at RT for 24 h. The complete consumption of starting material was confirmed by the TLC. EtOAc (150 mL pre washed with 5% NaHCO₃) was added and the resulting solution washed with saturated brine (5x100 mL). The aqueous washes were back extracted with EtOAc and the combined EtOAc phases were dried over anhydrous MgSO₄, filtered and evaporated under vacuum to yield a white foam. The crude product was precipitated in 20% DCM solutions of the amidites into cold (dry ice/isopropanol bath) rapidly stirred hexanes. The above precipitated product was purified by column chromatography on silica gel to get the product (6.9 g, 78.4%).

³¹P NMR (81 MHz, Acetone) δ 151.76 (s, 1P), 151.29 (s, 1P).

ESI-HRMS for C₅₁H₆₅N₆O₈PSiNa⁺: Calcd 971.42599, found 971.42602.

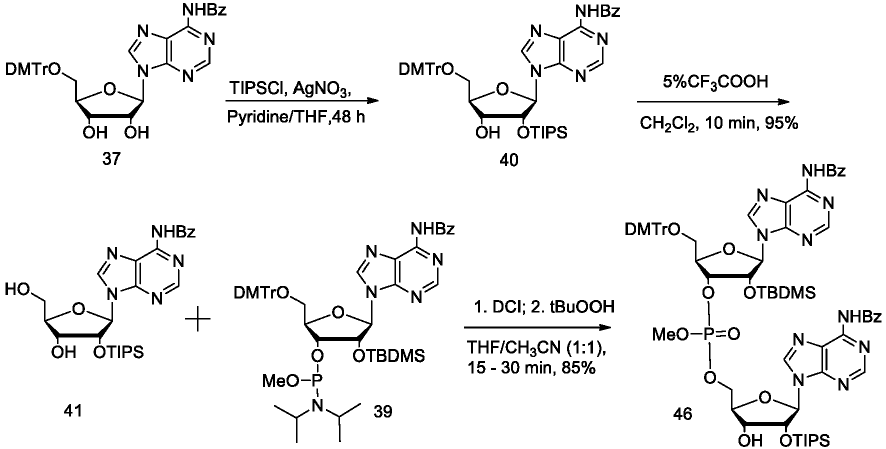

### 5'-DMTr-2'-TIPS-N-benzoyl-adenosine (40)

Compound **(37)** (10 g, 14.8 mmol) was dissolved in dry THF 150 mL. Dry pyridine and AgNO₃ were added and stirring was continued for 5 min, until almost all AgNO₃ dissolved. TIPSCl (3.5 mL, 16.62 mmol) was added all at once and the resulting cloudy milky solution was stirred at room temperature. After 12h, TLC analysis revealed there was still a lot of starting material in the reaction mixture. So, one more equivalent of AgNO₃ and TIPSCl was added and the mixture was stirred for another 12 h. The reaction mixture was filtered into 5% NaHCO₃ solution to avoid deprotection of DMT. The reaction mixture was extracted with DCM, evaporated and purified through column chromatography. The unreacted starting material was recovered and reused for the next time. Yield 2'-TIPS nucleoside **(40)** 4.7 g, 38%, and 3'-TIPS nucleoside 1.5 g, 12%.

¹H NMR (400 MHz, Acetone) δ 9.40 (s, 1H), 8.68 (s, 1H), 8.41 (s, 1H), 8.12 (d, *J* = 7.2, 2H), 7.82 - 7.27 (m, 12H), 7.03 - 6.86 (m, 4H), 6.19 (d, *J* = 5.1, 1H), 5.33 (t, *J* = 4.7, 1H), 4.55 (d, *J* = 4.2, 2H), 4.41 - 4.28 (m, 2H), 3.87 (s, 6H), 3.61 - 3.18 (m, 0H), 2.28 (br s, 1H), 1.25 - 0.91 (m, 21H).

¹³C NMR (300 MHz, Acetone) δ 171.17, 158.73, 151.86, 150.18, 145.16, 143.18, 135.87, 134.29, 132.61, 130.16, 128.69, 128.27, 128, 126.99, 124.95, 117.49, 113.19, 89.53, 86.38, 83.54, 75.4, 70.8, 63, 59.3, 55, 20.4, 17.4, 17.2, 13.8, 12, 0.59.

ESI-HRMS for C₄₇H₅₅N₅O₇SiNa⁺: Calcd 852.37579, found 852.37587.

### 2'-TIPS-N-benzoyl-adenosine (41)

Compound **(40)** (1 g, 1.203 mmol) was dissolved in 12 mL of DCM and 5 eq of 3% trifluoroacetic acid (TFA) (vol/vol) and allowed to stir for 5min then a few drops of MeOH was added. The solvent was evaporated and the process was repeated one more time. The sample was then re-suspended in DCM and loaded on a column. The DCM was washed off with hexanes and the tritanol was eluted with hexanes:ethyl acetate 50:50; the product was eluted with 80% ethyl acetate. Yield 0.6 g, 95%.

¹H NMR (400 MHz, acetone) δ 10.01 (s, 1H), 8.67 (s, 1H), 8.59 (s, 1H), 8.13 (d, *J* = 7.5, 3H), 7.7-7.5 (m, 3H) , 6.17 (d, *J* = 6.5, 1H), 5.3 - 5.1 (m, 1H), 4.45-4.3 (m, 1H), 4.24 (d, *J* = 2.0, 1H), 3.9-3.6 (m, 2H), 2.83 (br s, 1H), 1.10 - 0.8 (m, 21H).

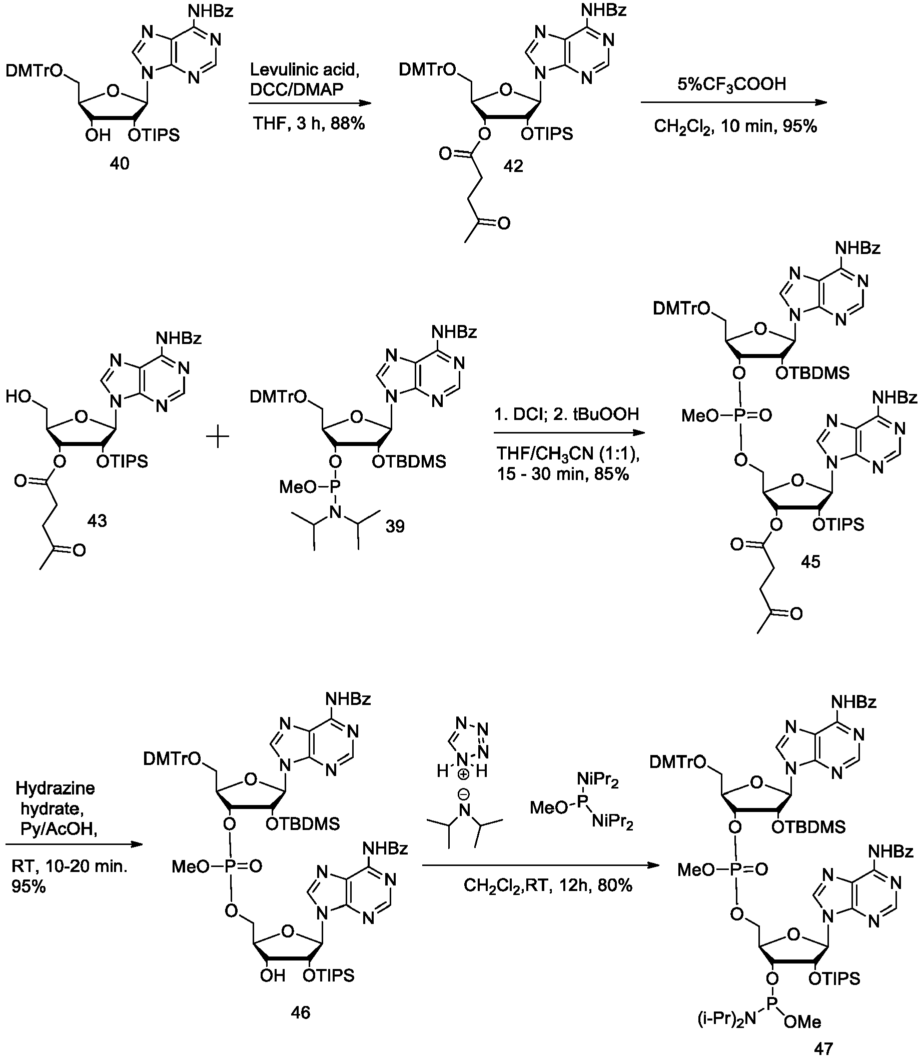

### 5'-DMTr-2'-TIPS-3'-levulinyl-N-benzoyl-adenosine (42)

Levulinic acid (0.21 g, 1.84 mmol), DCC (0.371 g 1.84 mmol) and DMAP (0.034 g, 0.30mmol) were dissolved in 3mL of THF and stirred at 0 °C for 10 min. Compound **(40)** (0.5 g, 0.613 mmol) was added dropwise to the above solution slowly at 0 °C. The reaction mixture was slowly warmed to room temperature and stirred an additional 3h. After the 3 h, the reaction mixture was slowly changing its colour to dark brown. TLC was monitored and the reaction was completed. The reaction mixture was filtered over Celite^{™}. The organic layer was washed with saturated NaHCO₃, and saturated NaCl. The compound was purified through column chromatography.

¹H NMR (400 MHz, Acetone) δ 9.99 (br s, 1H), 8.66 (s, 1H), 8.6 (s, 1H), 8.54 (d, *J* = 6.8, 2H), 8.13 (d, *J* =7.3, 2H), 7.85-7.18 (m, 10H), 6.86 (d, *J* = 10.1, 4H), 6.20 (d, *J* = 5.7, 1H), 5.73 - 5.53 (m, 1H), 4.37-4.3 (m, 1H), 3.78 (s, 6H), 3.62-3.3.36 (m, 2H), 3.11 - 2.52 (m, 4H), 2.16 (s, 3H), 1.29 (br s, 1H), 1.06 - 0.72 (m, 21H).

¹³C NMR (300 MHz, CDCl₃) δ 206, 174.6, 171.9, 170.8, 164.89, 158.5, 153.2, 152.5, 152, 150.7, 149.6, 144.5, 141.9, 135.4, 135.3, 134.1, 133.5, 132.6, 129.99, 129.3, 129.1, 128.63, 128.4, 128, 127.9, 126.8, 123.6, 113.1, 88, 86.7, 82.5, 73.75, 63.1, 60.1, 55, 37.6, 31.5, 29.7, 27.8, 20.8, 17.6, 17.4, 14.1, 11.9.

ESI-HRMS for C₅₂H₆₁N₅O₉SiNa⁺: calcd 950.41308, found 950.41355.

### 2'-TIPS-3'-levulinyl-N-benzoyl-adenosine (43)

Compound **(42)** (2.8 g, 3.02 mmol) was solvated in 12 mL of DCM and 5 eq of 3% trifluoroacetic acid (TFA) (vol/vol) and allowed to stir for 5min, then a few drops of MeOH was added. The solvent was evaporated and the process was repeated one more time. The sample was then re- suspended in DCM and loaded on a column. The DCM was washed off with hexanes and the tritanol was eluted with hexanes:ethyl acetate 50:50; the product was eluted with 80% ethyl acetate. Yield 1.75 g, 93%.

¹H NMR (400 MHz, CDCl₃) δ 9.0 (br s, 1H), 8.81 (s, 1H), 8.17 - 7.93 (m, 3H), 7.7-7.47 (m, 3H), 5.91 (d, *J* = 7.1, 1H), 5.48-5.32 (m, 2H), 4.29 (s, 1H), 3.90 (dd, *J* = 40.4, 12.9, 2H), 3.01 - 2.50 (m, 5H), 2.22 (s, 3H), 0.82 (d, *J* = 18.2, 21H).

¹³C NMR (300 MHz, CDCl₃) δ 206.2, 172, 165, 151.9, 150.5, 150.4, 143.3, 133.3, 132.6, 128.6, 127.9, 124.1, 90.7, 85.7, 74.6, 73, 62.4, 37.57, 29.66, 27.7, 17.3, 11.7.

ESI-HRMS for C₃₁H₄₃N₅O₇SiNa⁺: calcd 648.28240, found 648.28327.

### [5'-DMTr-2'TBDMS-N-Bz-rA]-[3'-p(OMe)-5']-[rA-2'TIPS-3'-Lev] (45)

DCI (0.223 g, 1.88mmol) was dissolved in 8 mL THF/CH₃CN (1:1) and added to the amidite **(39)** (1.63 g, 1.717 mmol). The above mixture was slowly added to the compound **(43)** (1.07 g, 1.717 mmol) in 8 mL THF/CH₃CN (1:1) at 0°C. The reaction mixture was slowly warmed to room temperature. After stirring for 30 min, 10 eq of tert-butyl hydroperoxide (6M in decane, 1.92 mL) was added to the reaction mixture and stirred for an additional 10 min. The reaction mixture was taken into MTBE and washed quickly with 5% sodium sulfite 2 times. The organic layer was dried over anhydrous MgSO₄ and subjected to column chromatography on silica gel using eluent EtOAc & DCM (0-50% EtOAc) to get the desired ApA dimer (2.15 g, 85%).

¹H NMR (400 MHz, Acetone) δ 9.97 (m, 2H), 8.70 (s, 2H), 8.69(s, 2H), 8.52-8.41 (m, 2H), 8.12 (d, *J* = 6.6, 2H), 7.72-7.13 (m, 15H), 6.94-6.77 (m, 4H), 5.64 - 5.34 (m, 2H), 5.22 - 5.04 (m, 2H), 4.62 - 4.36 (m, 4H), 3.76 (s, 9H), 3.64-3.37 (m, 2H), 2.16 (s, *J* = 4.9, 3H), 1.08 - 0.79 (m, 3H), 0.97 (s, 9H), 0.74 (d, *J* = 8.3, 18H), -0.01 (d, *J* = 7.9, 3H), -0.19 (d, *J* = 7.7, 3H).

¹³C NMR (300 MHz, Acetone) δ 172.0, 170.1, 165.3, 158.7, 152, 150.7, 145, 143.5, 142.6, 135.6, 134, 132.3, 130.1, 128.6, 127.9, 126.8, 125.4, 125.1, 113.1, 88.7, 88.3, 86.5, 82.8, 82.7, 81.4, 81.3, 77.6, 77.5, 77.2, 77.1, 73.7, 73.5, 72.8, 72.5, 67.1, 66.9, 63, 62.8, 60, 54.8, 54.4, 37.4, 25.2, 20.2, 17.7, 17.4, 17.2, 13.8, 12, -5.3, -5.8.

³¹P NMR (200 MHz, Acetone) δ 0.97 (s, 1P), 0.78 (s, 1P).

ESI-HRMS for C₇₆H₉₃N₁₀O₁₆Psi₂Na⁺: calcd 1511.59289, found 1511.59275.

### [5'-DMTr-2'TBDMS-N-Bz-rA]-[3'-p(OMe)-5']-[rA-2'TIPS](46)

Compound **(45)** (0.72 g, 0.484 mmol) was dissolved in minimum amounts of acetonitrile (1mL/mmol). 0.5 M hydrazine (0.244 mL) in pyridine/acetic acid (3:2) was added dropwise to the above reaction mixture at room temperature and stirred for 10-15 min. The reaction mixture was cooled to 0 °C and 2,4-pentanedione added at once (0.5 mL, 4.48mmol). The reaction mixture was warmed to room temperature and stirred for an additional 15 min. Reaction mixture was diluted with MTBE and washed 2 times with aq. NH₄Cl and saturated NaCl. The organic layer was dried over anhydrous MgSO₄, filtered and concentrated. The compound was subjected to column chromatography on silica gel using eluent DCM & EtOAc (0-50% EtAc) to get the product (0.64 g, 95%).

¹H NMR (400 MHz, Acetone) δ 10.01 (s, 2H), 8.67 (d, *J* = 6.1, 2H), 8.54-8.46 (m, *J* = 2.7, 2H), 8.12 (d, *J* = 5.7, 4H), 7.71 - 7.08 (m, 15H), 6.87 (d, *J* = 8.2, 4H), 6.24 (dd, *J* = 8.5, 4.3, 1H), 6.13 (dd, *J* = 13.9, 6.5, 1H), 5.49 - 5.01 (m, 4H), 4.71 - 4.15 (m, 4H), 3.76 (s, 9H), 3.64 - 3.33 (m, 3H), 3.12 (s, 1H), 1.32-0.8 (m, 3H), 1.13 (s, 9H), 0.74 (d, *J* = 3.2, 18H), -0.00 (d, *J* = 2.1, 3H), -0.20 (d, *J* = 13.0, 3H).

³¹P NMR (200 MHz, Acetone) δ 1.15 (s, 1P), 0.89 (s, 1P).

¹³C NMR (300MHz, Acetone) δ 164.9, 158.77, 152, 150.5, 145.0, 143.3, 143.2, 142.6, 142.4, 135.65, 134, 133.9, 132.35, 130, 128.49, 128.3, 128, 127.7, 126.7, 125.46, 125.2, 125.1, 113, 89.42, 89.3, 88.2, 88.1, 86.4, 82.78, 82.71, 77.42, 77.38, 76.93, 76.88, 75.5, 75.2, 72.8, 72.78, 71.8, 70.9, 70.8, 67.25, 67.19, 66.97, 66.91, 62.88, 54.64, 48.45, 26.3, 25, 17.3, 17.2, 12, - 5.5, -5.9.

ESI-HRMS for C₇₁H₈₇N₁₀O₁₄Psi₂Na⁺: calcd 1413.55611, found 1413.55628.

### [5'-DMTr-2'TBDMS-N-Bz-rA]-[3'-p(OMe)-5']-[rA-2'TIPS]-3'-methylphosphoramidite (47)

N,N-Diisopropylammonium tetrazolide (0.67 g, 3.59 mmol) was added to the bis(N,N-diisopropylamino)methylphosphine (1.05 mL, 3.59 mmol) in 1ml of CH₂Cl₂. The nucleoside **(46)** (0.5 g, 0.359 mmol) was added to the above reaction mixture at room temperature and stirring was continued for 12h. The reaction mixture was diluted with DCM and washed 2 times with water and saturated NaCl. The organic layer was dried over anhydrous MgSO₄, filtered and concentrated. The compound was dissolved in minimum DCM and precipitated in petroleum ether at -78 C. The precipitated compound was subjected to column chromatography on silica gel using eluent 1:1 DCM & EtOAc to get the ApA dimer amidite (0.45 g, 80%).

³¹P NMR (200 MHz, Acetone) δ 153.41 (s, 1P), 153.34 (s, 1P), 150.96 (s, 1P), 150.65 (s, 1P), 1.20 (s, 1P), 1.05 (s, 1P), 0.92 (s, 1P), 0.86 (s, 1P).

ESI-HRMS for C₇₈H₁₀₃N₁₁O₁₅Psi₂Na⁺: calcd 1574.65411, found 1574.65406.

### Synthesis of orthogonally cleavable tags.

### Ethyl 4-oxooct-7-enoate (54)

4-Pentenal **(51)** (3.8 g, 45 mmol) was mixed with 3-benzyl-5-(2-hydroxyethyl)-4-methylthiazolium chloride **(53,** 2.45 g, 9.1 mmol) and ethyl acrylate **(52,** 9.0 g, 90 mmol) and dissolved in 22 mL of anhydrous ethanol. The reaction mixture was heated to reflux and then triethylamine (7.6 mL) was added to begin the reaction. The reaction mixture was refluxed for 18 hours and was then cooled to room temperature. The ethanol was removed under reduced pressure and the reaction mixture was then suspended in dichloromethane and extracted with brine. The organic layer was dried over magnesium sulphate and the solvent was removed under reduced pressure. The products were purified by flash column chromatography using a hexanes/ethyl acetate gradient elution system to give **(54)** (3.96 g, mixed with **(55),** app. 25% yield) as a pale yellow oil. ¹H NMR (300MHz, Acetonitrile-*d*₃) δ = 5.99 - 5.73 (m, 1H, with acyloin impurity), 5.15 - 5.05 (m, 2 H), 5.04 - 4.93 (acyloin impurity), 4.15 - 4.06 (q, *J* = 7.0 Hz, 2 H), 2.76 - 2.67 (m, 2 H, with acyloin impurity), 2.67 - 2.60 (acyloin impurity), 2.55 - 2.48 (m, 2H, with acyloin impurity), 2.35 - 2.26 (m, 2 H), 2.20 - 2.17 (acyloin impurity), 2.16 - 2.12 (m, 2 H), 1.99 - 1.94 (acyloin impurity), 1.21 (t, *J* = 7.0 Hz, 3 H), C₁₀H₁₆O₃Na¹⁺ low resolution ESI-MS calculated: 207.10, found: 207.31.

### Ethyl 4,4-diethoxyoct-7-enoate (56)

Compound **(54)** (2.0 g, as mixture with **(55),** approx. 5.7 mmol) was dissolved in a 1:1 v/v mixture of ethyl orthoformate and ethanol (6 mL) and mixed with a catalytic amount of *p*TSA (0.1 g). The reaction mixture was then refluxed for 4 hours and it was then cooled to 0 °C. Saturated aqueous sodium bicarbonate (20 mL) was then added along with diethyl ether (20 mL). The aqueous phase was extracted several times with diethyl ether and then the combined organic layers were rinsed with aqueous brine and dried over magnesium sulphate. The solvent was removed under reduced pressure and the product was purified by flash column chromatography using a hexanes/ethyl acetate gradient system to give **(56)** (1.36 g, 92% yield) as a colourless oil. ¹H NMR (500MHz, DMSO-*d₆*) δ = 5.85 - 5.73 (m, 1 H), 5.05 - 4.88 (m, 2 H), 4.03 (q, *J* = 7.2 Hz, 2H), 3.32 (q, *J* = 7.0 Hz, 4H), 2.20 (t, *J* = 7.8 Hz, 2 H), 1.97 - 1.88 (m, 2 H), 1.79 (t, *J* = 7.8 Hz, 2 H), 1.53 (t, *J* = 8.4 Hz, 2 H), 1.16 (t, *J* = 7.2 Hz, 3 H), 1.06 (t, *J* = 7.0 Hz, 6 H), ¹³C NMR (75MHz, Acetonitrile-*d₃*) δ = 173.0, 138.5, 113.8, 102.0, 60.1, 55.0, 32.3, 28.7, 28.3, 27.8, 14.6, 13.6, C₁₄H₂₆O₄Na¹⁺ low resolution ESI-MS calculated: 281.17, found: 281.13.

### Ethyl 4,4-diethoxy-8-hydroxyoctanoate (57)

A 1 M solution of borane in THF (2.4 mL, 2.4 mmol) was cooled to 0 °C and to it was added, dropwise, 0.47 mL of cyclohexene (4.6 mmol). The reaction was stirred for 1 hour at 0 °C and to the resultant white slurry was added compound **(56)** (0.51 g, 2.0 mmol). The reaction was allowed to warm to room temperature and was stirred for 2 hours. After this time, sodium perborate tetrahydrate (1.07 g, 7.0 mmol) and 2.4 mL of water were added. The reaction was stirred for a further 2 hours and then the reaction mixture was extracted with ethyl acetate several times. The combined organic layers were dried of magnesium sulphate and the solvent was removed under reduced pressure. The products were purified using a dichloromethane/methanol gradient elution system to give **(57)** (0.45 g, 81% yield) as a colourless oil. Starting material **(56)** was also recovered. ¹H NMR (300MHz, DMSO-*d₆*) δ = 4.33 (t, *J* = 5.2 Hz, 1 H), 4.02 (q, *J* = 7.1 Hz, 2 H), 3.36 (m, 2 H), 3.31 (q, *J* = 7.3 Hz, 4 H), 2.18 (t, *J* = 7.7 Hz, 2 H), 1.76 (t, *J* = 7.7 Hz, 2 H), 1.46 - 1.41 (m, 2 H), 1.40 - 1.31 (m, 2 H), 1.25 - 1.19 (m, 2 H), 1.16 (t, *J* = 7.1 Hz, 3 H), 1.05 (t, *J* = 7.3 Hz, 6 H), ¹³C NMR (75MHz, DMSO-*d₆*) δ = 173.1, 102.4, 61.0, 60.3, 55.0, 33.2, 33.0, 29.0, 28.5, 20.2, 15.7, 14.5, C₁₄H₂₈O₅Na¹⁺ low resolution ESI-MS calculated: 299.18, found: 299.19.

### Ethyl 8-bromo-4,4-diethoxyoctanoate (58)

Compound **(57)** (0.50 g, 1.8 mmol) was mixed with triphenylphosphine (0.80 g, 3.0 mmol) and imidazole (0.20 g, 2.9 mmol) and dissolved in 18 mL of dichloromethane. The solution was cooled to 0 °C and a solution of carbon tetrabromide (0.85 g, 2.6 mmol) in dichloromethane (1.5 mL) was added slowly. The reaction mixture was allowed to warm to room temperature and was stirred for 1 hour. The reaction was then quenched by the addition of saturated aqueous sodium sulphite and extracted with dichloromethane. The combined organic layers were dried over sodium sulphate and the solvent was removed under reduced pressure. The product was purified by flash column chromatography with a hexanes/ethyl acetate gradient elution system to yield **(58)** (0.49 g, 81% yield) as a colourless oil. ¹H NMR (400MHz, DMSO-*d₆*) δ = 4.04 (q, *J* = 7.1 Hz, 2 H), 3.54 (t, *J* = 6.5 Hz, 2 H), 3.32 (q, *J* = 6.9 Hz, 4 H), 2.22 (t, *J* = 7.8 Hz, 2 H), 1.83 - 1.74 (m, 4 H), 1.47 (t, *J* = 7.2 Hz, 2 H), 1.35 - 1.25 (m, 2 H), 1.18 (t, *J* = 7.1 Hz, 3 H), 1.07 (t, *J* = 6.9 Hz), ¹³C NMR (126MHz ,Acetonitrile-*d₃*) δ = 173.0, 102.1, 60.1, 55.0, 34.2, 32.5, 32.1, 28.8, 28.4, 22.0, 14.7, 13.5, C₁₄H₂₇BrO₄Na¹⁺ low resolution ESI-MS calculated: 361.10, found: 361.11.

### Ethyl 4,4-diethoxy-8-(2,3-dimethyl-1H-imidazol-1-yl) octanoate bromide (59)

Compound **(58)** (0.31 g, 0.90 mmol) was dissolved in 5 mL of acetonitrile and mixed with 1,2-dimethylimidazole (0.13 g, 1.3 mmol). The reaction was warmed to 50 °C and stirred overnight. The reaction mixture was then cooled to room temperature and the solvent was then removed under reduced pressure. The resultant oil was rinsed several times with diethyl ether and then the compound was again subjected to reduced pressure. This gave **(59)** (0.38 g, 95% yield) as a colourless oil. ¹H NMR (500MHz, DMSO-*d₆*) δ = 7.63 (d, *J* = 2.2 Hz, 1 H), 7.60 (d, *J* = 2.2 Hz, 1 H), 4.09 (t, *J* = 7.2 Hz, 2 H), 4. 04 (q, *J* = 7.2 Hz, 2 H), 3.73 (s, 3 H), 3.31 (q, *J* = 6.9 Hz, 4 H), 2.56 (s, 3 H), 2.20 (t, *J* = 8.1 Hz, 2 H), 1.76 (t, *J* = 8.1 Hz, 2 H), 1.71 - 1.63 (m, 2 H), 1.49 (t, *J* = 7.6 Hz, 2 H), 1.24 - 1.19 (m, 2 H), 1.16 (t, *J* = 7.2 Hz, 3 H), 1.05 (t, *J* = 6.9 Hz, 6 H), ¹³C NMR (126MHz ,DMSO-*d₆*) δ = 173.6, 122.8, 121.3, 102.5, 60.7, 55.6, 48.6, 35.3, 33.2, 29.9, 29.3, 28.8, 20.8, 15.2, 14.1, 9.7, C₁₉H₃₅N₂O₄¹⁺ low resolution ESI-MS calculated: 355.26, found: 355.28.

### 8-(2,3-dimethyl-1H-imidazol-1-yl)-4-oxooctanoic acid bromide (60)

Compound **(59)** (0.44 g, 1.0 mmol) was mixed with 1 M aqueous sodium hydroxide (5 mL) and stirred overnight. The solution was then acidified to pH 1 by addition of concentrated aqueous HCl. The aqueous solution was then rinsed with diethyl ether, followed by the removal of water under reduced pressure. The resulting solid was dissolved in a minimum of cold acetone and dichloromethane any undissolved material was removed by filtration. The solvent was then removed under reduced pressure to give **(60)** (0.34 g, > 100% yield) as an off-white solid, likely mixed with a small amount of sodium chloride. ¹H NMR (400MHz, DMSO-*d₆*) δ = 7.63 (d, *J* = 2.1 Hz, 1 H), 7.61 (d, *J* = 2.1 Hz, 1 H), 4.09 (t, *J* = 7.3 Hz, 2 H), 3.73 (s, 3 H), 2.61 (t, *J* = 6.3 Hz, 2 H), 2.56 (s, 3 H), 2.49 (t, *J* = 7.3 Hz, 2 H), 2.37 (t, *J* = 6.3 Hz, 2 H), 1.65 (m, 2 H,), 1.43 (m, 2 H), ¹³C NMR (126MHz ,Acetonitrile-*d₃*) d = 208.9, 173.4, 122.3, 120.9, 48.0, 41.0, 36.7, 34.8, 28.6, 27.7, 19.9, 9.2, C₁₃H₂₁N₂O₃¹⁺ high resolution ESI-MS required: 253.15467, found: 253.15459.

### General procedure for the derivatization of (60) with nucleosides.

Compound **(60)** (0.4 mmol) was dissolved in 15% v/v DMF/acetonitrile and mixed with DCC (0.6 mmol), DMAP (0.2 mmol), and the desired 5'-DMTr-dT or 5'-DMTr 2'-TIPS rU **(12)** (0.5 mmol). The reaction mixture was stirred overnight and was then precipitated from MTBE. The resultant solid was removed by filtration and recovered from the filter by dissolving it in acetonitrile. The solvent was removed under reduced pressure and the resulting solid was taken up in dichloromethane and washed with dilute aqueous sodium tetrafluoroborate to achieve ion metathesis. The organic phase was dried over magnesium sulphate and the solvent was removed under reduced pressure. The resultant solid was then dissolved in acetonitrile and the solution was precipitated from MTBE, followed by filtration and recovery in acetonitrile. The solvent was then removed under reduced pressure to yield the desired tagged nucleoside in 80-90% yield.

**(61)** ¹H NMR (500MHz, DMSO-*d₆*) δ = 11.37 (s, 1 H), 7.59 (d, *J* = 2.1 Hz, 1 H), 7.57 (d, *J* = 2.1 Hz, 1 H), 7.49 (s, 1 H), 7.39 - 7.36 (m, 2 H), 7.34 - 7.30 (m, 2 H), 7.29 (t, *J* = 7.1 Hz, 2 H), 7.25 - 7.19 (m, 5 H), 6.90 - 6.85 (m, 4 H), 6.17 (dd, *J* = 5.9 Hz, 1 H), 5.25 (d, *J* = 6.3 Hz, 1 H), 4.08 (t, *J* = 7.1 Hz, 2 H), 4.01 (dd, *J* = 3.5 Hz, 1 H), 3.72 (s, 6 H), 3.71 (s, 3 H), 3.33 - 3.27 (m, 1H, and H₂O), 3.22 - 3.17 (dd, *J* = 7.2, 3.3 Hz, 1 H), 2.69 (t, *J* = 6.1 Hz, 2 H), 2.60 (s, 3 H), 2.55 - 2.44 (m, 8H, and DMSO), 2.53 (dd, *J* = 8.1, 5.4 Hz , 1 H), 1.72 - 1.61 (m, 2 H), 1.48 - 1.42 (m, 2 H), C₄₄H₅₁N₄O₉¹⁺ high resolution ESI-MS required: 779.36506, found: 779.36526.

**(62)** ¹H NMR (500MHz, DMSO-*d₆*) δ = 11.47 (s, 1 H, H3), 8.22 - 8.16 (m, 1 H, DMT), 7.72 - 7.70 (br. s, 1 H, H6), 7.69 - 7.68 (m, 1 H, tag CH=CH), 7.63 - 7.60 (m, 1 H, tag CH=CH), 7.60 - 7.58 (m, 2 H, DMTr), 7.58 - 7.56 (m, 2 H, DMTr), 7.36 - 7.27 (m, 2 H, DMT), 7.26 - 7.18 (m, 2 H, DMT), 6.91 - 6.84 (m, 4 H, DMTr), 5.87 (d, *J* = 6.8 Hz, 1 H, H1'), 5.49 (dd, *J* = 2.0, 5.9 Hz, 1 H, H5), 5.16 (t, *J* = 2.3 Hz, 1 H, H3'), 4.64 (t, *J* = 6.2 Hz, 1 H, H2'), 4.06 (t, *J* = 7.4 Hz, 2 H, NCH₂), 3.73 (s, 6 H, DMT OCH₃), 3.70 (s, 3 H, tag NCH₃), 3.29-3.15 (m, water and H5'&5"), 2.70 (t, *J* = 6.1 Hz, 2 H, tag C**H₂**CH₂COO), 2.63 - 2.58 (m, 3 H, tag CH₃CN₂), 2.55 (m, DMSO and tag CH₂C**H₂**COO and tag NCH₂CH₂CH₂C**H₂**), 2.54 - 2.45 (m, 5 H), 1.73 - 1.61 (m, tag NCH₂C**H₂**CH₂CH₂ and 10% impurity), 1.49 - 1.35 (m, tag, NCH₂CH₂C**H₂**CH₂ and 10% impurity), 1.28 - 1.13 (m, impurity), 0.99 - 0.90 (m, 9 H), C₅₂H₆₉N₄O₁₀Si¹⁺ high resolution ESI-MS required: 937.47775, found: 937.47680.

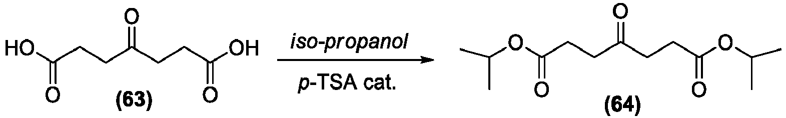

### Diisopropyl 4-oxoheptanedioate (64)

4-ketopemilic acid **(63)** (10 g, 57.4 mmol; purchased from Sigma-Aldrich) was suspended in 50 mL of isopropanol and 50 mL of benzene. Catalytic amount of *p*-toluene solfonic acid was added to the mixture and brought to reflux using a Dean Stark trap to remove the water produced. Once the volume had decreased to approximately 50 mL in the flask, another 50 mL of 50:50 Benzene:*iso*-propanol was added and further reduced to approximately 30 ml. The mixture was then taken up in ethyl acetate and extracted with NaHCO₃ (x3) and once with brine. The organic layer was dried with MgSO₄ and condensed to dryness yielding pure **(64):** 14.2 g (95%).

¹H NMR (300 MHz, CHLOROFORM-d) δ ppm 1.06 (d, *J*=6.45 Hz, 12 H) 2.39 (t, *J*=7.00 Hz, 1 H) 2.60 (t, *J*=6.70 Hz, 4 H) 4.72 - 4.89 (m, 2 H) ¹³C NMR (75 MHz, CHLOROFORM-d) δ ppm 21.57 (s, 1 C) 28.13 (s, 3 C) 28.13 (s, 3 C) 36.92 (s, 3 C) 67.66 (s, 1 C) 171.90 (s, 2 C) 206.82 (s, 1 C) C₁₃H₂₂O₅Na¹⁺ low resolution ESI-MS calculated: 258.14, found: 281.21.

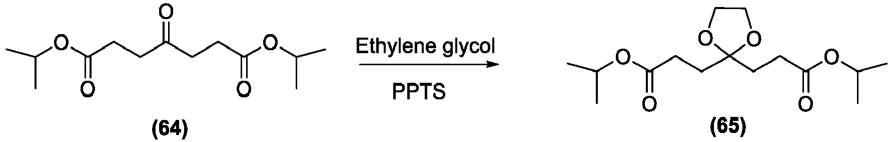

### Diisopropyl 3,3'-(1,3-dioxolane-2,2-diyl)dipropanoate (65)

Compound **(64)** (0.55 g, 2.1 mmol) was solvated with 5eq of ethylene glycol (0.58 mL, 10.5 mmol), 90 mL of dry toluene and catalytic amount of pyridinium *para*-toluene sulfonate. This mixture was refluxed at 140°C replacing the toluene 3 times and finally allowing the reaction to reflux overnight. The mixture was then distilled to approximately 30 mL, removed from heat and diluted with DCM and extracted with sat. NaHCO₃ (x2) then water (x3) to remove any excess ethylene glycol. The product was purified by column chromatography (DCM:MeOH, 100:0 → 95:5). Isolated yield: 0.41 g (65%).

¹H NMR (500 MHz, CHLOROFORM-d) δ ppm 1.05 (d, *J*=6.36 Hz, 12 H) 1.78 (t, *J*=7.58 Hz, 15 H) 2.16 (t, *J*=7.58 Hz, 15 H) 3.76 (s, 15 H) 4.82 (dt, *J*=12.53, 6.33 Hz, 8 H) ¹³C NMR (126 MHz, CHLOROFORM-d) δ ppm 21.59 (s, 1 C) 29.02 (s, 1 C) 32.07 (s, 1 C) 64.94 (s, 1 C) 67.23 (s, 1 C) 67.26 (s, 1 C) 109.84 (s, 1 C) 172.56 (s, 1 C) C₁₅H₂₆O₆Na¹⁺ low resolution ESI-MS calculated: 302.17, found: 325.0.

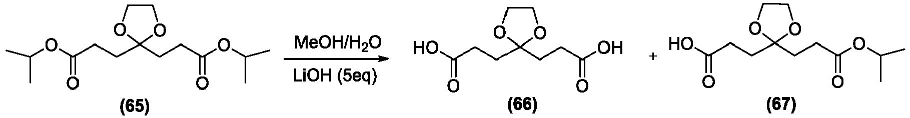

### 3-(2-(3-hydroxy-3-oxopropyl)-1,3-dioxolan-2-yl)propanoic acid (66) and 3-(2-(3-isopropoxy-3-oxopropyl)-1,3-dioxolan-2-yl)propanoic acid (67).

Compound **(65)** (1.3 g, 4.3vmmol) was solvated in 2.5 mL of MeOH to which was added 5 eq of LiOH (0.51 g, 21.5 mmol) in 2.5 mL of water. This mixture was allowed to stir for 6h until all starting material was consumed. The solution was brought to neutrality by the addition of 1 M HCl in MeOH.

This mixture was purified by column chromatography (DCM:MeOH, 100:0 → 90:10). Isolated yield of **(66):** 0.35 g (40%). Yield of **(67):** 0.68 g (60%).

¹H NMR (300 MHz, METHANOL-*d*₄) δ ppm 1.94 (t, *J*=8.20 Hz, 4 H) 2.33 (t, *J*=7.30 Hz, 15 H) 3.94 (s, 26 H) ¹³C NMR (75 MHz, METHANOL-*d*₄) δ ppm 28.19 (s, 1 C) 31.82 (s, 1 C) 64.77 (s, 1 C) 109.81 (s, 1 C) 175.90 (s, 1 C) C₉H₁₄O₆Li¹⁻ low resolution ESI-MS calculated: 218.07, found: 224.12. **(67)**

¹H NMR (500 MHz, METHANOL-*d*₄) δ ppm 1.22 (d, *J*=6.36 Hz, 6 H) 1.87 - 2.03 (m, 4 H) 2.23 - 2.34 (m, 4 H) 3.55 (m, *J*=5.14 Hz, 3 H) 3.67 (m, *J*=5.14 Hz, 3 H) 4.89 - 5.00 (m, 2 H) ¹³C NMR (126 MHz, METHANOL-*d*₄) δ ppm 7.75 (s, 1 C) 20.72 (s, 1 C) 28.80 (s, 1 C) 31.81 (s, 1 C) 60.87 (s, 1 C) 62.94 (s, 1 C) 67.60 (s, 1 C) 72.13 (s, 1 C) 109.92 (s, 1 C) 173.40 (s, 1 C) 176.73 (s, 1 C) C₁₂H₂₀O₆¹⁻low resolution ESI-MS calculated: 260.12, found: 259.03.

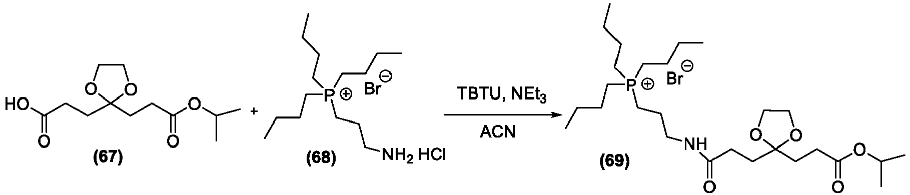

### Tributyl(3-(3-(2-(3-isopropoxy-3-oxopropyl)-1,3-dioxolan-2-yl)propanamido)propyl)phosphonium bromide (69)

Compound **(67)** (0.3 g, 1.1 mmol) was solvated in 1.5 mL of ACN followed by TBTU (0.39 g, 1.2 mmol), 2.5 eq of triethylamine (0.38 ml) and phosphonium ionic tag **(68)** (0.45 g, 1,2 mmol). This mixture was allowed to stir for 4h until the starting material **(67)** was completely consumed. The reaction mixture was diluted with ethyl acetate and extracted with 5% NaHCO₃ x2 and once with brine. The organic layer was dried and concentrated and purified by column chromatography. DCM:MeOH 100:0 → 95:5. Isolated yield: 0.54 g (84%).

¹H NMR (300 MHz, CHLOROFORM*-d*) δ ppm 0.91 (t, *J*=6.74 Hz, 9 H) 1.06 (d, *J*=6.45 Hz, 6 H) 1.25 (s, 6 H) 1.38 - 1.59 (m, 11 H) 1.80 - 1.95 (m, 7 H) 2.15 - 2.30 (m, 9 H) 2.18 (t, *J*=7.03 Hz, 8 H) 3.28 - 3.44 (m, 2 H) 3.84 (br. s., 5 H) 3.94 (s, 6 H) 4.72 - 4.89 (m, 1 H) C₂₇H₅₃NO₆P¹⁺ low resolution ESI-MS calculated: 502.36, found: 502.36.

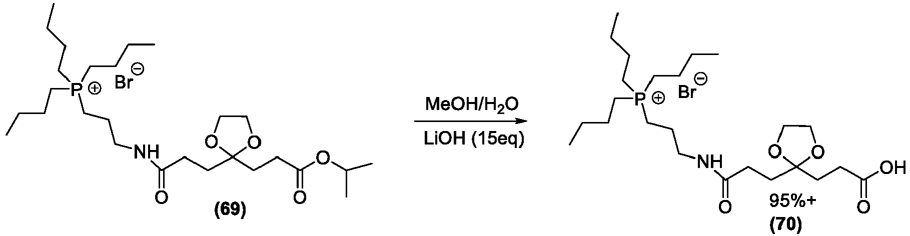

### Tributyl(3-(3-(2-(3-hydroxy-3-oxopropyl)-1,3-dioxolan-2-yl)propanamido) propyl)phosphonium bromide (70)

Compound **(69)** (0.25 g, 0.4 mmol) was solvated in 2.5 mL of MeOH to was added 10 eq of LiOH (0.1 g, 4 mmol) in mL of water. This mixture was allowed to stir for 3h until all starting material was consumed. The solution was brought to neutrality by the addition of 1M HCl in MeOH. This mixture was purified by column chromatography (DCM:MeOH, 100:0 → 90:10). Isolated yield of **(70):** 0.20 g (95%).

¹H NMR (300 MHz, CHLOROFORM-d) δ ppm 0.91 (t, *J*=6.74 Hz, 9 H) 1.25 (s, 6 H) 1.38 - 1.59 (m, 11 H) 1.96 - 2.05 (m, 7 H) 2.15 - 2.30 (m, 9 H) 2.40 (t, *J*=7.03 Hz, 8 H) 3.28 - 3.44 (m, 2 H) 3.84 (br. s., 5 H) 3.94 (s, 6 H) C₂₄H₄₇NO₅P¹⁺ low resolution ESI-MS calculated: 460.31, found: 460.30.

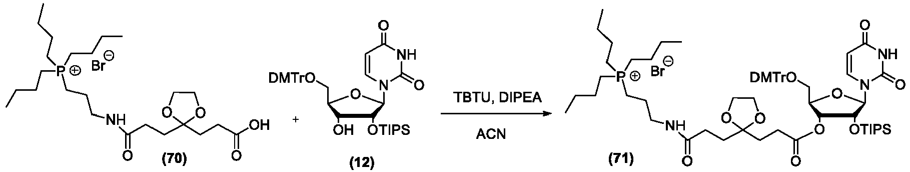

### 5'-DMTr-2'-TIPS-3'-[tributyl(3-(3-(2-(2-carboxyethyl)-1,3-dioxolan-2-yl)propanamido)propyl)phosphonium bromide] (71)

To a solution of compound **(70)** (0.2 g, 0.37 mmol) in ACN (1 mL) was added TBTU (0.19 g, 0.6 mmol), triethylamine (0.5 mL) and compound **(12)** (0.42 g, 0.6 mmol). The resulting mixture was allowed to stir for 12 h until the starting material **(70)** was completely consumed. The reaction mixture was diluted with ethyl acetate and extracted with 5% NaHCO₃ x2 and once with brine. The organic layer was dried and concentrated, taken up in minimal amounts of DCM at precipitated in 100 ml of MTBE, filtered over Celite^{©}. Isolated yield of **(71):** 0.20g (45%).

¹H NMR (300 MHz, CHLOROFORM-d) δ ppm 0.91 (t, *J*=6.74 Hz, 9 H)1.02 - 1.59 (m, 38 H) 1.96 - 2.05 (m, 7 H) 2.15 - 2.30 (m, 9 H) 2.40 (t, *J*=7.03 Hz, 8 H) 3.37 - 3.40 (m, 4 H) 3.78 - 3.94 (br. m, 17 H) 4.15 (d, *J*=2.77 Hz, 1 H) 4.63 - 4.67 (m, 1 H) 5.31 (dd, *J*=5.14, 2.96 Hz, 1 H) 5.40 - 5.46 (m, 1 H) 5.42 (s, 1 H) 5.99 (d, *J*=6.32 Hz, 1 H) 6.87 - 6.93 (m, 4 H) 7.27 - 7.37 (m, 7 H) 7.41 - 7.45 (m, 2 H) 7.75 (d, *J*=8.30 Hz, 1 H) C₆₃H₉₅N₃O₁₂PSi¹⁺ low resolution ESI-MS calculated: 1144.64, found: 1144.7.

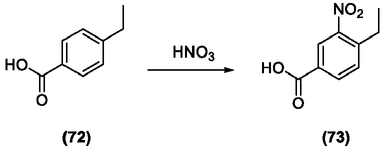

### 3-Nitro-4-ethyl-benzoic acid (73)

Fuming nitric acid (90%) (150 ml) was cooled with stirring to - 10°C and 4-ethyl benzoic acid **(72)** (30 g, 0.2 moles; Sigma-Aldrich) was added slowly over 30min directly into the sitting solution (1.33 mmol/ml of **(72)** to fuming nitric acid). The mixture was then allowed to stir for 30min after addition was complete. The mixture was then poured over approximately 600 g of crushed ice to quench the reaction. The product formed a white ppt which can be filtered over a sintered glass funnel.

The excess ice melted by washing the product with water. The sample was then re-crystallized from ethyl acetate/hexanes. Two rounds of crystallization were preformed. Yield: 37.2 g (95%).

¹H NMR (500 MHz, CHLOROFORM-d) δ ppm 1.33 (t, *J*=7.58 Hz, 3 H) 2.99 (q, *J*=7.58 Hz, 2 H) 7.52 (d, *J*=8.07 Hz, 1 H) 8.23 (dd, *J*=8.07, 1.71 Hz, 1 H) 8.58 (d, *J*=1.71 Hz, 1 H) ¹³C NMR (126 MHz, CHLOROFORM*-d*) δ ppm 14.62 (s, 1 C) 26.38 (s, 1 C) 126.37 (s, 1 C) 128.29 (s, 1 C) 131.67 (s, 1 C) 133.87 (s, 1 C) 144.85 (s, 1 C) 149.36 (s, 1 C) 170.40 (s, 1 C) C₉H₉NO₄Na¹⁺ low resolution ESI-MS calculated: 195.05, found: 218.2.

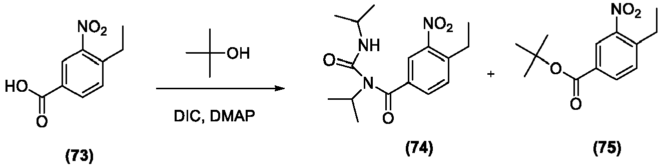

### tert-Butyl 3-nitro-4-ethyl-benzoate (75)

Compound **(73)** (19.65 g 0.10 moles) was solvated in 500ml of THF (0.2 M) followed by 1.15 eq (10.14 g, 0.15 moles) of diisopropylcarbodimide. This mixture was allowed to stir for 5min followed by 1.5 eq of *tert*-butanol (17.9 mL) and catalytic amounts of 4-(dimethylamino)-pyridine. The mixture was allowed to stir for 60 h before the reaction was complete. The reaction was diluted with diethyl ether and filtered to remove the diisopropylurea (DIU) and condensed to dryness. The mixture was solvated in ethyl acetate and extracted with 5% NaHCO₃. The product was separated from **(74)** by column chromatography (solvent system: hexanes:DCM 100:0 →0:100). Yield of **(75):** 15.1g (60%); yield of **(74):** 30%.

¹H NMR (300 MHz, CHLOROFORM-d) δ ppm 0.94 (d, *J*=6.45 Hz, 6 H) 1.26 (t, *J*=7.47 Hz, 3 H) 1.39 (d, *J*=6.74 Hz, 6 H) 2.91 (q, *J*=7.33 Hz, 2 H) 3.80 (dq, *J*=13.88, 6.70 Hz, 1 H) 4.44 (quin, *J*=6.74 Hz, 1 H) 6.49 (d, *J*=7.91 Hz, 1 H) 7.40 (d, *J*=8.20 Hz, 1 H) 7.67 (dd, *J*=7.91, 1.76 Hz, 1 H) 8.02 (d, *J*=1.76 Hz, 1 H) ¹³C NMR (75 MHz, CHLOROFORM-*d*) δ ppm 14.80 (s, 1 C) 20.75 (s, 1 C) 22.04 (s, 1 C) 26.12 (s, 1 C) 42.96 (s, 1 C) 49.54 (s, 1 C) 76.63 (s, 1 C) 77.05 (s, 1 C) 77.48 (s, 1 C) 123.13 (s, 1 C) 130.76 (s, 1 C) 131.52 (s, 1 C) 135.78 (s, 1 C) 141.49 (s, 1 C) 148.95 (s, 1 C) 153.60 (s, 1 C) 168.67 (s, 1 C) C₁₆H₂₃N3O₄Na¹⁺ low resolution ESI-MS calculated: 321.16, found: 344.23.

**(75)** ¹H NMR (300 MHz, CHLOROFORM-*d*) δ ppm 1.26 (t, *J*=7.47 Hz, 3 H) 1.57 (s, 11 H) 2.91 (q, *J*=7.33 Hz, 2 H) 7.40 (d, *J*=7.91 Hz, 1 H) 8.08 (dd, *J*=7.91, 1.47 Hz, 1 H) 8.38 (d, *J*=1.47 Hz, 1 H) ¹³C NMR (75 MHz, CHLOROFORM-*d*) δ ppm 14.70 (s, 1 C) 26.18 (s, 1 C) 28.05 (s, 1 C) 76.64 (s, 1 C) 77.06 (s, 1 C) 77.49 (s, 1 C) 82.13 (s, 1 C) 125.42 (s, 1 C) 131.12 (s, 1 C) 131.18 (s, 1 C) 133.23 (s, 1 C) 143.00 (s, 1 C) 149.12 (s, 1 C) 163.56 (s, 1 C) C₁₃H₁₇NO₄Na¹⁺ low resolution ESI-MS calculated: 251.11, found: 274.32.

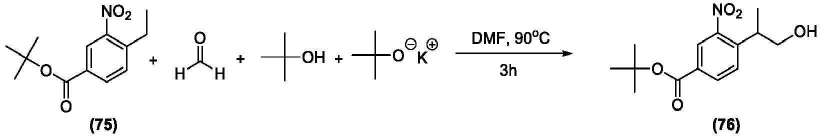

### tert-Butyl 4-(1-hydroxypropan-2-yl)-3-nitrobenzoate (76)

Compound **(75)** (10.5 g, 41.7 mmol) was solvated in 19 mL of DMF (2.2M) to which 1.5 eq (1.88 g) of paraformaldehyde was added followed by a solution of potassium *tert*-butoxide (0.12 eq, 0.56 g) in tert-butanol (5.7 mL). This mixture was allowed to stir at room temperature for 10 min before being brought up 90°C for 3h. The mixture was then acidified to neutrality by the addition of a 1M HCl monitored a by a pH meter. This mixture was then diluted with sat. NaCl and ethyl acetate (x2). Compound **(76)** was purified by column chromatography in DCM:ethyl acetate 100:0 →90:10. Yield: 9.4 g (85%).

¹H NMR (400 MHz, ACETONITRILE-*d*₃) δ ppm 1.41 (d, *J*=7.03 Hz, 3 H) 3.80 (sxt, *J*=6.88 Hz, 1 H) 4.49 - 4.67 (m, 2 H) 7.40 (d, *J*=7.91 Hz, 1 H) 8.08 (dd, *J*=7.91, 1.47 Hz, 1 H) 8.38 (d, *J*=1.47 Hz, 1 H) ¹³C NMR (75 MHz, CHLOROFORM-d) δ ppm 17.35 (s, 1 C) 27.96 (s, 1 C) 36.57 (s, 1 C) 67.01 (s, 1 C) 82.27 (s, 1 C) 124.77 (s, 1 C) 128.50 (s, 1 C) 131.04 (s, 1 C) 131.06 (s, 1 C) 132.81 (s, 1 C) 142.62 (s, 1 C) 150.41 (s, 1 C) 163.58 (s, 1 C) C₁₄H₁₉NO₅Na¹⁺ low resolution ESI-MS calculated: 281.12, found: 304.02.

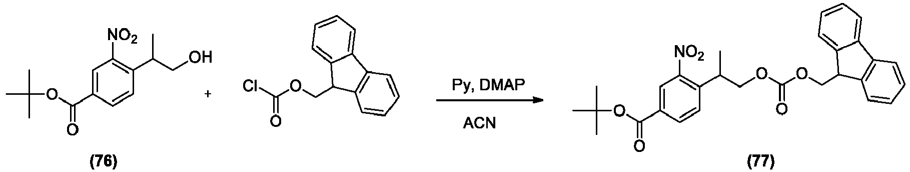

### tert-Butyl 4-(1-(F-moc)propan-2-yl)-3-nitrobenzoate (77)

Compound **(76)** (5.38 g, 20.2 mmol) was co-evaporated with pyridine (x3) and solvated in 97 mL of ACN (0.2M) and 2eq of pyridine (3.13 mL, 38.7 mmol). Fmoc-Cl (5.0g, 19.33 mmol) was added directly to solution and was allowed to stir for 16h in the dark, covered with aluminum foil. The reaction went to completion by TLC. The solution extracted (x3) with 5% ammonium chloride and once with brine and purified by column chromatography Hex/EtAc 100:0 → 75:25. Isolated yield: 8.19 g (86%).

¹H NMR (500 MHz, ACETONITRILE-*d*₃) δ ppm 1.30 (d, *J*=7.09 Hz, 3 H) 1.60 (s, 9 H) 3.65 (sxt, *J*=6.94 Hz, 1 H) 4.19 (t, *J*=6.24 Hz, 1 H) 4.23 - 4.34 (m, 2 H) 4.39 - 4.53 (m, 2 H) 7.27 - 7.34 (m, 2 H) 7.41 (t, *J*=7.46 Hz, 2 H) 7.53 (d, *J*=7.34 Hz, 2 H) 7.62 (d, *J*=8.31 Hz, 1 H) 7.80 (d, *J*=7.58 Hz, 2 H) 8.13 (dd, *J*=8.19, 1.59 Hz, 1 H) 8.28 (d, *J*=1.71 Hz, 1 H) ¹³C NMR (126 MHz, ACETONITRILE-*d*₃) δ ppm -0.10 (s, 1 C) 0.06 (s, 1 C) 0.23 (s, 1 C) 0.40 (s, 1 C) 0.56 (s, 1 C) 0.73 (s, 1 C) 0.89 (s, 1 C) 16.83 (s, 1 C) 27.30 (s, 1 C) 27.32 (s, 1 C) 33.47 (s, 1 C) 46.64 (s, 1 C) 68.89 (s, 1 C) 70.90 (s, 1 C) 82.13 (s, 1 C) 117.34 (s, 1 C) 120.06 (s, 1 C) 124.56 (s, 1 C) 124.87 (s, 1 C) 124.90 (s, 1 C) 127.17 (s, 1 C) 127.18 (s, 1 C) 127.81 (s, 1 C) 127.83 (s, 1 C) 128.87 (s, 1 C) 131.72 (s, 1 C) 132.75 (s, 1 C) 141.03 (s, 1 C) 141.17 (s, 1 C) 143.50 (s, 1 C) 143.55 (s, 1 C) 150.42 (s, 1 C) 154.54 (s, 1 C) 163.26 (s, 1 C) C₂₉H₂₉NO₇Na¹⁺ low resolution ESI-MS calculated: 503.19, found: 526.41.

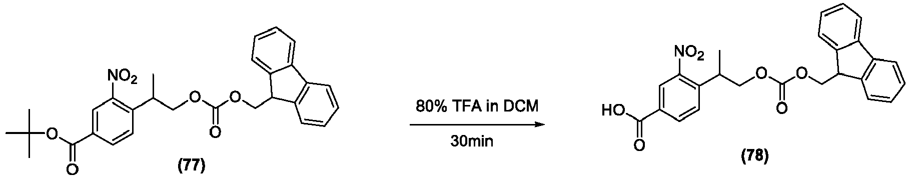

### 4-(1-(Fmoc)propan-2-yl)-3-nitrobenzoic acid (78)

Compound **(77)** (8.9 g, 17.7 mmol) was directly solvated in a solution of 80% TFA in DCM (50 mL) and allowed to stir for 30min, until all starting material had been consumed. The sample was then evaporated to dryness on the rotovap and purified by column chromatography, Hex/EtAc 100:0 → 60:40. Isolated yield: 6.09 g (77%).

¹H NMR (500 MHz, CHLOROFORM-*d*) δ ppm 1.43 (d, *J*=6.85 Hz, 3 H) 3.86 (sxt, *J*=6.80 Hz, 1 H) 4.32 - 4.46 (m, 4 H) 7.28 - 7.36 (m, 2 H) 7.41 (t, *J*=7.58 Hz, 2 H) 7.57 (dd, *J*=6.97, 4.52 Hz, 2 H) 7.65 (d, *J*=8.07 Hz, 1 H) 7.76 (d, *J*=7.34 Hz, 2 H) 8.29 (dd, *J*=8.07, 1.71 Hz, 1 H) 8.53 (d, *J*=1.71 Hz, 1 H) 11.69 (br. s., 1 H) ¹³C NMR (126 MHz, CHLOROFORM-*d*) δ ppm 17.58 (s, 1 C) 33.71 (s, 1 C) 46.62 (s, 1 C) 70.08 (s, 1 C) 71.14 (s, 1 C) 76.80 (s, 1 C) 77.05 (s, 1 C) 77.31 (s, 1 C) 120.06 (s, 1 C) 125.10 (s, 1 C) 125.11 (s, 1 C) 126.16 (s, 1 C) 127.16 (s, 1 C) 127.17 (s, 1 C) 127.91 (s, 1 C) 127.93 (s, 1 C) 128.87 (s, 1 C) 129.00 (s, 1 C) 133.71 (s, 1 C) 141.26 (s, 1 C) 141.27 (s, 1 C) 142.82 (s, 1 C) 143.14 (s, 1 C) 143.20 (s, 1 C) 150.39 (s, 1 C) 155.00 (s, 1 C) 169.79 (s, 1 C) C₂₅H₂₀NO₇¹⁻ low resolution ESI-MS calculated: 447.13, found: 446.0.

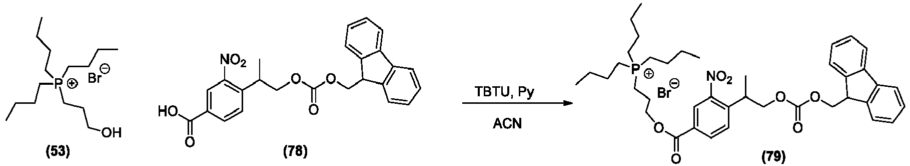

### Phosphonium tag 4-(1-(Fmoc)propan-2-yl)-3-nitrobenzoate (79)

Compound **(78)** (3.726 g, 8.33 mmol) was solvated in half the solvent (ACN:Py, 28 mL:1.25 mL)to which was added a solution of the phosphonium tag **(53)** (3.86 g, 9.16 mmol) in the other half of the solvent, followed directly by TBTU (4.0 g, 12.5 mmol). The solution was allowed to stir overnight and by morning the reaction was complete (12 h), and was concentrated to half solvent volume then extracted with ethyl acetate and 5% NaHCO₃ (x3) and once with brine. The organic layer was dried with MgSO₄ and condensed to dryness. The compound was then precipitated in 500 mL of MTBE to remove the excess pyridine and TBTU byproduct. The precipitated white goo was filtered and collected over Celite^{©} then purified by column chromatography, DCM:MeOH 100:0 → 92:8. Isolated yield of **(79):** 5.52 g (86%).

¹H NMR (300 MHz, CHLOROFORM-d) δ ppm 0.81 - 0.99 (m, 8 H) 1.35 (d, *J*=7.03 Hz, 3 H) 1.38 - 1.61 (m, 11 H) 1.99 - 2.38 (m, 8 H) 2.66 (br. s., 2 H) 3.54 - 3.83 (m, 3 H) 4.10 - 4.41 (m, 5 H) 7.21 - 7.44 (m, 4 H) 7.57 (dd, *J*=10.11, 8.06 Hz, 3 H) 7.72 (d, *J*=7.33 Hz, 2 H) 8.55 (d, *J*=1.76 Hz, 1 H) 8.70 (dd, *J*=8.20, 1.76 Hz, 1 H) ¹³C NMR (75 MHz, CHLOROFORM-*d*) δ ppm 13.35 (s, 1 C) 17.68 (s, 1 C) 18.59 (s, 1 C) 19.22 (s, 1 C) 23.55 (s, 1 C) 23.62 (s, 1 C) 23.81 (s, 1 C) 24.01 (s, 1 C) 33.40 (s, 1 C) 46.62 (s, 1 C) 69.91 (s, 1 C) 71.36 (s, 1 C) 76.67 (s, 1 C) 77.09 (s, 1 C) 77.52 (s, 1 C) 119.96 (s, 1 C) 124.31 (s, 1 C) 125.21 (s, 1 C) 127.18 (s, 1 C) 127.83 (s, 1 C) 128.61 (s, 1 C) 131.93 (s, 1 C) 133.41 (s, 1 C) 139.59 (s, 1 C) 141.19 (s, 1 C) 143.27 (s, 1 C) 143.33 (s, 1 C) 150.18 (s, 1 C) 154.87 (s, 1 C) 165.19 (s, 1 C) ³¹P NMR (81 MHz, CHLOROFORM-*d*) δ ppm 35.07 (s, 1 P) C₄₀H₅₃NO₇P¹⁺ low resolution ESI-MS calculated: 690.30, found: 690.35.

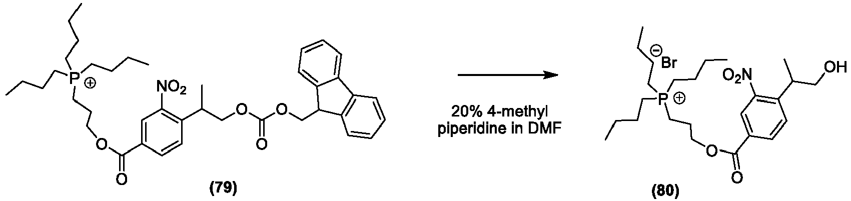

### Phosphonium tag 4-(1-hydroxypropan-2-yl)-3-nitrobenzoate (80)

To compound **(79)** (6.408 g, 9.29 mmol) was added a 20% solution of 4-methylpiperidine in DMF (20ml). After 2 h the reaction was complete by TLC and the solution was evaporated to dryness, then taken up in DCM and precipitated in 500ml of MTBE to yield 3.09 g of **(80)** (71%).

¹H NMR (400 MHz, CHLOROFORM-*d*) δ ppm 0.91 (t, *J*=7.03 Hz, 11 H) 1.26 (d, *J*=7.03 Hz, 3 H) 1.38 - 1.59 (m, 13 H) 1.98 (d, *J*=7.03 Hz, 2 H) 2.11 - 2.28 (m, 7 H) 2.36 - 2.51 (m, 2 H) 3.40 - 3.59 (m, 3 H) 3.64 - 3.82 (m, 2 H) 7.51 (d, *J*=8.21 Hz, 1 H) 8.22 (d, *J*=8.21 Hz, 1 H) 8.26 (s, 1 H) C₂₅H₄₃NO₅P¹⁺ low resolution ESI-MS calculated: 468.28, found: 468.28.

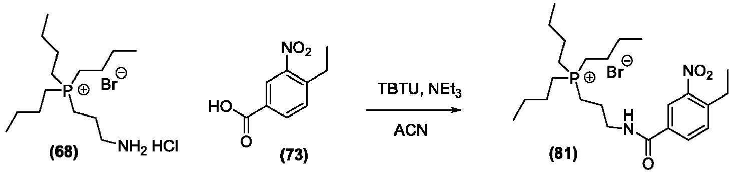

### Tributyl(3-(4-ethyl-3-nitrobenzamido)propyl)phosphonium bromide (81)

To a solution of 3-nitro-4-ethyl-benzoic acid **(73)** (3.15 g, 16.1 mmol) and diisopropylethylamine (4 eq, 11.25 ml) in ACN (30 mL) was added compound **(68)** (1.3 eq, 20.9 mmol) and TBTU (1.3 eq, 6.74 g, 20.9 mmol). This mixture was allowed to stir for 12 h. The dark brown solution was concentrated to a viscous oil, and taken up in DCM and precipitated in 500 ml of MTBE. The solid was collected and re-purified by silica gel column chromatography (DCM:MeOH, 100:0 → 85:15) eluting as very dark yellow oil. Isolated yield: 7.07 g (84%).

¹H NMR (500 MHz, CHLOROFORM-d) δ ppm 0.90 (t, *J*=6.97 Hz, 9 H) 1.24 (t, *J*=7.46 Hz, 3 H) 1.35 - 1.56 (m, 12 H) 1.96 (d, *J*=8.07 Hz, 2 H) 2.05 - 2.19 (m, 7 H) 2.25 - 2.38 (m, 2 H) 2.78 (s, 2 H) 2.87 (q, *J*=7.34 Hz, 2 H) 3.60 (q, *J*=5.79 Hz, 2 H) 7.40 (d, *J*=8.07 Hz, 1 H) 7.78 (t, *J*=5.50 Hz, 1 H) 8.07 (d, *J*=8.07 Hz, 1 H) 8.37 (s, 1 H) ¹³C NMR (126 MHz, CHLOROFORM-d) δ ppm 13.21 (s, 1 C) 14.61 (s, 1 C) 18.17 (s, 1 C) 18.56 (s, 1 C) 23.33 (s, 1 C) 23.37 (s, 1 C) 23.76 (s, 1 C) 23.88 (s, 1 C) 25.97 (s, 1 C) 38.57 (s, 1 C) 76.77 (s, 1 C) 77.02 (s, 1 C) 77.28 (s, 1 C) 123.82 (s, 1 C) 131.09 (s, 1 C) 131.47 (s, 1 C) 132.89 (s, 1 C) 141.77 (s, 1 C) 149.21 (s, 1 C) 165.48 (s, 1 C) C₂₄H₄₂N₂O₃P¹⁺ low resolution ESI-MS calculated: 437.29, found: 437.30.

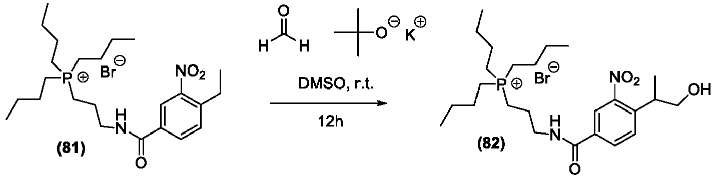

### Tributyl(3-(4-(1-hydroxypropan-2-yl)-3- nitrobenzamido)propyl) phosphonium bromide (82)

To a solution of compound **(81)** (3.56 g, 6.89 mmol) dry DMSO (13.8 mL), was added *para*-formaldehyde (2.1eq, 0.43 g, 14.4 mmol). This mixture was sonicated for 20 min till all of *para-*formaldehyde dissolved. The resulting mixture was treated with 1.5 eq of potassium tert-butoxide (1.16 g, 10.3 mmol). The reaction turned a dark purple immediately. The reaction was allowed stir for 12 h at room temperature. The reaction was monitored by MS, showing the disappearance of the starting material. The reaction was treated with 1M HCl in MeOH to bring to neutrality at which point the reaction was precipitated in diethyl ether, and then in DCM. This last precipitation step separated the product from unreacted para-formaldehyde. The product was purified by reverse phase chromatography, using 100 mM TEAA buffer in water (pH 7): ACN 80:20 → 20:80. Isolated yield: 1.3 g (35%).

¹H NMR (400 MHz, CHLOROFORM-*d*) δ ppm 0.84 - 0.98 (m, 9 H) 1.26 (d, *J*=7.03 Hz, 3 H) 1.38 - 1.59 (m, 11 H) 1.98 (d, *J*=5.86 Hz, 2 H) 2.09 - 2.27 (m, 6 H) 2.34 - 2.50 (m, 2 H) 3.39 - 3.49 (m, 1 H) 3.53 (d, *J*=5.47 Hz, 2 H) 3.64 - 3.81 (m, 2 H) 7.51 (d, *J*=8.21 Hz, 1 H) 8.22 (d, *J*=8.21 Hz, 1 H) 8.26 (s, 1 H) 8.45 (br. s., 1 H) ¹³C NMR (75 MHz, CHLOROFORM-d) δ ppm 13.38 (s, 1 C) 17.65 (s, 1 C) 18.43 (s, 1 C) 19.05 (s, 1 C) 23.61 (s, 1 C) 23.68 (s, 1 C) 23.78 (s, 1 C) 23.99 (s, 1 C) 36.68 (s, 1 C) 50.06 (s, 1 C) 66.17 (s, 1 C) 76.69 (s, 1 C) 77.11 (s, 1 C) 77.54 (s, 1 C) 124.73 (s, 1 C) 128.38 (s, 1 C) 129.42 (s, 1 C) 133.08 (s, 1 C) 144.51 (s, 1 C) 150.43 (s, 1 C) 164.33 (s, 1 C) ³¹P NMR (81 MHz, CHLOROFORM-*d*) δ ppm 35.07 (s, 1 P) C₂₅H₄₄N₂O₄P¹⁺ low resolution ESI-MS calculated: 467.30, found: 467.31.

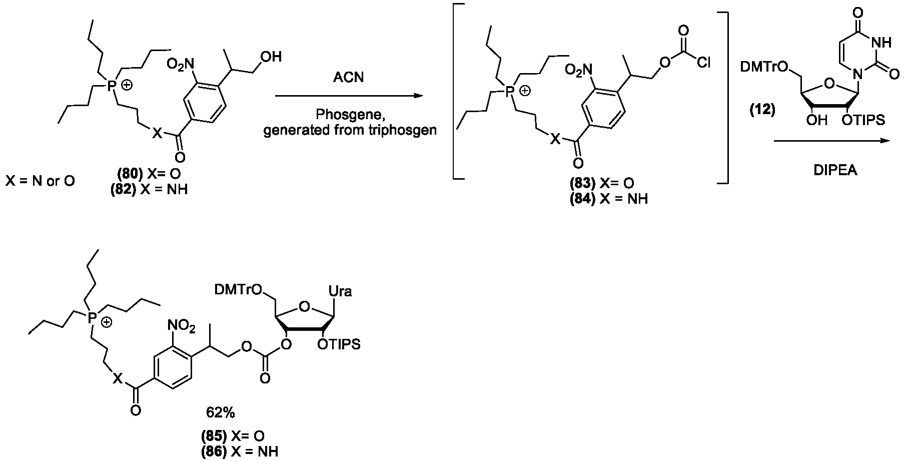

### 5'-DMTr-2'-TIPS-3'-( tributyl(3-(4-(1-hydroxypropan-2-yl)-3-nitrobenzamido)propyl) phosphonium bromide)-uridine (86)

A two necked flask containing the triphosgene and phenanthridine was connected to a distillation head and condenser. The receiving end of the condenser was attached to an ammonia trap and cooled by a dry ice/acetone bath to condense the phosgene that was produced. The bottom of the ammonia trap was connected to a three necked round bottom flask with a stir bar which was also cooled by a dry ice/acetone bath. All open necks of round bottoms were sealed by fresh septa wrapped with Teflon tape. One neck of the three necked flask was punctured with a 20G needle attached to a tygon tube and two bubblers in series; the first was empty and the second contained mineral oil. Tygon tubing was used to connect the second bubbler to a 9-inch 20G needle that was fully inserted into a saturated solution of sodium hydroxide in methanol. A second needle and tube was inserted into the septa of the methanolic sodium hydroxide, which acted as a vent up into the fume hood. Triphosgene (1.87 g, 6.33 mmol) and cat. phenanthridine were heated to 90°C, at which point the triphosgene melted and solvated the phenanthridine catalyst, promoting the evolution of phosgene gas. After 30 min, all triphosgene was consumed and phosgene had begun condensing in the receiving flask. At this point a balloon of argon was punctured through the septa on the two necked flask, pushing any phosgene gas to the condenser and quenching solution. Once phosgene had stopped condensing, an acetonitrile solution of **(80)** (1.06 g, 1.9 mmol) was added dropwise to the stirring phosgene, then removed from the dry ice/acetone bath after 10 min. The reaction was stirred for 2 h at room temperature. Next, argon gas was passed over the whole apparatus and also bubbled through the reaction mixture into the methanolic sodium hydroxide to remove and quench the excess phosgene. NOTE: use a very low flow from a balloon at first to ensure the phosgene was quenched. Once all phosgene was removed, DIPEA (4 mL) was added to the mixture to quench the HCl produced in the reaction with phosgene and compound **(82).**

A solution of nucleoside **(12)** (1 eq, 1.35 g, 1.9 mmol) in ACN (3 mL) was added directly to the above mixture, and the resulting solution allowed to stir for 8 h at room temperature. The solution was diluted with ethyl acetate and extracted with sat. NaHCO₃ (x3) and once with brine. The mixture was precipitated in 300 mL of MTBE to remove excess nucleoside and DIPEA. The resulting precipitate was then purified by column chromatography (DCM:MeOH 100:0 → 90:10) to afford 1.32 g of **(86)** (62% yield).

¹H NMR (300 MHz, CHLOROFORM-*d*) δ ppm 0.82 - 1.16 (m, 34 H) 1.34 (dd, J=6.89, 2.20 Hz, 3 H) 1.38 - 1.63 (m, 15 H) 2.05 (br. s., 2 H) 2.11 - 2.31 (m, 12 H) 2.67 (br. s., 1 H) 3.39 - 3.53 (m, 1 H) 3.57 - 3.72 (m, 3 H) 3.78 (s, 7 H) 4.08 - 4.38 (m, 2 H) 4.59 - 4.69 (m, 1 H) 5.16 - 5.33 (m, 2 H) 5.63 - 5.76 (m, 1 H) 6.00 (dd, *J*=5.27, 2.05 Hz, 1 H) 6.81 (dd, *J*=8.79, 1.47 Hz, 4 H) 7.15 (d, *J*=8.79 Hz, 1 H) 7.18 - 7.41 (m, 10 H) 7.56 (d, *J*=7.91 Hz, 1 H) 7.88 (dd, *J*=8.20, 1.76 Hz, 1 H) 8.53 (d, *J*=7.91 Hz, 1 H) 8.67 (t, *J*=8.94 Hz, 2 H) 9.64 (br. s., 1 H) C₆₅H₉₂N₄O₁₃PSi¹⁺ low resolution ESI-MS calculated: 1195.61, found: 1195.60.

### Ion-tagged synthesis of tetramer rGACU from nucleoside (86)

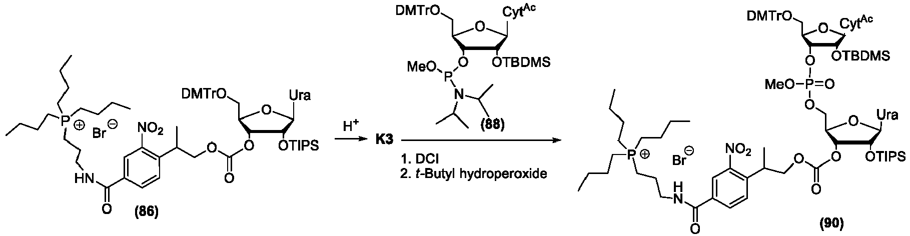

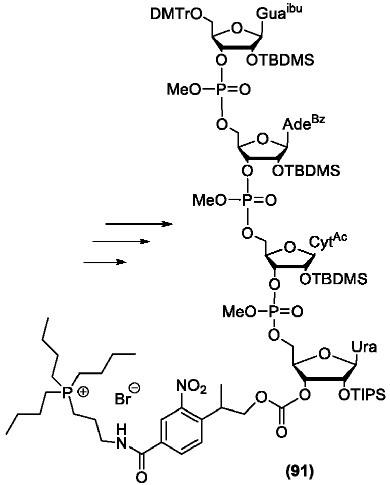

Reactions were carried out in the dark by wrapping the reactions flasks with aluminum foil.

### 5'-OH-2'-TIPS-3'-(tributyl(3-(4-(1-hydroxypropan-2-yl)-3-nitrobenzamido)propyl)phosphonium bromide)-uridine (K3)

3'-Tagged-uridine **(86)** (0.49 g, 0.38 mmol) was dissolved in 3% TFA in DCM and allowed to stir for 5 min before adding methanol to quench the trityl cation. The reaction was then concentrated to an oil taken up in minimal amounts of DCM and precipitated in MTBE to remove dimethoxytritanol. The compound was filtered over celite, collected in DCM and purified by column chromatography (DCM:MeOH, 100:0 →90:10%). Isolated yield of **K3:** 0.37 g (95%).

¹H NMR (500 MHz, ACETONITRILE-*d*₃) δ ppm 0.87 - 1.12 (m, 15 H) 1.38 (dd, *J*=6.97, 3.55 Hz, 1 H) 1.42 - 1.61 (m, 6 H) 1.96 (dt, *J*=4.89, 2.45 Hz, 1 H) 1.98 - 2.07 (m, 1 H) 2.07 - 2.17 (m, 3 H) 2.22 - 2.33 (m, 1 H) 3.49 (br. s., 1 H) 3.66 - 3.79 (m, 1 H) 4.13 (dd, *J*=13.45, 1.96 Hz, 1 H) 4.30 - 4.46 (m, 2 H) 4.57 - 4.66 (m, 1 H) 5.03 (ddd, *J*=10.82, 5.07, 1.71 Hz, 1 H) 5.70 (d, *J*=8.07 Hz, 1 H) 5.85 (dd, *J*=6.97, 4.28 Hz, 1 H) 7.73 - 7.83 (m, 1 H) 8.27 (d, *J*=8.31 Hz, 1 H) 8.39 (t, *J*=1.71 Hz, 1 H) 9.17 (br. s., 1 H) ¹³C NMR (126 MHz, ACETONITRILE-*d*₃) δ ppm -0.16 (s, 1 C) 0.17 (s, 1 C) 0.34 (s, 1 C) 0.36 (s, 1 C) 0.50 (s, 1 C) 0.52 (s, 1 C) 0.54 (s, 1 C) 0.67 (s, 1 C) 0.83 (s, 1 C) 11.91 (s, 1 C) 11.93 (s, 1 C) 12.57 (s, 1 C) 15.13 (s, 1 C) 15.52 (s, 1 C) 16.93 (s, 1 C) 16.99 (s, 1 C) 17.00 (s, 1 C) 17.01 (s, 1 C) 17.11 (s, 1 C) 17.69 (s, 1 C) 18.07 (s, 1 C) 20.64 (s, 1 C) 20.67 (s, 1 C) 22.84 (s, 1 C) 22.88 (s, 1 C) 23.45 (s, 1 C) 23.57 (s, 1 C) 33.49 (s, 1 C) 33.64 (s, 1 C) 61.34 (s, 1 C) 61.37 (s, 1 C) 64.62 (s, 1 C) 64.76 (s, 1 C) 71.11 (s, 1 C) 71.31 (s, 1 C) 73.49 (s, 1 C) 76.99 (s, 1 C) 77.10 (s, 1 C) 82.99 (s, 1 C) 87.73 (s, 1 C) 87.76 (s, 1 C) 102.64 (s, 1 C) 102.66 (s, 1 C) 117.32 (s, 1 C) 124.83 (s, 1 C) 124.86 (s, 1 C) 129.10 (s, 1 C) 129.32 (s, 1 C) 129.84 (s, 1 C) 129.85 (s, 1 C) 133.06 (s, 1 C) 133.08 (s, 1 C) 140.43 (s, 1 C) 140.46 (s, 1 C) 141.68 (s, 1 C) 141.82 (s, 1 C) 150.41 (s, 1 C) 150.53 (s, 1 C) 150.81 (s, 1 C) 150.83 (s, 1 C) 154.10 (s, 1 C) 154.15 (s, 1 C) 162.65 (s, 1 C) 164.03 (s, 1 C) 164.04 (s, 1 C) C₄₄H₇₄N₄O₁₁PSi¹⁺ low resolution ESI-MS calculated: 893.48, found: 893.50.

**Synthesis of rCpU (90).** Compound **K3** (0.37 g, 0.38mmol) was dried by two co-evaporations of dry toluene:DCM on a rotovap before placed under high vacuum. K3 was dissolved in 15 ml of ACN containing 2 eq of DCI (0.982 g, 8.32 mmol). Immediately after, phoshoramidite **(88)** (0.51 g 0.57 mmol) was added, and the resulting solution was allowed to stir at room temperature for 3 h. MS analysis indicated that the reaction was complete (no starting material K3 present). Ten eq of *tert*-butanol was added to quench excess phosphoramidite, followed by 10 eq of *tert*-butyl hydroperoxide (1 mL of a 6 M solution in decane), and the resulting mixture allowed to stir for 20 min until all the phosphite triester was converted to the phosphate, as monitored by MS. The reaction was then concentrated to an oil, taken up in minimal amounts of DCM, and precipitated in MTBE to remove all excess reagents. The precipitation process was repeated if the presence of any quenched phosphoramidite was detected by TLC. Tagged dimer **(90)** was isolated in 95% yield (0.63 g). C₆₃H₁₂₂N₇O₂₁P₂Si₂¹⁺ low resolution ESI-MS calculated: 1670.77, found: 1670.73.

The above process was repeated as described above, using the appropriate phosphoramidites till tetramer **(92)** was obtained. Intermediates isolated were characterized (data shown in Table 7 below).

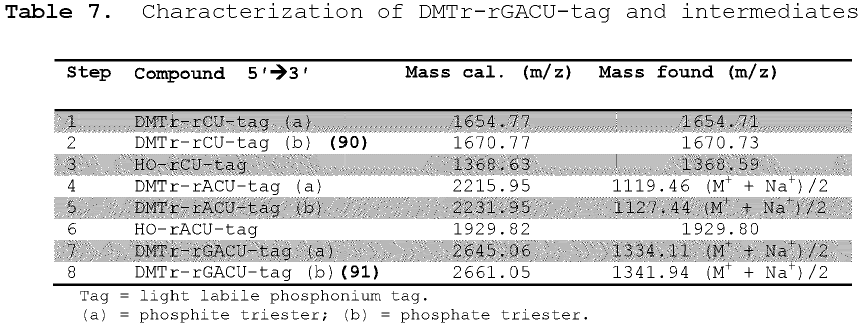

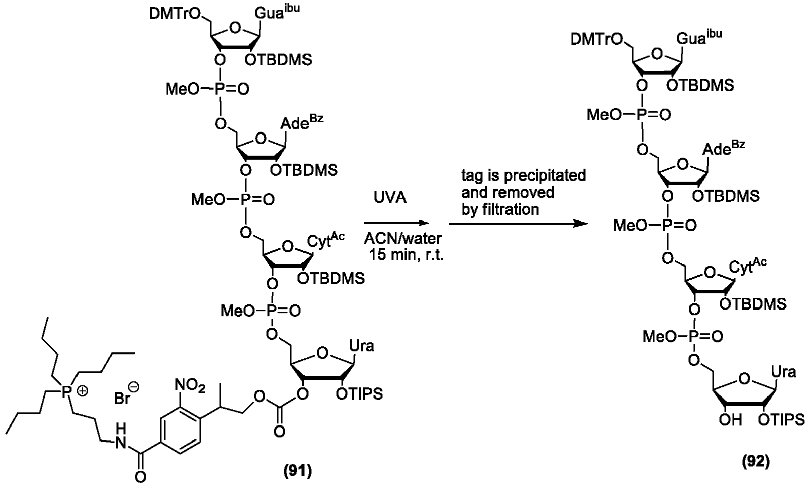

### Photocleavage of orthogonal phosphonium tag from tetramer (91), affording protected DMTr-rGACU-3'OH (92)

Teramer **(91)** (0.120 g, 0.042mmol) was dissolved in 1 mL of wet ACN (1200 ppm of H₂O), and transferred into a quartz cuvette (no frosted sides) containing a small stir bar. The cuvette was placed inside a photoreactor for 15 min with stirring. The reaction appeared complete (TLC analysis). The mixture was concentrated to about half volume and the cleaved tag precipitated in MTBE (25 mL) and filtered to afford a white powder. The desired tetramer was found in the MTBE solution, which was collected after concentrating the solution to dryness. Isolated yield: 0.092g, 0.00403 mmol (95%).

### Ion-tagged supported RNA synthesis via dimer block coupling.

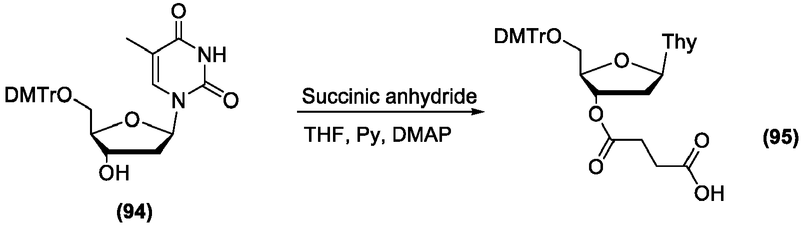

Compound **(94)** (7.9 g, 14.5 mmol) was dissolved in 75 ml of THF, 150ml of pyridine and 5 ml of DMF. Succinic anhydride (7.25 g, 72.5 mmol) was added with 5% w/w, 0.12g of 4-dimethylaminopyridine (DMAP). This solution stirred for 24h, then heated to 50 °C for 4 h. The solution was then condensed and redissolved in 400ml of dichloromethane (DCM) and extracted with 15% NaCl w/v (300ml x3) to remove excess succinic anhydride. The organic layer was collected and dried over magnesium sulphate, filtered and condensed to dryness. The crude mixture was purified using flash chromatography with silica gel (pre-treated with triethylamine), eluting with a DCM-MeOH gradient (100%DCM→95%DCM-5%MeOH). Yield of **(95):** 8.5 g (91 %).

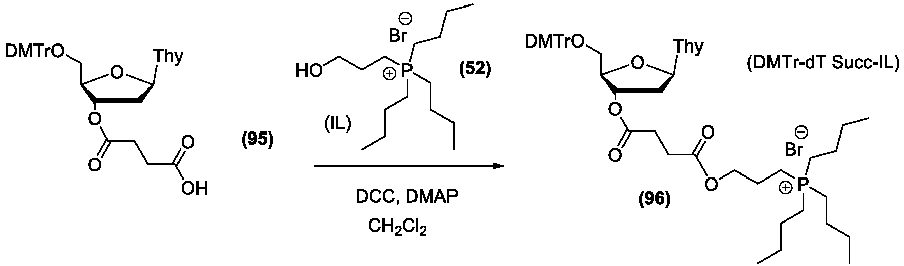

Compound **(52)** (12 g, 18.6 mmol) was added to a stirring solution of **(95)** (12 g, 18.6 mmol) and freshly distilled dicyclohexylcarbodiimide (DCC) (2 eq) in 100ml of dry CH₂Cl₂. The reaction mixture was stirred for 3 days at room temperature. TLC analysis in 9:1 dichloromethane-methanol was performed for the disappearance of compound **(95).** The crude reaction mixture was filtered to remove the precipitated dicyclohexylurea (*DCU*) then condensed to reduce the volume of DCM washed with 200ml 15% NaCl w/v solution (x2). The organic layer was collected and dried over magnesium sulphate filtered and condensed to dryness. This crude mixture was dissolved in 1:1 acetone, acetonitrile and precipitated in MTBE for 2-3 times to afford **(96)** in 83% yield (14.9 g).

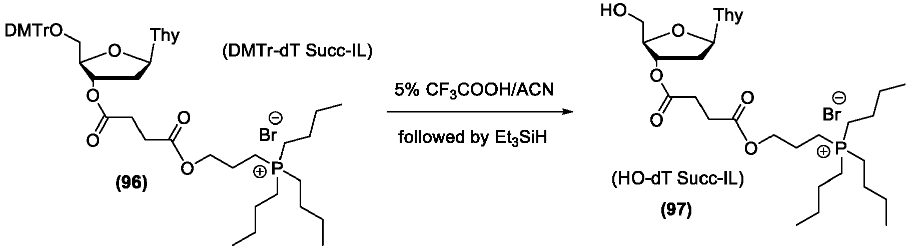

Detritylation of compound **(96)** (2.78, 2.88 mmol) was performed by adding 5 eq of trifluoroacetic acid as a 5% v/v solution in acetonitrile and 5 eq triethylsilane and stirring for 5-10 min. Three trifluoroacetic acid treatments were required to completely remove the trityl group. The reaction mixture was precipitated from MTBE ether after each trifluoroacetic acid treatment and finally twice to make sure that no traces of acid remained. Then the anticipated compound **(97)** was characterized by ¹H NMR, MS and alternatively by TLC. After the necessary number of detritylations were completed, the compound **(97)** (1.4 g, 73.3%) was condensed into a 250 mL round bottom flask and dried on high vacuum for a minimum of one hour to remove residual solvent, TFA and water, as any of these will adversely affect the next coupling step. The detritylated compound was highly hygroscopic so it should not be exposed to air.

¹H NMR (400 MHz, CD₃CN) δ 9.19(brs, 1H), 7.72 (s, 1H), 6.20 (dd, *J* = 8.6, 5.9, 1H), 6.20 (dd, *J* = 8.6, 5.9, 1H), 5.36 - 5.25 (m, 1H), 4.15 (t, *J* = 5.9, 3H),4.02-3.98 (m, 1H), 3.75-3.67 (m, 2H), 2.69-2.55 (m, 4H), 2.37-2.03 (m, 13H), 1.89-1.76 (m, 2H), 1.59-1.35 (m, 12H), 0.94 (t, *J* = 7.1, 9H).

¹³C NMR (400 MHz, CD₃CN) δ 173.5, 173.1, 165.1, 152, 137.4, 118.8, 86.3, 85.8, 76.9, 65, 64.8, 62.8, 38.3, 30.3, 30.1, 27.5, 24.9, 24.7, 24.2, 22, 19.5, 19, 16.8, 16.3, 14, 13.

³¹P NMR (200 MHz, CD₃CN) δ 35.475.

ESI-MS for C₂₉H₅₀N₂O₈P⁺: 585.33.

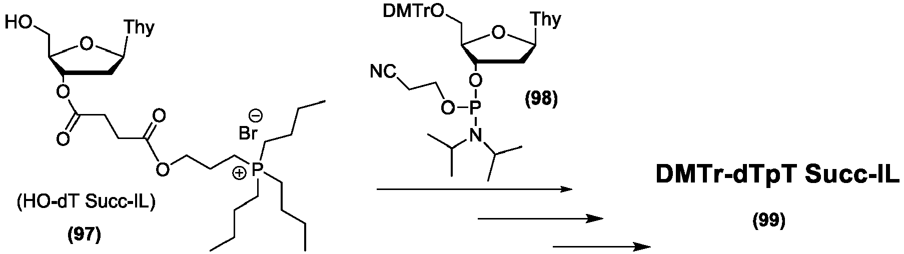

Amidite **(98)** (2.35g, 3.15mmol) and 4, 5-dicyanoimidazole (DCI) (0.49g, 4.2mmol) was co-evaporated with benzene and dried under vaccum for 2 hours prior to starting the reaction. The compound **(97)** (1.4g, 2.1 mmol) was co-evaporated with benzene and dried under vaccum for 2 hours. 15% v/v dimethylformamide (DMF) in acetonitrile (ACN) was added to make a 0.06 M solution of oligonucleotide (0.12 M in amidite) . 26 mL of the solvent was added to the amidite and DCI with syringe. The above solution was transferred to the oligo with cannula. The above reaction mixture was stirred for 3 hours.

After three hours, *tert-*butanol was melted (m.p. 25°C), 1 eq were added and allowed to stir for 10 min to quench the excess amidite, making it more soluble in ether. This solution was then added to a small dropper funnel, rinsing the flask with ACS grade acetone to final volume of about 40ml. This solution was precipitated by dropwise addition to 500-600 mL of methyl *tert-*butyl ether (MTBE) over about 15min. The solution was then filtered over Celite^{™} to collect the precipitated dimer DMTr-dTT Succ IL **(99).** The MTBE was then condensed under reduced pressure and the remaining solution was once again precipitated from ether to recover oligomer that was dissolved in the MTBE.

After the sample was collected and condensed to dryness, the sample was re-suspended in about 30ml of acetone and THF and precipitated once more to ensure complete removal of excess amidite before oxidation.

The phosphite triester containing oligomer was dissolved in same amount of 15% v/v DMF in acetonitrile and 0.5 eq each of Cap A and Cap B solution (16% N-methylimidazole in THF, and acetic anhydride:pyridine:THF, 1:2:2 ratio) was added. The reaction was allowed to stir for 10min. Make sure to only allow 10min as longer reaction times discolour the reaction mixture to a dark yellow/brown color. Next, 5 eq of 6M *tert*-butyl hydroperoxide was added in decane to oxidize. Then it was allowed to stir for a further 15min. This solution was then again precipitated as described above, recovering the sample from the filter in a 250 mL round bottom flask. This solid compound was then placed under a high vacuum for 15-20 min to make sure there was no residual *tert*-butyl hydroperoxide or pyridine remaining.

¹³C NMR (400 MHz, CD₃CN) δ 208, 173.4, 173.2, 165.3, 160, 151.9, 145.9, 136.7, 131.3, 129.2, 128.3, 114.4, 111.9, 87.9, 86.1, 85.5, 83.3, 75.2, 68.8, 64.9, 64.4, 64.1, 56.2, 48.2, 39.4, 37.5, 31.2, 30, 29.8, 24.8, 24.7, 24, 21.8, 20.5, 19.3, 18.8, 16.6, 16.1, 13.9, 12.9, 12.5.

³¹P NMR (200 MHz, CD₃CN) δ 35.457, -1.599, -1.720.

ESI-HRMS for C₆₃H₈₄N₅O₁₇P₂⁺: Calcd 1244.52949, found 1244.52977.

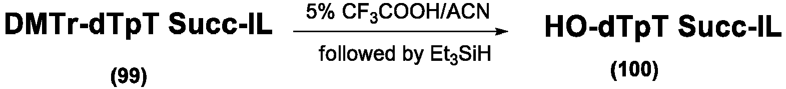

Detritylation of compound **(99)** (2.0 g, 1.51 mmol) was performed by adding 5 eq of trifluoroacetic acid as a 5% v/v solution in acetonitrile and 5 eq triethylsilane and stirring for 5-10 min. Three trifluoroacetic acid treatments were required to completely remove the dimethoxytrityl group. The reaction mixture was precipitated from MTBE ether after each trifluoroacetic acid treatment and finally twice to remove traces of acid. After the necessary number of detritylations are completed, compound **(100)** (1.506 g, 97.7%) was dried on high vacuum for a minimum of one hour to remove residual solvent, TFA and water, as any of these will adversely affect the next coupling step. The detritylated compound was highly hygroscopic so it should not be exposed to air.

¹³C NMR (400 MHz, CD₃CN) δ 207.7, 173.1, 165.3, 151.7, 137.3,111.9, 111.4, 86.7, 85.7, 85.4, 83.1, 80.4, 74.9, 69.4, 68.4, 64.5, 63.9, 62.2, 55.4, 39.1, 37.1, 31, 29.8, 29.6, 24.5, 24.4, 23.8, 22.8, 21.6, 20.2, 19, 18.5, 16.2, 15.8, 13.6, 12.6.

³¹P NMR (200 MHz, CD₃CN) δ 35.41, -1.67.

ESI-HRMS for C₄₂H₆₆N₅O₁₅P₂⁺: Calcd 942.40067, found 942.39979.

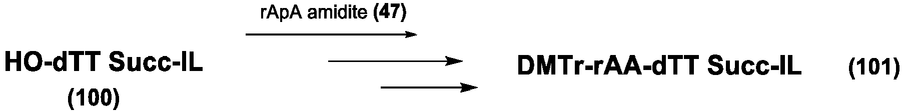

rApA amidite **(47)** (0.455 g, 0.293 mmol) and 4, 5-dicyano imidazole (DCI) (0.04 g, 0.342 mmol) were co-evaporated with benzene and dried under vaccum for 2 hours. 5'-HO-dTT Succ-IL **(100)** (0.1 g, 0.098 mmol) was co-evaporated with benzene and dried under vaccum for 2 hours. 1.95 mL of 15% v/v dimethylformamide (DMF) in acetonitrile (ACN) was added to the DCI and transfered to the amidite. This solution was then transferred to (101) via a cannula, and the resulting mixture stirred for 48 hours.

After three hours, melted *tert*-butanol (m.p. 25°C) was added (10 eq) to quench the amidite and the mixture allowed to stir for 10 min. This solution was then added to a small dropper funnel, rinsing the flask with ACS grade acetone to final volume of about 7 ml. This solution was precipitated by dropwise addition to 60-70 ml of methyl *tert*-butyl ether (MTBE) over a 15 min period. The solid was filtered over celite. The remaining solution was condensed under reduced pressure and again precipitated from ether to recover more oligomer.

After the sample was collected, the sample was re-suspended in about 30 ml of acetone and THF and precipitated once more to ensure complete removal of excess monomer before oxidation.

The phosphite triester containing oligomer was dissolved in same amount of 15% v/v DMF in acetonitrile and 6 M *tert*-butylhydro peroxide was added in decane to effect oxidation (15 min). This solution was again precipitated, recovering the sample from the filter in a 100 ml round bottom flask. The solid compound was then placed under a high vacuum for 15-20 min to ensure complete removal of residual *tert*-butyl hydroperoxide.

The oligomer was dissolved in 15% v/v DMF in acetonitrile and 2 eq each of Cap A and Cap B solution (16% N-methylimidazole in THF, and acetic anhydride:pyridine:THF, 1:2:2 ratio) was added. The reaction was allowed to stir for 10 min. This solution was precipitated, recovering the sample from the filter in a 100 ml round bottom flask. The compound collected was transferred into a 25 mL round bottom flask, co-evaporated with benzene twice and then placed under a high vacuum for 2 hours for the next reaction. Yield of (101): 95 %

³¹P NMR (200 MHz, Acetone-d₆) δ 35.56, 1.67 to -0.39, -0.87 to - 1.54.

ESI-MS for C₁₁₄H₁₅₄N₁₅O₃₁P₄Si₂⁺: calcd 2408.94, found 2408.93987.

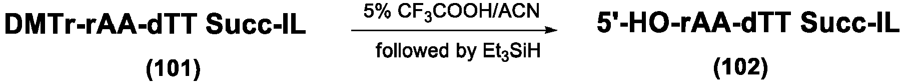

Detritylation of compound **(101)** (0.2 g, 0.08 mmol) was performed by adding 5 eq of trifluoroacetic acid as a 5% v/v solution in acetonitrile and 5 eq triethylsilane and stirring for 5-10 min. Two trifluoroacetic acid treatments were required to completely remove the dimethoxytrityl group. The reaction mixture was precipitated from MTBE ether after each trifluoroacetic acid treatment and finally twice to remove traces of acid. After the necessary number of detritylations were completed, compound **(102)** (0.172 g, 98%) was dried on high vacuum for a minimum of one hour to remove residual solvent, TFA and water, as any of these will adversely affect the next coupling step. The detritylated compound was highly hygroscopic so it should not be exposed to air.

³¹P NMR (200 MHz, Acetone-d₆) δ 35.49, 1.68 to -0.18, -1.0 to - 1.55.

ESI-MS for C₉₃H₁₃₆N₁₅O₂₉P₄Si₂⁺: calcd 2106.81 found 2106.80889.

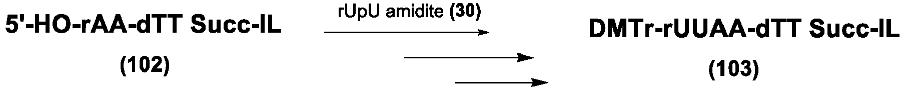

Amidite **(30)** (0.288 g, 0.222 mmol) and 4, 5-dicyanoimidazole (DCI) (0.03 g, 0.259 mmol) were co-evaporated with benzene and dried under vaccum for 2 hours. The 5'-OH-tetramer **(102)** (0.162 g, 0.074 mmol) was co-evaporated with benzene and dried under vaccum for 2 hours. 1.48 mL of 15% v/v dimethylformamide (DMF) in acetonitrile (ACN) was added to the DCI and transferred to the amidite. The above solution was transferred to the oligo via a cannula, and the resulting mixture stirred for 48 hours at r.t.

After three hours, melted tert-butanol (m.p. 25°C) was added (10 eq) to quench the amidite and the mixture allowed to stir for 10 min. This solution was then added to a small dropper funnel, rinsing the flask with ACS grade acetone to final volume of about 6 ml. This solution was precipitated by dropwise addition to 60-70 ml of methyl tert-butyl ether (MTBE) over a 15 min period. The solid was filtered over celite. The remaining solution was condensed under reduced pressure and again precipitated from ether to recover more oligomer.

After the sample was collected, the sample was re-suspended in about 30 ml of acetone and THF and precipitated once more to ensure complete removal of excess monomer before oxidation.

The phosphite triester containing oligomer was dissolved in same amount of 15% v/v DMF in acetonitrile and 6 M tert-butylhydro peroxide was added in decane to effect oxidation (15 min). This solution was again precipitated, recovering the sample from the filter in a 100 ml round bottom flask. The solid compound was then placed under a high vacuum for 15-20 min to ensure complete removal of residual *tert*-butyl hydroperoxide.

The oligomer was dissolved in 15% v/v DMF in acetonitrile and 2 eq each of Cap A and Cap B solution (16% N-methylimidazole in THF, and acetic anhydride:pyridine:THF, 1:2:2 ratio) was added.

The reaction was allowed to stir for 10 min. This solution was precipitated, recovering the sample from the filter in a 100 ml round bottom flask. The compound collected was transferred into a 25 mL round bottom flask, co evaporated with benzene twice and then placed under a high vacuum for 2 hours for the next reaction. Yield of **(103):** 99 %

³¹P NMR (200 MHz, Acetone-d₆) δ 35.56, 1.73 to -0.11, -0.87 to - 1.65

ESI-MS for C₁₄₉H₂₁₄N₁₉O₄₇P₆Si₄Na²⁺: calcd 1671.1148, found 1671.11493

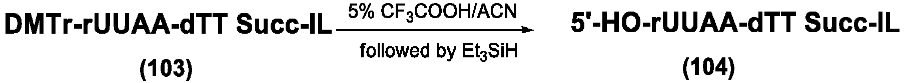

Detritylation of compound **(103)** (0.24 g, 0.072 mmol) was performed by adding 5 eq of trifluoroacetic acid as a 5% v/v solution in acetonitrile (10-15 min) followed by 5 eq triethylsilane and stirring for 10 min. Two trifluoroacetic acid treatments were required to completely remove the dimethoxytrityl group. The reaction mixture was precipitated from MTBE ether after each trifluoroacetic acid treatment and finally twice to remove traces of acid. After the necessary number of detritylations were completed, compound **(104)** (0.21 g, 94%) was dried on high vacuum for a minimum of one hour to remove residual solvent, TFA and water, as any of these will adversely affect the next coupling step.

³¹P NMR (200 MHz, Acetone-d₆) δ 35.55, 1.62 to -0.17, -0.89 to - 1.59.

ESI-MS for C₁₂₈H₁₉₆N₁₉O₄₅P₆Si₄Na²⁺: calcd 1520.055, found 1520.05007.

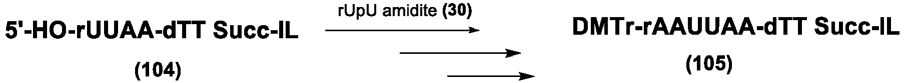

Amidite **(30)** (0.187 g, 0.121 mmol) and 4, 5-dicyanoimidazole (DCI) (0.016 g, 0.14 mmol) were co-evaporated with benzene and dried under vaccum for 2 hours prior to start the reaction. The 5'-OH-hexamer **(104)** (0.125 g, 0.04 mmol) was co-evaporated with benzene and dried under vaccum for 2 hours. 0.8 mL of 15% v/v dimethylformamide (DMF) in acetonitrile (ACN) was added to the DCI and transferred to the amidite. The above solution was transferred to the oligo with cannula, and the resulting mixture stirred for 48 hours at r.t.

After three hours, melted *tert*-butanol (m.p. 25°C) was added (10 eq) to quench the amidite and the mixture allowed to stir for 10 min. This solution was added to a small dropper funnel, rinsing the flask with ACS grade acetone to final volume of about 5 ml. This solution was precipitated by dropwise addition to 40-50 ml of methyl tert-butyl ether (MTBE) over about 15min. The solution was then filtered over celite to collect the precipitated oligo. The remaining solution was condensed under reduced pressure and again precipitated from ether to recover more oligomer.

After the sample was collected, the sample was re-suspended in about 30 ml of acetone and THF and precipitated once more to ensure complete removal of excess monomer before oxidation.

The phosphite triester containing oligomer was dissolved in same amount of 15% v/v DMF in acetonitrile and 6 M *tert*-butyl hydro peroxide was added in decane to effect oxidation (15 min). This solution was again precipitated, recovering the sample from the filter in a 50 ml round bottom flask. The solid compound was then placed under a high vacuum for 15-20 min to ensure complete removal of residual *tert*-butyl hydroperoxide.

The oligomer was dissolved in 15% v/v DMF in acetonitrile and 2 eq each of Cap A and Cap B solution (16% N-methylimidazole in THF, and acetic anhydride:pyridine:THF, 1:2:2 ratio) was added. The reaction was allowed to stir for 10 min. This solution was precipitated, recovering the sample from the filter in a 50 ml round bottom flask. The compound collected was transferred into a 10 mL round bottom flask, co-evaporated with benzene twice and then placed under a high vacuum for 2 hours for the next reaction. Yield of **(105):** 98 %

³¹P NMR (200 MHz, Acetone-d₆) δ 35.6, 2.93 to -0.63, -0.89 to - 1.84.
Formula C₂₀₀H₂₈₄N₂₉O₆₁P₈Si₆⁺

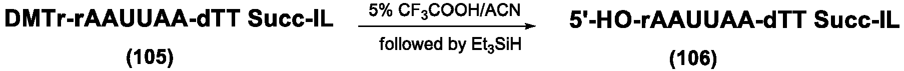

Detritylation of compound **(105)** (0.15 g, 0.032 mmol) was performed by adding 5 eq of trifluoroacetic acid as a 5% v/v solution in acetonitrile (10-15 min) followed by 5 eq triethylsilane and stirring for 10 min. Three trifluoroacetic acid treatments were required to completely remove the dimethoxytrityl group. The reaction mixture was precipitated from MTBE ether after each trifluoroacetic acid treatment and finally twice to make sure that no traces of acid remained. After the necessary number of detritylations were completed, compound **(106)** (0.138 g, 99%) was dried on high vacuum for a minimum of one hour to remove residual solvent, TFA and water, as any of these will adversely affect the next coupling step.

³¹P NMR (200 MHz, Acetone-d₆) δ 35.52, 4.99 to -0.62, -0.82 to - 3.54.
Formula C₁₇₉H₂₆₆N₂₉O₅₉P₈Si₆⁺

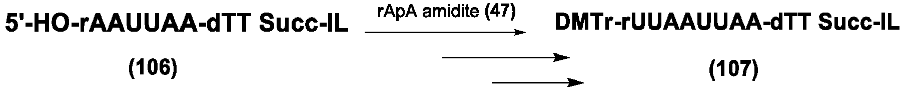

Amidite **(47)** (0.119 g, 0.121 mmol) and 4, 5-dicyanoimidazole (DCI) (0.012 g, 0.107 mmol) was co-evaporated with benzene and dried under vaccum for 2 hours prior to start the reaction. The 5'- OH-octamer **(106)** (0.13 g, 0.03 mmol) was co-evaporated with benzene and dried under vaccum for 2 hours. 0.61 mL of 15% v/v dimethylformamide (DMF) in acetonitrile (ACN) was added to the DCI and transfered to the amidite. The above solution was transferred to the oligo with cannula. The above reaction mixture was stirred for 48 hours.

After three hours, melted tert-butanol (m.p. 25°C) was added (10 eq) to quench the amidite and the mixture allowed to stir for 10 min. This solution was then added to a small dropper funnel, rinsing the flask with ACS grade acetone to final volume of about 5 ml. This solution was precipitated by dropwise addition to 40-50 ml of methyl tert-butyl ether (MTBE) over about 15 min. The solution was then filtered over celite to collect the precipitated oligo. The MTBE was condensed under reduced pressure and the remaining solution was once again precipitated from ether to recover more oligomer.

The sample was collected and condensed to dryness, re-suspended in about 30ml of acetone and THF, and precipitated once more to ensure complete removal of excess monomer before oxidation.

The phosphite triester containing oligomer was dissolved in same amount of 15% v/v DMF in acetonitrile and 6 M *tert*-butyl hydro peroxide was added in decane to effect the oxidation, and the mixture allowed to stir for 15min. This solution was again precipitated as described above, recovering the sample from the filter in a 50 ml round bottom flask. This solid compound was then placed under a high vacuum for 15-20 min to make sure there was no residual *tert*-butyl hydro peroxide or pyridine remaining.

The above reaction was repeated one more time. Yield of **(107):** 96 %.

³¹P NMR (200 MHz, Acetone-d₆) δ 35.57, 3.1 to - 3.76.
Formula C₂₃₅H₃₄₄N₃₃O₇₇P₁₀Si₈⁺

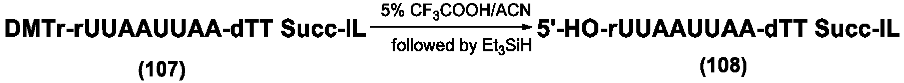

Detritylation of compound **(107)** (0.015 g, 0.0027 mmol) was performed by adding 5 eq of trifluoroacetic acid as a 5% v/v solution in acetonitrile and 5 eq triethylsilane and stirring for 10-15 min. Three trifluoroacetic acid treatments were required to completely remove the dimethoxytrityl group. The reaction mixture was precipitated from MTBE ether after each trifluoroacetic acid treatment and finally twice to ensure complete removal of the acid. After the necessary number of detritylations were completed, compound **(108)** (0.013 g, 93%) was precipitated, and dried in a speed-Vac concentrator for several hours.

### General Procedures for the Deprotection of Oligonucleotides prepared via Ion-Supported Method.

A 5-10 mg sample of the oligonucleotide was placed in an eppendorf and fully deprotected by carrying out the following steps.

Detritylation was effected using a solution of 5% CF₃CO₂H in MeCN (7.71 mL/mmol of oligo; 10-15 min, r.t.), and then adding Et₃SiH (0.79 mL / mmol of oligo; 5-10 min, r.t.) followed by precipitation in MTBE. This step was repeated 2-3 times till detritylation was complete.

Reagent for removing methyl phosphate protecting group; A= 2-methyl-5-*tert*-butylthiophenol, ambient temperature, 2 h, or, B= *tert*-butylamine, 50 °C, 16 h. Following demethylation, the oligonucleotides were treated with 1:3 (ethanol: 29% *aq.* NH₄OH, v/v), r.t., 48 h, to remove N-benzoyl groups. One final step removed TIPS and TBDMS groups (NEt₃.3HF; 300 µL/µmol of oligo, r.t., 48 h).

Following deprotection, the samples are analyzed by HPLC and characterized by MS, and by comparison to the same oligomers prepared via solid-phase (see Table 5 and Figure 17).

### Solid-phase Oligonucleotide Synthesis:

Slight modifications to the standard phosphoramidite solid-phase synthesis conditions were used for the synthesis of all oligonucleotides (see below for details). Syntheses were performed on an Applied Biosystems 3400 DNA Synthesizer at a 1 µmole scale using thymidine derivatised to CPG support through a succinyl linker [Damha, M. J. et al. Nucleic Acids Res. 18: 3813-21 (1990)] All phosphoramidites were prepared as 0.15 M solutions in acetonitrile (ACN). 4,5-Dicyanoimidazole (0.25 M in ACN) was used to activate phosphoramidites for coupling. Detritylations were accomplished with 3% trichloroacetic acid in CH₂Cl₂ (110 s). Capping of failure sequences was achieved with acetic anhydride in THF and 16% N-methylimidazole in THF for (30 s). Oxidation was done using a 3M solution of *tert-*butylhydroperoxide in toluene (40 s).

This solution was prepared as described in a literature procedure [Hartsel, S.A., Kitchen, D.E., Scaringe, S.S., and Marshall, W.S. (2005). RNA oligonucleotide synthesis via 5'-silyl-2'-orthoester chemistry. In Methods in Molecular Biology, Vol 288: Oligonucleotide Synthesis: Methods and Applications (P. Herdewijn, ed.) pp.33-49. Humana Press, Totowa, N.J.]: "In a 2-L separatory funnel, shake 360 mL of 70% t-butyl hydroperoxide with 1.5 L of toluene. Allow layers to separate for 30 min and discard the bottom aqueous layer. Decant the top layer into a bottle, and place in a freezer with a sintered glass funnel. Allow the water to freeze, then using vacuum filtration quickly separate the reagent from the ice crystals using the chilled fritted filter. Store reagent at -20°C."

Coupling times were 600 see for standard RNA cyanoethyl amidites, The dimer and trimer amidites (**25**), (**30**) and (**50**) were coupled for 1200s.

### Deprotection of Oligonucleotides:

The oligonucleotides were demethylated by treatment with a solution of 2-methyl-5-tert-butylthiophenol:triethylamine: acetonitrile (1:1:3; v/v/v) at ambient temperature for 2 h. Cleavage from the solid support was accomplished with either 3:1 NH₄OH:EtOH for 48 hours at room temperature, or with saturated ethanolic ammonia at 55°C [Scaringe S.A. et al. Nucleic Acids Research 18, 5433 (1990)] or room temperature. The ethanolic ammonia was prepared directly before use by taking freshly distilled ethanol that was stored 3Å molecular sieves and cooling it to 0°C. Ammonia gas was bubbled through the solution with stirring for 15min. This solution was kept over argon and stored at 0°C on ice until use. Desilylation was achieved with neat triethylamine trihydrofluoride (TREAT-HF) for 48 hours at room temperature. The crude oligonucleotides were precipitated from the TREAT-HF by quenching the solution with 30µL of a 3M sodium acetate solution buffered to pH 5.5. The quenched solution was washed away by the addition of dry, cold (-20°C) 1-butanol. The sample was centrifuged to a pellet and the butanol was decanted. This procedure was repeated once more and the sample was either immediately analyzed by HPLC or it was desalted with Nap-25 Sephadex columns from GE Healthcare.

Anion-exchange HPLC was performed on a Agilent HPLC using a Waters Protein Pack DEAE- 5PW column (7.5 mm x 7.5 cm). MALDI-TOF mass spectrometry was carried out on a Kratos Kompact III instrument.

## Claims

1. A compound of formula (II): 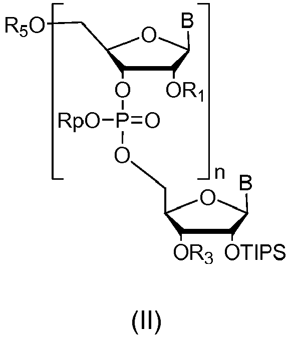 wherein
n is an integer from 1 to 19;
R₁ is a protecting group selected from the group consisting of t-butyldimethylsilyl (TBDMS), triisopropylsilyl (TIPS); triisopropyloxymethyl (TOM); cyanoethylmethyl (CEM); 2-(4-tolylsulfonyl)ethoxymethyl (TEM)2'-O-bis(2-acetoxyethoxy)methyl (ACE) orthoester; 1-(2-fluorophenyl)-4-methoxypiperidin-4-yl (Fpmp); 1-(4-chlorophenyl)-4-ethoxypiperidin-4-yl (Cpep); 4-(N-dichloroacetyl-N-methylamino) benzyloxymethyl (4-MABOM); dimethoxytrityl (DMTr), monomethoxytrityl (MMTr) 2-tert-butyldithiomethyl (DTM); allyl levulinyl (Lev) and acetal levulinyl (ALE); 2-(2-nitrophenyl)propoxycarbonyl (NPPOC), α-methylnitropiperonyloxycarbonyl (MeNPOC), thioxanthone-nitrobenzyl group conjugates, and a 5'-O-dimethoxybenzoincarbonate group (DMBOC);
R₃ is a protecting group which is a levulinyl group (Lev);
R₅ is H, or a protecting group selected from the group consisting of 9-phenylxanthyl (pixyl or Px), MMTr, and DMTr;
Rₚ is a protecting group selected from the group consisting of methyl (Me), 2-cyanoethyl (CNEt), p-nitro-phenylethyl (NPE), and para- and ortho-chloro-phenyl (p- or o-CIPh);
R is lower alkyl, or the N(R)₂ moiety is a cyclic alkylamine, or a substituted cyclic alkylamine;
B is a nitrogen-containing base;
wherein each B, R₁ and Rₚ may be the same or different from any other B, R₁ and Rₚ, respectively;
and wherein
the term "lower alkyl" as used herein refers to acyclic, straight or branched chain alkyl groups containing from one to six carbons.

2. The compound of claim 1, wherein
R₁ is TBDMS;
R₅ is DMTr or MMTr; and
B is a nucleobase protected on at least one nitrogen by a N-protecting group selected from levulinyl, acetyl, difluoroacetyl, trifluoroacetyl, isobutyryl, benzoyl, 9- fluorenylmethoxycarbonyl, phenoxyacetyl, dimethylformamidine, and N,N-diphenyl carbamate.

3. The compound of claim 1, wherein R₅ is DMTr, and Rₚ is methyl.

4. A process for preparing a compound of claim 1 wherein
n is 1, 2, or 3;
R₁ is a protecting group as defined in claim 1;
R₅ is a protecting group as defined in claim 1;
R₃ is a levulinyl group (Lev);
Rₚ and B are as defined in claim 1;
and each B, R₁ and Rₚ may be the same or different from any other B, R₁ and Rₚ, respectively;
the process comprising the steps of:
a) condensing a phosphoramidite of formula (III): 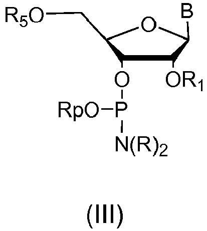
wherein B, R₁, R₅, and Rₚ are as defined above; and
R is lower alkyl, or the N(R)₂ moiety is a cyclic alkylamine, or a substituted cyclic alkylamine;
with a nucleoside of formula (IV): 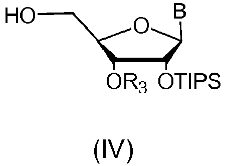
wherein B and R₃ are as defined above; and
b) oxidizing the product of step (a) to produce the compound of formula (II) where n is 1, and B, R₁, R₃, R₅, and Rₚ are as defined above; and
c) where n>1, the process further comprising:
(i) deprotecting the terminal -OR₅ group of the product of the previous step to form a free 5'-OH group;
(ii) condensing the product of step (i) with a phosphoramidite of formula (III), wherein B, R₁, R₅, Rₚ and R are as defined above, and each B, R₁, R₅, Rₚ and R may be the same or different from any other B, R₁, R₅, Rₚ and R, respectively;
(iii) oxidizing the product of step (ii);
(iv) repeating steps (i)-(iii) n-2 times;
to form the compound of formula (II);
the term "lower alkyl" as used herein refers to acyclic, straight or branched chain alkyl groups containing from one to six carbons.

5. The process of claim 4, wherein R₃ is a protecting group which is a levulinyl (Lev) group;
the process further comprising:
d) removal of the R₃ protecting group.

6. The process of claim 5, further comprising phophitylation of the product of step d) to form a compound of formula (IIa): 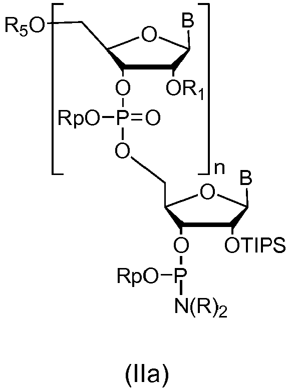 wherein n, B, R₁, R₅, Rₚ and R are as defined in claim 5.

7. The process of any one of claims 4 to 6, wherein:
R₁ is TBDMS;
R₅ is DMTr or MMTr;
Rₚ is methyl (Me), 2-cyanoethyl (CNEt), ortho-chlorophenyl (o-CIPh), or para-chlorophenyl (p-CIPh);
R is isopropyl, methyl, or ethyl; and
B is a nucleobase protected on at least one nitrogen by a N-protecting group selected from levulinyl, acetyl, difluoroacetyl, trifluoroacetyl, isobutyryl, benzoyl, 9- fluorenylmethoxycarbonyl, phenoxyacetyl, dimethylformamidine, and N,N-diphenyl carbamate.

8. A process for preparing the compound of claim 1, the compound having formula (V): 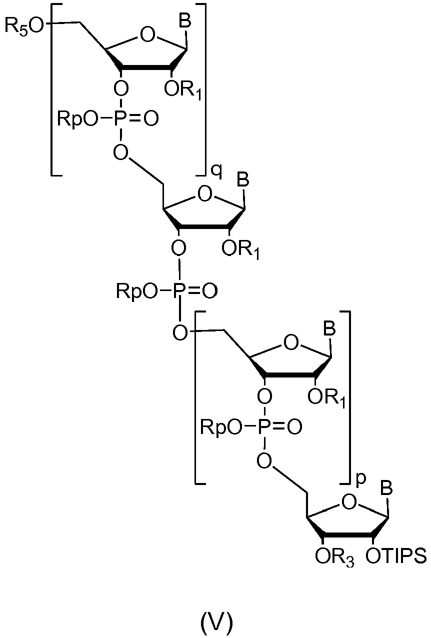 wherein
p is 0, 1, or 2;
q is 0, 1, or 2;
and p+q is less than or equal to 2;
R₅ is a protecting group as defined in claim 1;
R₃ is a protecting group which is a levulinyl group (Lev);
R₁, Rₚ and B are as defined in claim 1;
wherein each B, R₁ and Rₚ may be the same or different from any other B, R₁ and Rₚ, respectively;
the process comprising the steps of:
a) condensing a phosphoramidite of formula (VI): 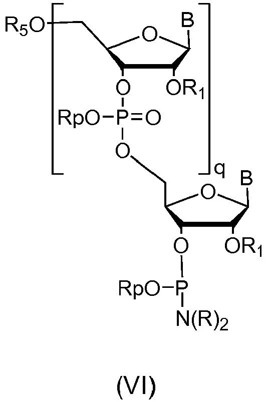
wherein q, B, R₁, R₅ and Rₚ are as defined above, and
R is lower alkyl, or the N(R)₂ moiety is a cyclic alkylamine, or a substituted cyclic alkylamine;
with a compound of formula (VII): 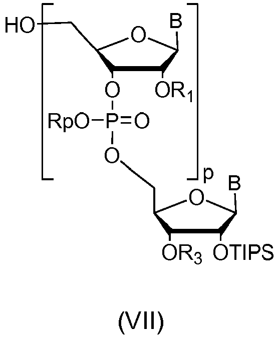 wherein p, B, R₁, R₃, and Rₚ are as defined above; and
b) oxidizing the product of step (a) to produce the compound of formula (V);
the term "lower alkyl" as used herein refers to acyclic, straight or branched chain alkyl groups containing from one to six carbons.

9. The process of claim 8, wherein:
R₁ is TBDMS;
R₅ is DMTr or MMTr;
Rₚ is methyl (Me), 2-cyanoethyl (CNEt), ortho-chlorophenyl (o-CIPh), or para-chlorophenyl (p-CIPh);
R is isopropyl, methyl, or ethyl; and
B is a nucleobase protected on at least one nitrogen by a N-protecting group selected from levulinyl, acetyl, difluoroacetyl, trifluoroacetyl, isobutyryl, benzoyl, 9- fluorenylmethoxycarbonyl, phenoxyacetyl, dimethylformamidine, and N,N-diphenyl carbamate.

## Patentansprüche

1. Verbindung der Formel (II): 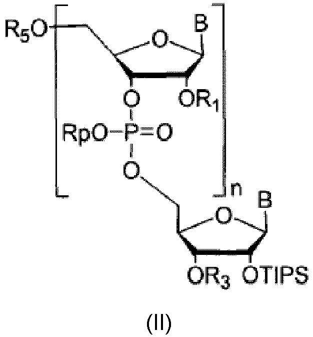 worin
n eine ganze Zahl von 1 bis 19 ist;
R₁ eine Schutzgruppe ist, die ausgewählt ist aus der Gruppe bestehend aus t-Butyldimethylsilyl (TBDMS), Triisopropylsilyl (TIPS); Triisopropyloxymethyl (TOM); Cyanoethylmethyl (CEM); 2-(4-Tolylsulfonyl)ethoxymethyl (TEM), 2'-O-bis(2-Acetoxyethoxy)methyl (ACE) Orthoester; 1-(2-Fluorphenyl)-4-methoxypiperidin-4-yl (Fpmp); 1-(4-Chlorphenyl)-4-ethoxypiperidin-4-yl (Cpep); 4-(N-Dichloracetyl-N- methylamino)benzyloxymethyl (4-MABOM); Dimethoxytrityl (DMTr), Monomethoxytrityl (MMTr), 2-tert-Butyldithiomethyl (DTM); Allyllevulinyl (Lev) und Acetallevulinyl (ALE); 2-(2-Nitrophenyl)propoxycarbonyl (NPPOC), α-Methylnitropiperonyloxycarbonyl (MeNPOC), Thioxanthon-Nitrobenzylgruppen-Konjugate und einer 5'-O-Dimethoxybenzoincarbonat-Gruppe (DMBOC);
R₃ eine Schutzgruppe ist, die eine Levulinyl-Gruppe (Lev) ist;
R₅ H oder eine Schutzgruppe ist, die aus der Gruppe ausgewählt ist, die aus 9-Phenylxanthyl (Pixyl oder Px), MMTr und DMTr besteht;
Rₚ eine Schutzgruppe ist, die aus der Gruppe ausgewählt ist, die aus Methyl (Me), 2-Cyanoethyl (CNEt), p-Nitrophenylethyl (NPE) und para- and ortho-Chlorphenyl (p- oder o-ClPh) besteht;
R Niederalkyl ist oder die N(R)₂-Einheit ein zyklisches Alkylamin oder ein substituiertes zyklisches Alkylamin ist;
B eine Stickstoff-enthaltende Base ist;
wobei jedes B, R₁ und Rₚ gleich oder unterschiedlich von jedem anderen B, R₁ bzw. Rₚ sein kann;
und wobei der Begriff "Niederalkyl" sich, wie hierin verwendet, auf azyklische, gerade oder verzweigte Alkylketten mit ein bis sechs Kohlenstoffen bezieht.

2. Verbindung nach Anspruch 1, worin:
R₁ TBDMS ist;
R₅ DMTr oder MMTr ist; und
B eine Nukleobase ist, die an mindestens einem Stickstoff durch eine N-Schutzgruppe geschützt ist, die aus Levulinyl, Acetyl, Difluoracetyl, Trifluoracetyl, Isobutyryl, Benzoyl, 9-Fluorenylmethoxycarbonyl, Phenoxyacetyl, Dimethylformamidin und N,N-Diphenylcarbamat ausgewählt ist.

3. Verbindung nach Anspruch 1, worin R₅ DMTr ist und Rₚ Methyl ist.

4. Verfahren zur Herstellung einer Verbindung nach Anspruch 1, worin
n 1, 2 oder 3 ist;
R₁ eine Schutzgruppe wie in Anspruch 1 definiert ist;
R₅ eine Schutzgruppe wie in Anspruch 1 definiert ist;
R₃ eine Levulinyl-Gruppe (Lev) ist;
Rₚ und B wie in Anspruch 1 definiert sind;
und jedes B, R₁ und Rₚ gleich oder unterschiedlich von jedem anderen B, R₁ bzw. Rₚ sein kann;
wobei das Verfahren die folgenden Schritte umfasst:
a) Kondensieren eines Phosphoramidits der Formel (III): 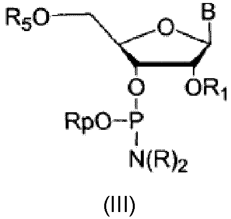
worin B, R₁, R₅, and Rₚ wie oben definiert sind; und
R Niederalkyl ist oder die N(R)₂-Einheit ein zyklisches Alkylamin oder ein substituiertes zyklisches Alkylamin ist;
mit einem Nukleosid der Formel (IV): 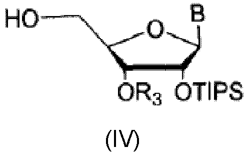
worin B und R₃ wie oben definiert sind; und
b) Oxidieren des Produkts aus Schritt (a) um die Verbindung der Formel (II) herzustellen, worin n 1 ist und B, R₁, R₃, R₅ und Rₚ wie oben definiert sind; und
c) wenn n>1, das Verfahren ferner umfasst:
(i) Entschützen der terminalen -OR₅-Gruppe des Produkts des vorhergehenden Schritts, um eine freie 5'-OH-Gruppe zu bilden;
(ii) Kondensieren des Produkts aus Schritt (i) mit einem Phosphoramidit der Formel (III), worin B, R₁, R₅, Rₚ und R wie oben definiert sind und jedes B, R₁, R₅, Rₚ und R gleich oder unterschiedlich von jedem anderen B, R₁, R₅, Rₚ bzw. R sein kann;
(iii) Oxidieren des Produkts aus Schritt (ii);
(iv) Wiederholen der Schritte (i)-(iii) n-2 mal;
um die Verbindung der Formel (II) zu bilden;
wobei der Begriff "Niederalkyl" sich, wie hierin verwendet, auf azyklische, gerade oder verzweigte Alkylketten mit ein bis sechs Kohlenstoffen bezieht

5. Verfahren nach Anspruch 4, wobei R₃ eine Schutzgruppe ist, die eine Levulinyl (Lev) Gruppe ist;
wobei das Verfahren ferner umfasst:
d) Entfernen der R₃-Schutzgruppe.

6. Verfahren nach Anspruch 5, ferner umfassend Phosphitylierung des Produkts aus Schritt (d) um eine Verbindung der Formel (IIa) zu bilden: 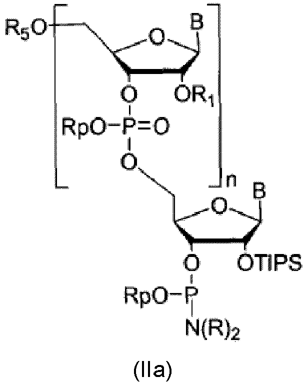 worin n, B, R₁, R₅, Rₚ und R wie in Anspruch 5 definiert sind.

7. Verfahren nach einem der Ansprüche 4 bis 6, worin
R₁ TBDMS ist;
R₅ DMTr oder MMTr ist;
Rₚ Methyl (Me), 2-Cyanoethyl (CNEt), ortho-Chlorphenyl (o-ClPh) oder para-Chlorphenyl (p-ClPh) ist;
R Isopropyl, Methyl oder Ethyl ist; und
B eine Nukleobase ist, die an mindestens einem Stickstoff durch eine N-Schutzgruppe geschützt ist, die aus Levulinyl, Acetyl, Difluoracetyl, Trifluoracetyl, Isobutyryl, Benzoyl, 9-Fluorenylmethoxycarbonyl, Phenoxyacetyl, Dimethylformamidin und N,N-Diphenylcarbamat ausgewählt ist.

8. Verfahren zur Herstellung der Verbindung nach Anspruch 1, wobei die Verbindung die Formel (V) hat: 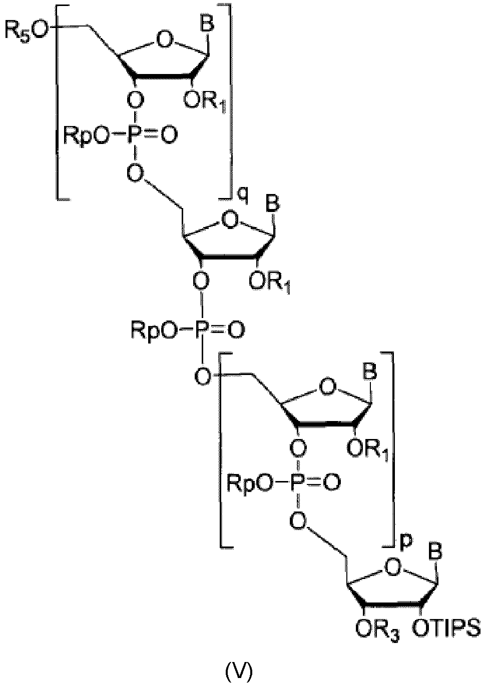 worin
p 0, 1 oder 2 ist;
q 0, 1 oder 2 ist;
und p+q weniger als oder gleich 2 ist;
R₅ eine Schutzgruppe wie in Anspruch 1 definiert ist;
R₃ eine Schutzgruppe ist, die eine Levulinyl-Gruppe (Lev) ist;
R₁, Rₚ und B wie in Anspruch 1 definiert sind;
worin jedes B, R₁ und Rₚ gleich oder unterschiedlich von jedem anderen B, R₁ bzw. Rₚ sein kann;
wobei das Verfahren die folgenden Schritte umfasst:
a) Kondensieren eines Phosphoramidits der Formel (VI): 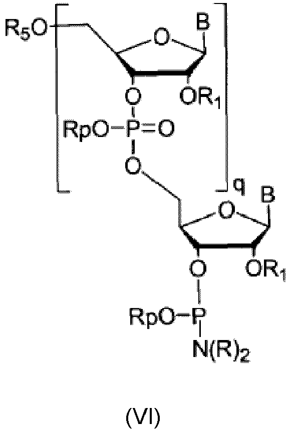
worin q, B, R₁, R₅ und Rₚ wie oben definiert sind und
R Niederalkyl ist oder die N(R)₂-Einheit ein zyklisches Alkylamin oder ein substituiertes zyklisches Alkylamin ist;
mit einer Verbindung der Formel (VII): 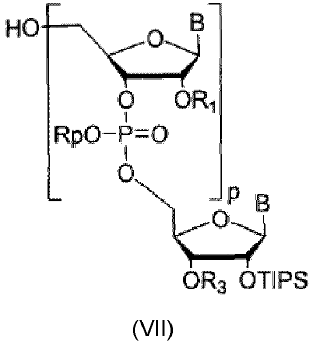
worin p, B, R₁, R₃ und Rₚ wie oben definiert sind; und
b) Oxidieren des Produkts aus Schritt (a) um die Verbindung der Formel (V) herzustellen;
wobei der Begriff "Niederalkyl" sich, wie hierin verwendet, auf azyklische, gerade oder verzweigte Alkylketten mit ein bis sechs Kohlenstoffen bezieht.

9. Verfahren nach Anspruch 8, worin
R₁ TBDMS ist;
R₅ DMTr oder MMTr ist;
Rₚ Methyl (Me), 2-Cyanoethyl (CNEt), ortho-Chlorphenyl (o-ClPh) oder para-Chlorphenyl (p-ClPh) ist;
R Isopropyl, Methyl oder Ethyl ist; und
B eine Nukleobase ist, die an mindestens einem Stickstoff durch eine N-Schutzgruppe geschützt ist, die aus Levulinyl, Acetyl, Difluoracetyl, Trifluoracetyl, Isobutyryl, Benzoyl, 9-Fluorenylmethoxycarbonyl, Phenoxyacetyl, Dimethylformamidin und N,N-Diphenylcarbamat ausgewählt ist.

## Revendications

1. Composé de formule (II) : 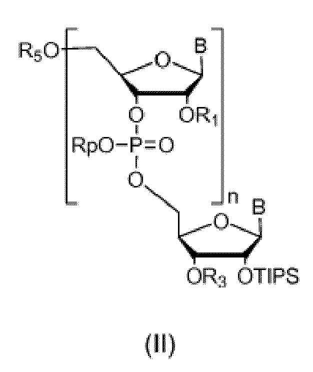 dans laquelle
n est un nombre entier de 1 à 19 ;
R₁ est un groupe protecteur choisi dans le groupe constitué par le t-butyldiméthylsilyle (TBDMS), le triisopropylsilyle (TIPS) ; le triisopropyloxyméthyle (TOM) ; le cyanoéthylméthyle (CEM) ; le 2-(4-tolylsulfonyl)éthoxyméthyle (TEM) ; l'orthoester de 2'-O-bis(2-acétoxyéthoxy)méthyle (ACE) ; le 1-(2-fluorophényl)-4-méthoxypipéridin-4-yle (Fpmp) ; le 1-(4-chlorophényl)-4-éthoxypipéridin-4-yle (Cpep) ; le 4-(N-dichloroacétyl-N-méthylamino)benzyloxyméthyle (4-MABOM) ; le diméthoxytrityle (DMTr), le monométhoxytrityle (MMTr) ; le 2-tert-butyldithiométhyle (DTM) ; l'allyl lévulinyle (Lev) et l'acétal lévulinyle (ALE) ; le 2-(2-nitrophényl)propoxycarbonyle (NPPOC), l'α-méthylnitropipéronyloxycarbonyle (MeNPOC), les conjugués de groupe thioxanthone-nitrobenzyle et un groupe 5'-O-diméthoxybenzoïnecarbonate (DMBOC) ;
R₃ est un groupe protecteur qui est un groupe lévulinyle (Lev) ;
R₅ est H, ou un groupe protecteur choisi dans le groupe constitué par le 9-phénylxanthyle (pixyle ou Px), le MMTr et le DMTr ;
Rₚ est un groupe protecteur choisi dans le groupe constitué par le méthyle (Me), le 2-cyanoéthyle (CNEt), le p-nitro-phényléthyle (NPE) et le para- et ortho-chloro-phényle (p- ou o-CIPh) ;
R est un alkyle inférieur, ou la fraction N(R)₂ est une alkylamine cyclique, ou une alkylamine cyclique substituée ;
B est une base contenant de l'azote ;
dans laquelle chaque B, R₁ et Rₚ peut être identique ou différent de tout autre B, R₁ et Rₚ, respectivement ;
et dans laquelle
le terme « alkyle inférieur » tel qu'il est utilisé ici fait référence à des groupes alkyle acycliques, à chaîne droite ou ramifiée contenant de un à six carbones.

2. Composé selon la revendication 1, dans lequel
R₁ est le TBDMS ;
R₅ est le DMTr ou le MMTr ; et
B est une nucléobase protégée sur au moins un azote par un groupe N-protecteur choisi parmi le lévulinyle, l'acétyle, le difluoroacétyle, le trifluoroacétyle, l'isobutyryle, le benzoyle, le 9-fluorénylméthoxycarbonyle, le phénoxyacétyle, la diméthylformamidine et le N,N-diphénylcarbamate.

3. Composé selon la revendication 1, dans lequel R₅ est le DMTr et Rₚ est le méthyle.

4. Procédé de préparation d'un composé selon la revendication 1, dans lequel
n est 1, 2 ou 3 ;
R₁ est un groupe protecteur tel que défini dans la revendication 1 ;
R₅ est un groupe protecteur tel que défini dans la revendication 1 ;
R₃ est un groupe lévulinyle (Lev) ;
Rₚ et B sont tels que définis dans la revendication 1 ;
et chaque B, R₁ et Rₚ peut être identique ou différent de tout autre B, R₁ et Rₚ, respectivement ;
le procédé comprenant les étapes de :
a) condensation d'un phosphoramidite de formule (III) : 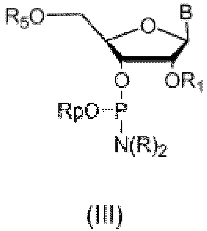
dans laquelle B, R₁, R₅ et Rₚ sont tels que définis ci-dessus ; et
R est un alkyle inférieur, ou la fraction N(R)₂ est une alkylamine cyclique, ou une alkylamine cyclique substituée ;
avec un nucléoside de formule (IV) : 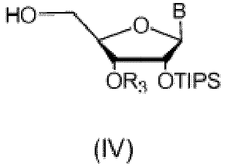
dans laquelle B et R₃ sont tels que définis ci-dessus ; et
b) l'oxydation du produit de l'étape (a) pour produire le composé de formule (II) où n est 1, et B, R₁, R₃, R₅ et Rₚ sont tels que définis ci-dessus ; et
c) où n > 1, le procédé comprenant en outre :
(i) la déprotection du groupe terminal -OR₅ du produit de l'étape précédente pour former un groupe 5'-OH libre ;
(ii) la condensation du produit de l'étape (i) avec un phosphoramidite de formule (III), dans laquelle B, R₁, R₅, Rₚ et R sont tels que définis ci-dessus, et chaque B, R₁, R₅, Rₚ et R peut être identique ou différent de tout autre B, R₁, R₅, Rₚ et R, respectivement ;
(iii) l'oxydation du produit de l'étape (ii) ;
(iv) la répétition des étapes (i) à (iii) n-2 fois ;
pour former le composé de formule (II) ;
le terme « alkyle inférieur » tel qu'il est utilisé ici fait référence à des groupes alkyle acycliques, à chaîne droite ou ramifiée contenant de un à six carbones.

5. Procédé selon la revendication 4, dans lequel R₃ est un groupe protecteur qui est un groupe lévulinyle (Lev) ;
le procédé comprenant en outre :
d) l'élimination du groupe protecteur R₃.

6. Procédé selon la revendication 5, comprenant en outre la phosphitylation du produit de l'étape d) pour former un composé de formule (Ila) : 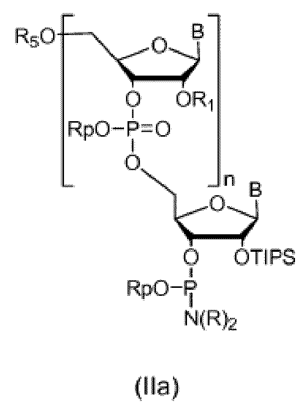 dans laquelle n, B, R₁, R₅, Rₚ et R sont tels que définis dans la revendication 5.

7. Procédé selon l'une quelconque des revendications 4 à 6, dans lequel :
R₁ est le TBDMS ;
R₅ est le DMTr ou le MMTr ;
Rₚ est le méthyle (Me), le 2-cyanoéthyle (CNEt), l'ortho-chlorophényle (o-CIPh) ou le para-chlorophényle (p-CIPh) ;
R est l'isopropyle, le méthyle ou l'éthyle ; et
B est une nucléobase protégée sur au moins un azote par un groupe N-protecteur choisi parmi le lévulinyle, l'acétyle, le difluoroacétyle, le trifluoroacétyle, l'isobutyryle, le benzoyle, le 9-fluorénylméthoxycarbonyle, le phénoxyacétyle, la diméthylformamidine et le N,N-diphényl carbamate.

8. Procédé de préparation du composé selon la revendication 1, le composé ayant la formule (V) : 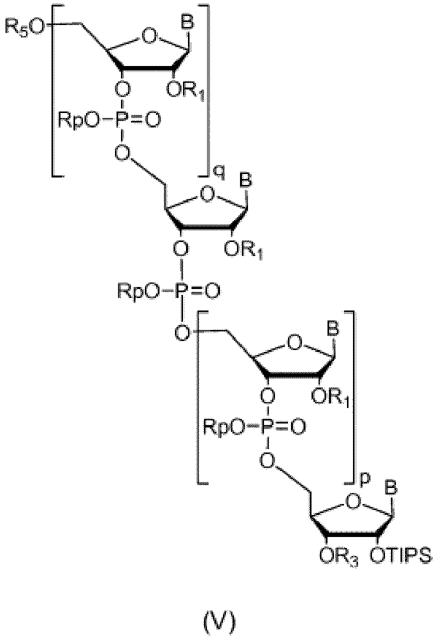 dans laquelle
p est 0, 1 ou 2 ;
q est 0, 1 ou 2 ;
et p+q est inférieur ou égal à 2 ;
R₅ est un groupe protecteur tel que défini dans la revendication 1 ;
R₃ est un groupe protecteur qui est un groupe lévulinyle (Lev) ;
R₁, Rₚ et B sont tels que définis dans la revendication 1 ;
dans laquelle chaque B, R₁ et Rₚ peut être identique ou différent de tout autre B, R₁ et Rₚ, respectivement ;
le procédé comprenant les étapes de :
a) condensation d'un phosphoramidite de formule (VI) : 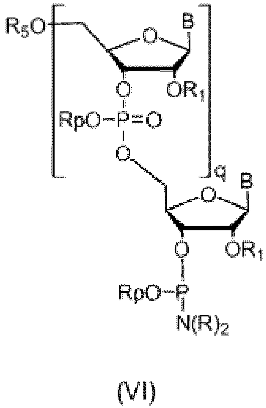
dans laquelle q, B, R₁, R₅ et Rₚ sont tels que définis ci-dessus, et
R est un alkyle inférieur, ou la fraction N(R)₂ est une alkylamine cyclique, ou une alkylamine cyclique substituée ;
avec un composé de formule (VII) : 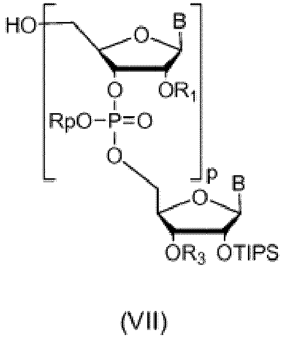
dans laquelle p, B, R₁, R₃ et Rₚ sont tels que définis ci-dessus ; et
b) oxydation du produit de l'étape (a) pour produire le composé de formule (V) ;
le terme « alkyle inférieur » tel qu'il est utilisé ici fait référence à des groupes alkyle acycliques, à chaîne droite ou ramifiée contenant de un à six carbones.

9. Procédé selon la revendication 8, dans lequel :
R₁ est le TBDMS ;
R₅ est le DMTr ou le MMTr ;
Rₚ est le méthyle (Me), le 2-cyanoéthyle (CNEt), l'ortho-chlorophényle (o-CIPh) ou le para-chlorophényle (p-CIPh) ;
R est l'isopropyle, le méthyle ou l'éthyle ; et
B est une nucléobase protégée sur au moins un azote par un groupe N-protecteur choisi parmi le lévulinyle, l'acétyle, le difluoroacétyle, le trifluoroacétyle, l'isobutyryle, le benzoyle, le 9-fluorénylméthoxycarbonyle, le phénoxyacétyle, la diméthylformamidine et le N,N-diphényl carbamate.
